# EUROPÄISCHE PATENTSCHRIFT

(11) **EP 3 596 065 B1**
(45) Veröffentlichungstag und Bekanntmachung des Hinweises auf die Patenterteilung: **25.12.2024**
(21) Anmeldenummer: 18709022.0
(22) Anmeldetag: 12.03.2018
(51) Int. Cl.: H10K 50/16, C07D 405/14, C07D 401/04, C07D 401/10, C07D 403/04, C07D 405/10, C07D 405/12, C07D 471/04, C07D 495/04, C07D 239/84, C07D 417/04, C07D 491/04, C07D 405/04

(54) **VERBINDUNGEN MIT ARYLAMIN-STRUKTUREN**
COMPOUNDS WITH ARYLAMINE STRUCTURES
COMPOSÉS AYANT DE STRUCTURES ARYLAMINE

(30) Priorität: 13.03.2017 EP 17160526
(43) Veröffentlichungstag der Anmeldung: 22.01.2020
(73) Patentinhaber: Merck Patent GmbH, 64293 Darmstadt (DE)
(72) Erfinder: PARHAM, Amir, 60486 Frankfurt am Main (DE); JOOSTEN, Dominik, 60487 Frankfurt am Main (DE); LUDEMANN, Aurélie, 60322 Frankfurt am Main (DE); GROSSMANN, Tobias, 64297 Darmstad (DE); KROEBER, Jonas, 60311 Frankfurt am Main (DE)
(74) Vertreter: Merck Patent Association
(86) Internationale Anmeldenummer: PCT/EP2018/055996
(87) Internationale Veröffentlichungsnummer: WO 2018/166932

(56) Entgegenhaltungen:
- WO-A1-2014/079527
- WO-A1-2017/126818
- WO-A1-2017/150859
- WO-A2-2012/143080

## Beschreibung

Die vorliegende Erfindung beschreibt Arylaminderivate, welche mit Diazanaphthalin-Gruppen substituiert sind, insbesondere zur Verwendung in elektronischen Vorrichtungen. Die Erfindung betrifft ferner ein Verfahren zur Herstellung der erfindungsgemäßen Verbindungen sowie elektronische Vorrichtungen enthaltend diese Verbindungen.

In organischen Elektrolumineszenzvorrichtungen (OLEDs) werden als emittierende Materialien häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Phosphoreszenz zeigen, immer noch Verbesserungsbedarf, beispielsweise im Hinblick auf Effizienz, Betriebsspannung und Lebensdauer.

Die Eigenschaften organischer elektrolumineszierender Vorrichtungen werden nicht nur durch die eingesetzten Emitter bestimmt. Hier sind insbesondere auch die anderen verwendeten Materialien, wie Host- und Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können zu deutlichen Verbesserungen elektrolumineszierender Vorrichtungen führen.

WO 2012/143080 beschreibt verbrückte Carbazolderivate, welche durch (hetero)aromatische Gruppen substituiert sein können, wobei diese aromatischen Gruppen mit einer Diarylaminogruppe substituiert sein können. WO 2014/079527 beschreibt Verbindungen, bei welchen eine N-Carbazolylgruppe und eine Diarylaminogruppe über eine aromatische Gruppe miteinander verbunden sind. All diese Verbindungen können in OLEDs eingesetzt werden.

Gemäß dem Stand der Technik werden als Matrixmaterialien für phosphoreszierende Verbindungen sowie als Elektronentransportmaterialien häufig heteroaromatische Verbindungen eingesetzt, wie zum Beispiel Chinazolinderivate. Weiterhin werden als Matrixmaterialien auch Triarylaminderivate verwendet, wobei auch Verbindungen bekannt sind, die sowohl Triarylamin-Strukturen als auch Gruppen aufweisen, die von Chinazolin abgeleitet sind. Allerdings weisen diese Verbindungen nicht zwingend an mindestens zwei der von der Triarylaminstruktur abgeleiteten Arylresten, die mit dem Stickstoffatom verbunden sind, eine Substitution mit einer Aryl- oder Heteroarylgruppe auf. Ferner sind nicht alle diese Verbindungen an dem Ring der Diazanaphthalinstruktur, an den die Diarylamingruppe bindet, mit einer weiteren Aryl- oder Heteroarylgruppe substituiert. Weiterhin bilden einige der Verbindungen Carbazolgruppen mit einem Arylrest, der an das Stickstoffatom der Diarylamingruppe bindet.

Generell besteht bei diesen Materialien, beispielsweise für die Verwendung als Matrixmaterialien, Lochtransportmaterialien oder Elektronentransportmaterialien noch Verbesserungsbedarf, insbesondere in Bezug auf die Lebensdauer, aber auch in Bezug auf die Effizienz und die Betriebsspannung der Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung eignen und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung, Verbindungen zur Verfügung zu stellen, die zu hoher Lebensdauer, guter Effizienz und geringer Betriebsspannung führen. Gerade auch die Eigenschaften der Matrixmaterialien, der Lochtransportmaterialien oder der Elektronentransportmaterialien haben einen wesentlichen Einfluss auf die Lebensdauer und die Effizienz der organischen Elektrolumineszenzvorrichtung.

Eine weitere Aufgabe der vorliegenden Erfindung kann darin gesehen werden, Verbindungen bereitzustellen, welche sich für den Einsatz in einer phosphoreszierenden oder fluoreszierenden OLED eignen, insbesondere als Matrixmaterial. Insbesondere ist es eine Aufgabe der vorliegenden Erfindung, Matrixmaterialien bereitzustellen, welche sich für rot, gelb und grün phosphoreszierende OLEDs eignen.

Weiterhin sollten die Verbindungen, insbesondere bei ihrem Einsatz als Matrixmaterialien, als Lochtransportmaterialien oder als Elektronentransportmaterialien in organischen Elektrolumineszenzvorrichtung zu Vorrichtungen führen, die eine ausgezeichnete Farbreinheit aufweisen.

Weiterhin sollten sich die Verbindungen möglichst einfach verarbeiten lassen, insbesondere eine gute Löslichkeit und Filmbildung zeigen. Beispielsweise sollten die Verbindungen eine erhöhte Oxidationsstabilität und eine verbesserte Glasübergangstemperatur zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen
Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, nachfolgend näher beschriebene Verbindungen diese Aufgaben lösen und den Nachteil aus dem Stand der Technik beseitigen. Die Verwendung der Verbindungen führt zu sehr guten Eigenschaften organischer elektronischer Vorrichtungen, insbesondere von organischen Elektrolumineszenzvorrichtungen, insbesondere hinsichtlich der Lebensdauer, der Effizienz und der Betriebsspannung. Elektronische Vorrichtungen, insbesondere organische Elektrolumineszenzvorrichtungen, welche derartige Verbindungen enthalten, sowie die entsprechenden bevorzugten Ausführungsformen sind daher Gegenstand der vorliegenden Erfindung.

Gegenstand der vorliegenden Erfindung ist daher eine Verbindung, umfassend mindestens eine Struktur gemäß der folgenden Formeln (IIIa), (IIIb), (XXIa), (XXIb), (XXIVa) oder (XXIVb) 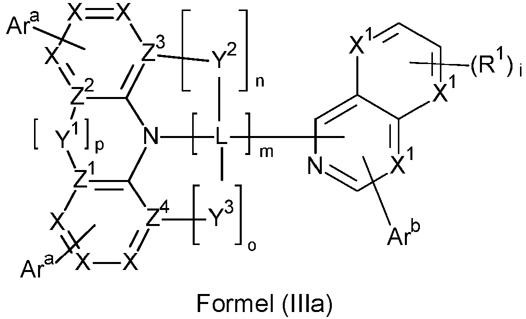 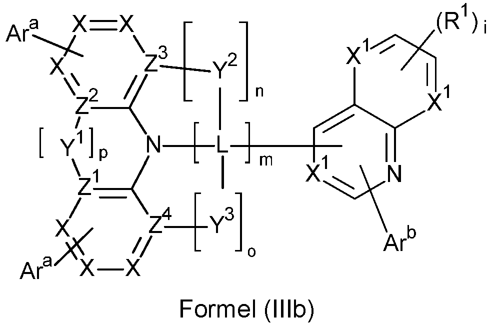 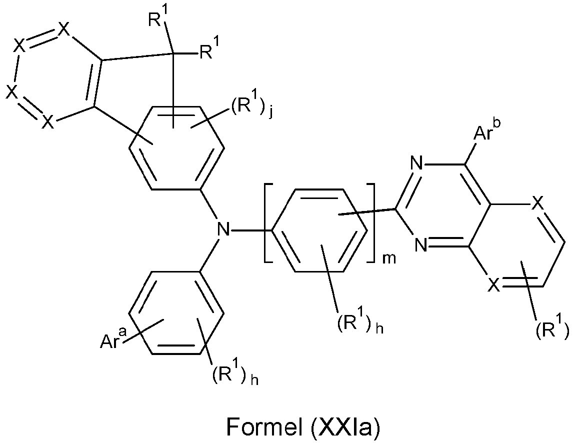 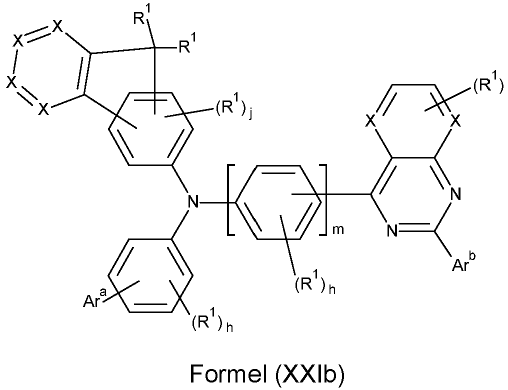 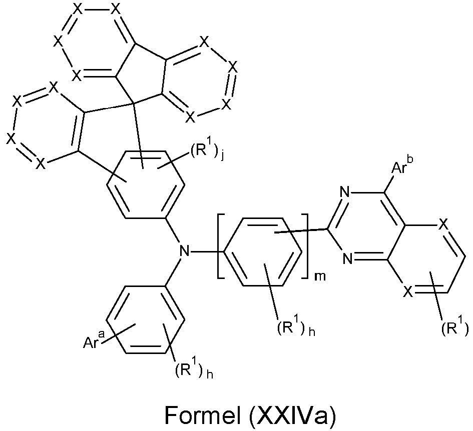 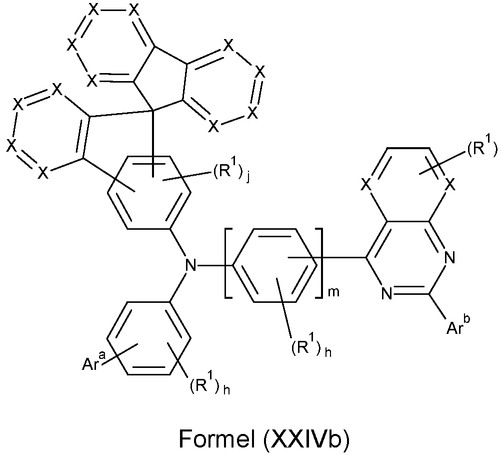 wobei für die verwendeten Symbole gilt:
- L: ist ein aromatisches oder heteroaromatisches Ringsystem mit 5 bis 30 aromatischen Ringatomen, das mit einem oder mehreren nicht-aromatischen Resten R¹ substituiert sein kann;
- Z¹, Z², Z³, Z⁴: ist X;
- X: ist bei jedem Auftreten gleich oder verschieden N oder CR¹, wobei maximal zwei X pro Ring für N stehen, vorzugsweise CR¹, oder C, falls an X ein Rest Ar^{a} bindet;
- X¹: ist bei jedem Auftreten gleich oder verschieden N oder CR¹;
- n, o, p: ist 0;
- m: ist 0 oder 1 ;
- i: ist 0, 1 oder 2, bevorzugt 0 oder 1;
- j: ist 0, 1, 2 oder 3, bevorzugt 0, 1 oder 2, bevorzugter 0 oder 1;
- h: ist 0, 1, 2, 3 oder 4, bevorzugt 0, 1 oder 2, bevorzugter 0 oder 1;
- Ar^{a}: ist bei jedem Auftreten gleich oder verschieden ein aromatisches oder heteroaromatisches Ringsystem mit 5 bis 30 aromatischen Ringatomen, welches durch einen oder mehrere Reste R¹ substituiert sein kann, wobei der Rest Ar^{a} keine Carbazol-Gruppe umfasst oder mit der Aryl- oder Heteroarylgruppe bildet, an die Ar^{a} bindet, wobei dies Substituenten R¹, R² und R³, die an den Rest Ar^{a} gebunden sein können, einschließt;
- Ar^{b}: ist ein aromatisches oder heteroaromatisches Ringsystem mit 5 bis 45 aromatischen Ringatomen, welches durch einen oder mehrere Reste R¹ substituiert sein kann;
- R¹: ist bei jedem Auftreten gleich oder verschieden H, D, CN, N(Ar¹)₂, eine geradkettige Alkylgruppe mit 1, 2, 3 oder 4 C-Atomen oder eine verzweigte oder cyclische Alkylgruppe mit 3 bis 8 C-Atomen oder ein aromatisches oder heteroaromatisches Ringsystem mit 5 bis 40 aromatischen Ringatomen, das jeweils durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Kombination dieser Systeme; dabei können zwei oder mehrere, vorzugsweise benachbarte Substituenten R¹ auch miteinander ein monocyclisches, aliphatisches oder ein aromatisches Ringsystem bilden;
- Ar¹: ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R² substituiert sein kann; dabei können zwei Reste Ar¹, welche an dasselbe Si-Atom, N-Atom, P-Atom oder B-Atom binden, auch durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verbrückt sein;
- R²: ist bei jedem Auftreten gleich oder verschieden H, D, CN, eine geradkettige Alkylgruppe mit 1, 2, 3 oder 4 C-Atomen oder eine verzweigte Alkylgruppe mit 3 oder 4 C-Atomen, die jeweils mit einem oder mehreren Resten R³ substituiert sein kann, wobei ein oder mehrere H-Atome durch D oderCN ersetzt sein können, oder ein aromatisches oder heteroaromatisches Ringsystem mit 5 bis 40 aromatischen Ringatomen, das jeweils durch einen oder mehrere Reste R³ substituiert sein kann, oder eine Kombination dieser Systeme; dabei können zwei oder mehrere, vorzugsweise benachbarte Substituenten R² auch miteinander ein monocyclisches, aliphatisches oder aromatisches Ringsystem bilden;
- R³: ist bei jedem Auftreten gleich oder verschieden ausgewählt aus der Gruppe bestehend aus H, D, CN, einem aliphatischen Kohlenwasserstoffrest mit 1, 2, 3 oder 4 C-Atomen oder einem aromatischen oder heteroaromatischen Ringsystem mit 5 bis 30 aromatischen Ringatomen, in dem ein oder mehrere H-Atome durch DI oder CN ersetzt sein können und das durch ein oder mehrere Alkylgruppen mit jeweils 1 bis 4 Kohlenstoffatomen substituiert sein kann; dabei können zwei oder mehr, vorzugsweise benachbarte Substituenten R³ miteinander ein monocyclisches, aliphatisches Ringsystem bilden;

mit der Maßgabe, dass mindestens eine Gruppe X¹ in Formel (IIIa) bzw. (IIIb) für N steht, und
mit der Maßgabe, dass Verbindungen der Formel (A) 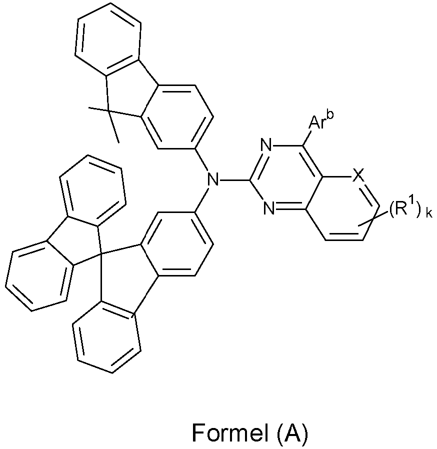
ausgenommen sind, wobei die verwendeten Symbole X, Ar^{b} und R¹ die zuvor dargelegte Bedeutung aufweisen und k 0 oder 1 ist.

Aus der obigen Formulierung ist ersichtlich, dass, da die Indexes n, o und p = 0 sind, die entsprechenden Brücken Y¹, Y², Y³ nicht vorhanden sind und das entsprechende Symbol Z¹, Z², Z³, Z⁴ in diesem Fall für X steht. Dagegen bedeutet m = 0, dass eine Einfachbindung zwischen dem Stickstoffatom und dem Heteroaromaten vorliegt. Besonders bevorzugt ist m = 0. Hierdurch bindet die Diazanaphthalin-Gruppe bzw. Chinazolin-Gruppe unmittelbar an das Stickstoffatom der Diarylamingruppe.

Ferner ist aus den obigen Strukturen der Formeln (IIIa) und (IIIb) ersichtlich, dass die Gruppe Ar^{b} und die Gruppe L an denselben Ring der Diazanaphthyl-Gruppe binden, wobei die beiden Bindungsstellen an die Diazanaphthyl-Gruppe nicht benachbart, sondern durch eine Gruppe X¹ getrennt sind. Vorzugsweise bindet die Gruppe L an eine Stelle an die Diazanaphthyl-Gruppe, die benachbart zu einem N-Atom der Diazanaphthyl-Gruppe ist, so dass das Symbol X', welches benachbart zur Bindungsstelle der Gruppe L ist, für ein Stickstoffatom steht. Vorzugsweise bindet die Gruppe Ar^{b} an eine Stelle an die Diazanaphthyl-Gruppe, die benachbart zu einem N-Atom der Diazanaphthyl-Gruppe ist, so dass das Symbol X', welches benachbart zur Bindungsstelle der Gruppe Ar^{b} ist, für ein Stickstoffatom steht.

Benachbarte Kohlenstoffatome im Sinne der vorliegenden Erfindung sind Kohlenstoffatome, die direkt miteinander verknüpft sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

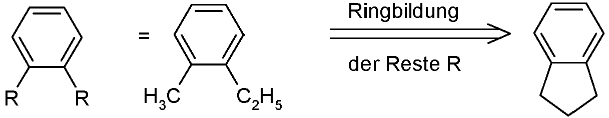

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

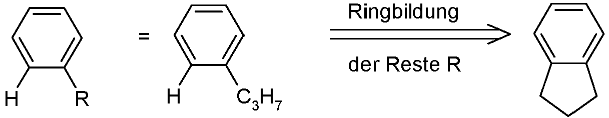

Eine kondensierte Arylgruppe, ein kondensiertes aromatisches Ringsystem oder ein kondensiertes heteroaromatisches Ringsystem im Sinne der vorliegenden Erfindung ist eine Gruppe, in der zwei oder mehr aromatische Gruppen über eine gemeinsame Kante aneinander ankondensiert, d. h. anelliert, sind, so dass beispielsweise zwei C-Atome zu den mindestens zwei aromatischen oder heteroaromatischen Ringen zugehören, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen. Entsprechende Definitionen gelten für Heteroarylgruppen sowie für kondensierte Ringsysteme, die auch Heteroatome enthalten können, jedoch nicht müssen.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₂₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ferner sind Verbindungen mit Strukturen gemäß Formel (IIIa) oder (IIIb) bevorzugt, in der mindestens zwei, vorzugweise mindestens drei der Symbole X für CR¹ stehen, besonders bevorzugt mindestens drei der Symbole X ausgewählt sind aus C-H und C-D.

Vorzugsweise können die erfindungsgemäßen Verbindungen mindestens eine Struktur der Formel (IVa) und/oder (IVb) umfassen, 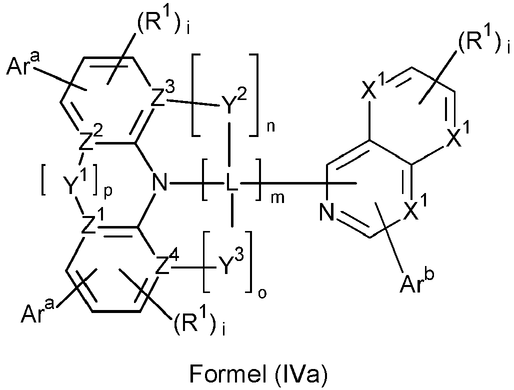 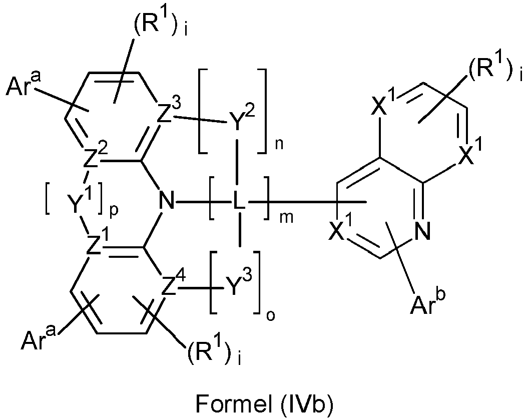 wobei die verwendeten Symbole m, n, o, p, L, R¹, Ar^{a}, Ar^{b}, Y¹, Y², Y³, Z¹, Z², Z³, Z⁴ und X¹ die zuvor dargelegte Bedeutung aufweisen, mindestens eine Gruppe X¹ für N steht und i für 0, 1 oder 2, vorzugsweise für 0 oder 1 steht.

Bevorzugt können die erfindungsgemäßen Verbindungen mindestens eine Struktur der Formel (Va) und/oder (Vb) umfassen, 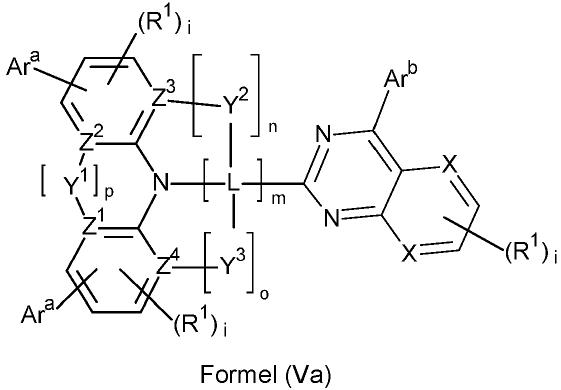 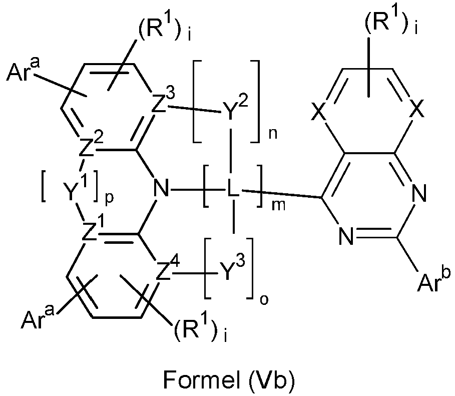 wobei die verwendeten Symbole m, n, o, p, L, R¹, Ar^{a}, Ar^{b}, Y¹, Y², Y³, Z¹, Z², Z³, Z⁴ und X die zuvordargelegte Bedeutung aufweisen und i für 0, 1 oder 2, vorzugsweise für 0 oder 1 steht.

In einer weiterhin bevorzugten Ausführungsform können die erfindungsgemäßen Verbindungen mindestens eine Struktur der Formel (VIa) und/oder (VIb) aufweisen, 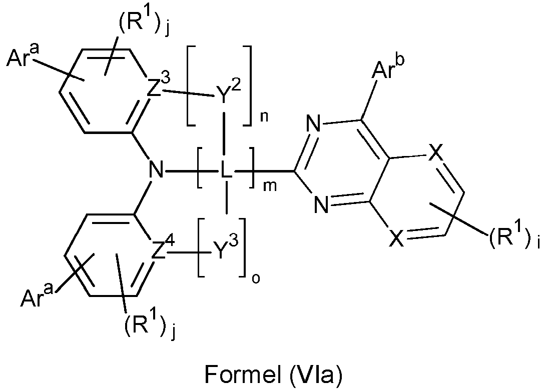 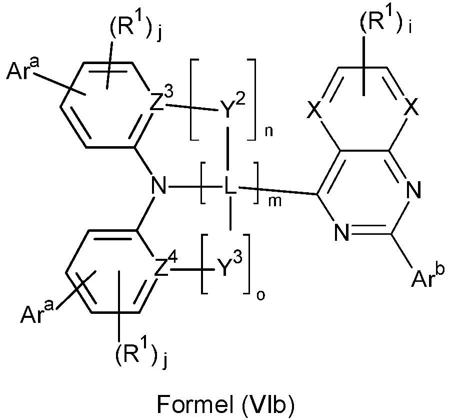 wobei die verwendeten Symbole m, n, o, L, R¹, Ar^{a}, Ar^{b}, Y¹, Y², Y³, Z³, Z⁴ und X die zuvorgenannte Bedeutung aufweisen, i für 0, 1 oder 2, vorzugsweise für 0 oder 1 und j für 0, 1, 2 oder 3, vorzugsweise für 0, 1 oder 2, besonders bevorzugt für 0 oder 1 steht.

Ferner kann vorgesehen sein, dass erfindungsgemäße Verbindungen mindestens eine Struktur der Formel (Vlla) und/oder (VIIb) umfassen, 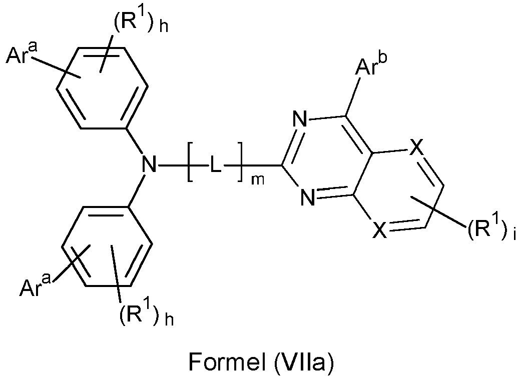 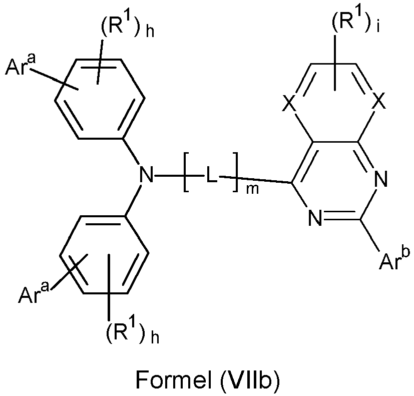 wobei die verwendeten Symbole m, L, Ar^{a}, Ar^{b}, R¹ und X die zuvor dargelegte Bedeutung aufweisen, i für 0, 1 oder 2, vorzugsweise für 0 oder 1 und h für 0, 1, 2, 3 oder 4, vorzugsweise für 0, 1 oder 2, besonders bevorzugt für 0 oder 1 steht.

Weiterhin sind Verbindungen, umfassend mindestens eine Struktur der Formel (Vllla) und/oder (Vlllb), bevorzugt, 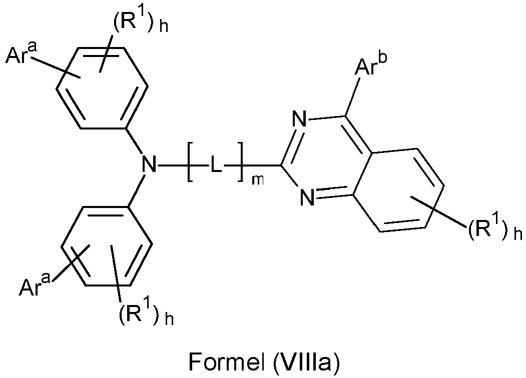 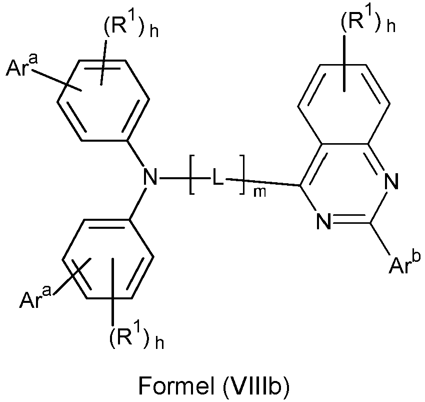 wobei die verwendeten Symbole m, L, Ar^{a}, Ar^{b} und R¹ die zuvor dargelegte Bedeutung aufweisen und h für 0, 1, 2, 3 oder 4, vorzugsweise für 0, 1 oder 2, besonders bevorzugt für 0 oder 1 steht.

Vorzugsweise kann der Rest Ar^{a} insbesondere in den Formeln (IIIa), (IIIb), (IVa), (IVb), (Va), (Vb), (VIa), (VIb), (Vlla), (Vllb), (Vllla), (Vlllb) eine Spirobifluoren-, Fluoren-, Dibenzofuran- oder Dibenzothiophen-Gruppe umfassen oder mit dem Aryl- oder Heteroarylrest bilden, an den der Rest Ar^{a} bindet.

In einer weiterhin bevorzugten Ausführungsform können die erfindungsgemäßen Verbindungen mindestens eine Struktur der Formel (IXa) oder (IXb) umfassen, 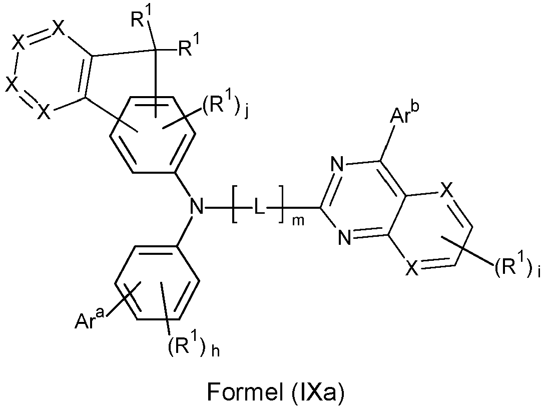 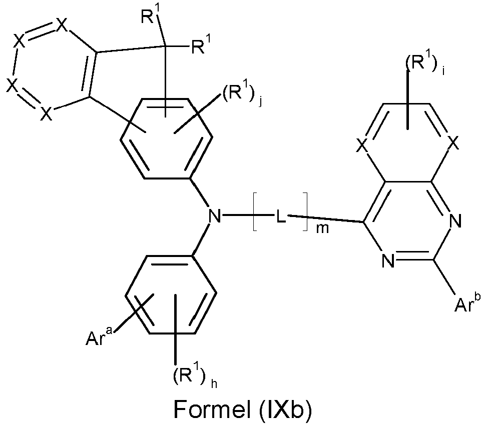 wobei die verwendeten Symbole m, L, Ar^{a}, Ar^{b}, R¹ und X die zuvor dargelegte Bedeutung aufweisen, j für 0, 1, 2 oder 3, vorzugsweise für 0, 1 oder 2, besonders bevorzugt für 0 oder 1 steht und h für 0, 1, 2, 3 oder 4, vorzugsweise für 0, 1 oder 2, besonders bevorzugt für 0 oder 1 steht und i für 0, 1 oder 2, vorzugsweise für 0 oder 1 steht.

Weiterhin können die erfindungsgemäßen Verbindungen mindestens eine Struktur der Formel (Xa) oder (Xb) umfassen, 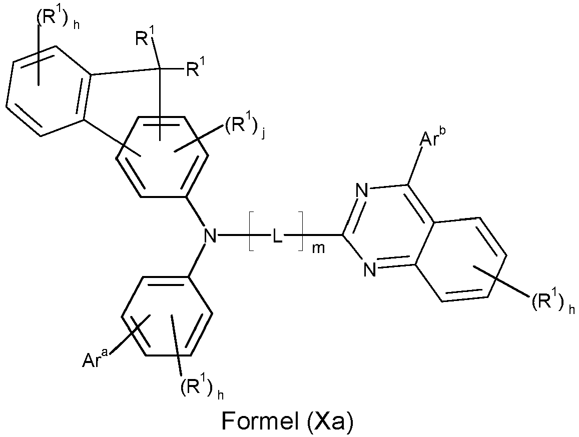 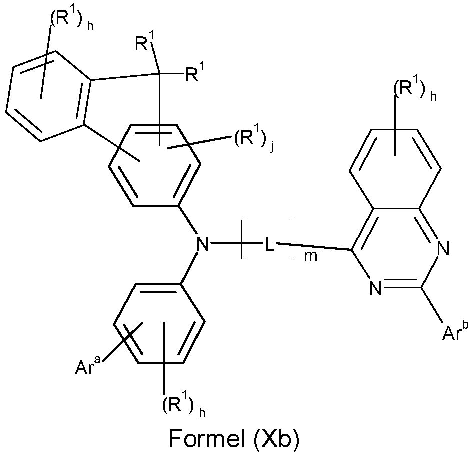 wobei die verwendeten Symbole m, L, Ar^{a}, Ar^{b} und R¹ die zuvor dargelegte Bedeutung aufweisen, j für 0, 1, 2 oder 3, vorzugsweise für 0, 1 oder 2, besonders bevorzugt für 0 oder 1 und h für 0, 1, 2, 3 oder 4, vorzugsweise für 0, 1 oder 2, besonders bevorzugt für 0 oder 1 steht.

In einer weiterhin bevorzugten Ausführungsform können die erfindungsgemäßen Verbindungen mindestens eine Struktur der Formel (XIa), (XIb), (XIc), (XId), (XIe) oder (XIf) aufweisen, 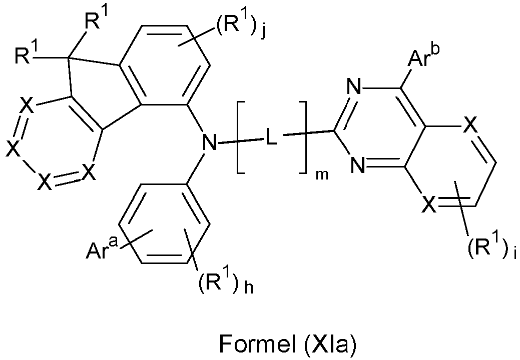 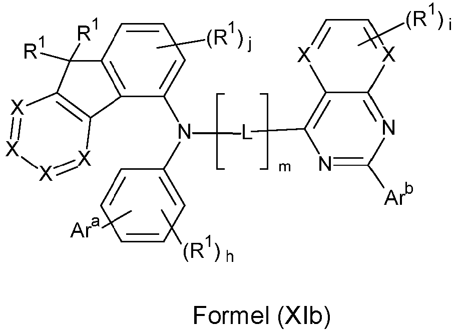 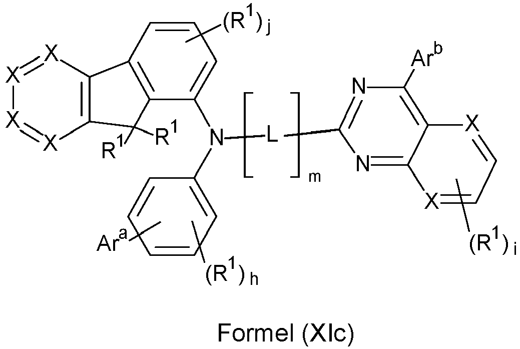 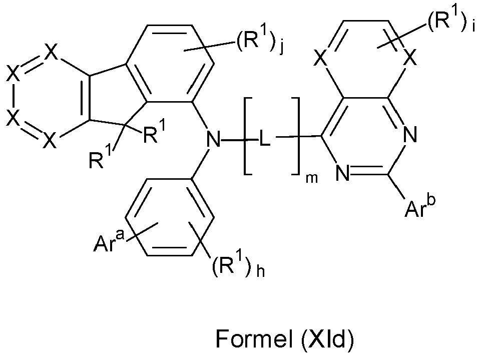 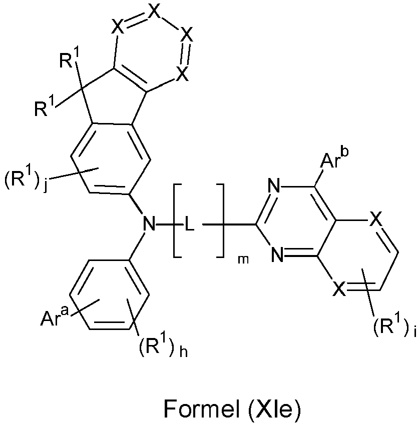 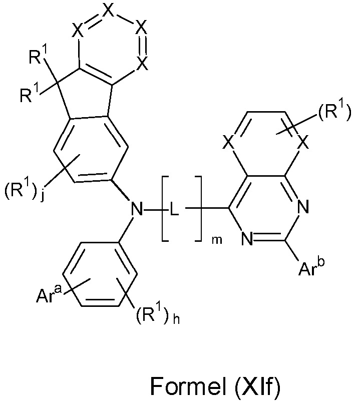 wobei die verwendeten Symbole m, L, Ar^{a}, Ar^{b}, R¹ und X die zuvor dargelegte Bedeutung aufweisen, i für 0, 1 oder 2, vorzugsweise für 0 oder 1, j für 0, 1, 2 oder 3, vorzugsweise für 0, 1 oder 2, besonders bevorzugt für 0 oder 1 und h für 0, 1, 2, 3 oder 4, vorzugsweise für 0, 1 oder 2, besonders bevorzugt für 0 oder 1 steht.

Ferner kann vorgesehen sein, dass erfindungsgemäße Verbindungen mindestens eine Struktur der Formel (Xlla) oder (XIIb) umfassen, 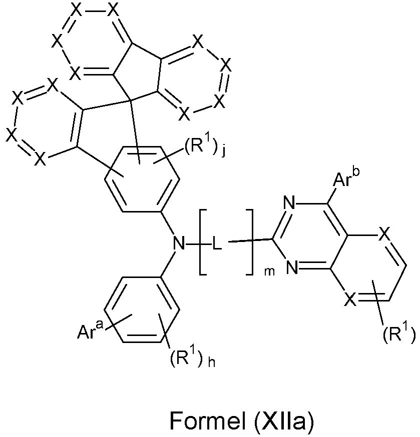 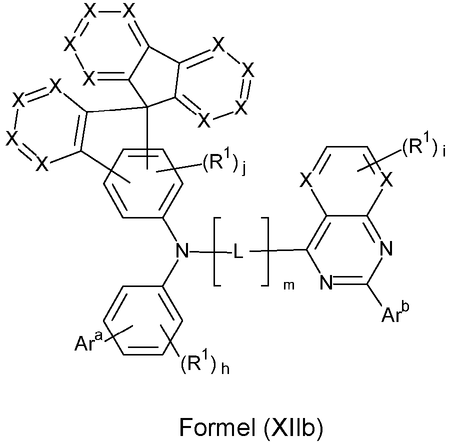 wobei die verwendeten Symbole m, L, Ar^{a}, Ar^{b}, R¹ und X die zuvor dargelegte Bedeutung aufweisen, i für 0, 1 oder 2, vorzugsweise für 0 oder 1, j für 0, 1, 2 oder 3, vorzugsweise für 0, 1 oder 2, besonders bevorzugt für 0 oder 1 und h für 0, 1, 2, 3 oder 4, vorzugsweise für 0, 1 oder 2, besonders bevorzugt für 0 oder 1 steht.

Bevorzugt kann die Gruppe L mit dem Diazanaphthalin-Rest, an den die Gruppe L gemäß Formeln (IIIa), (IIIb), (IVa), (IVb), (Va), (Vb), (VIa), (VIb), (Vlla), (Vllb), (Vllla), (Vlllb), (IXa), (IXb), (Xa), (Xb), (XIa), (XIb), (XIc), (XId), (XIe), (XIf), (Xlla), (Xllb) gebunden ist, und mit der Diarylamingruppe dieser Formeln eine durchgängige Konjugation ausbilden. In einer weiteren bevorzugten Ausführungsform der Erfindung steht L für ein aromatisches oder heteroaromatisches Ringsystem mit 5 bis 14 aromatischen oder heteroaromatischen Ringatomen, vorzugsweise ein aromatisches oder heteroaromatisches Ringsystem mit 6 bis 13 Kohlenstoffatomen, welches durch einen oder mehrere Reste R¹ substituiert sein kann, bevorzugt aber unsubstituiert ist, wobei R¹ die zuvor genannte Bedeutung aufweisen kann. Besonders bevorzugt steht L für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste R¹ substituiert sein kann, bevorzugt aber unsubstituiert ist, wobei R¹ die zuvor genannte Bedeutung aufweisen kann.

Weiterhin bevorzugt steht das unter anderem in den Strukturen gemäß Formeln (IIIa), (IIIb), (IVa), (IVb), (Va), (Vb), (VIa), (VIb), (Vlla), (Vllb), (Vllla), (Vlllb), (IXa), (IXb), (Xa), (Xb), (XIa), (XIb), (XIc), (XId), (XIe), (XIf), (XIIa) und/oder (Xllb) dargelegte Symbol L gleich oder verschieden bei jedem Auftreten für eine Bindung, was m = 0 entspricht, oder einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die unter anderem in den Strukturen gemäß Formeln (IIIa), (IIIb), (IVa), (IVb), (Va), (Vb), (VIa), (VIb), (Vlla), (Vllb), (Vllla), (Vlllb), (IXa), (IXb), (Xa), (Xb), (XIa), (XIb), (XIc), (XId), (XIe), (XIf), (XIIa) und/oder (Xllb) dargelegte Gruppe L ein aromatisches Ringsystem mit höchstens zwei kondensierten aromatischen und/oder heteroaromatischen Sechsringen, vorzugsweise kein kondensiertes aromatisches oder heteroaromatisches Ringsystem umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen. Speziell bevorzugt für die in Formeln (Illa), (IIIb), (IVa), (IVb), (Va), (Vb), (VIa), (VIb), (Vlla), (Vllb), (Vllla), (Vlllb), (IXa), (IXb), (Xa), (Xb), (XIa), (XIb), (XIc), (XId), (XIe), (XIf), (Xlla) und/oder (Xllb) dargelegte Gruppe L sind Phenyl-, Biphenyl-, Dibenzofuranyl- und/oder Dibenzothienyl-Strukturen, die durch einen oder mehrere Reste R¹, wie zuvor definiert, substituiert sein können. Beispiele für geeignete aromatische oder heteroaromatische Ringsysteme L sind ausgewählt aus der Gruppe bestehend aus ortho-, meta- oder para-Phenylen, ortho-, meta- oder para-Biphenylen, Terphenylen, insbesondere verzweigtes Terphenylen, Quaterphenylen, insbesondere verzweigtes Quaterphenylen, Fluorenylen, Spirobifluorenylen, Dibenzofuranylen, Dibenzothienylen und Carbazolylen, die jeweils durch einen oder mehrere Reste R² substituiert sein können, bevorzugt aber unsubstituiert sind.

Ferner kann vorgesehen sein, dass die unter anderem in den Strukturen gemäß Formeln (IIIa), (IIIb), (IVa), (IVb), (Va), (Vb), (VIa), (VIb), (Vlla), (Vllb), (Vllla), (Vlllb), (IXa), (IXb), (Xa), (Xb), (XIa), (XIb), (XIc), (XId), (XIe), (XIf), (Xlla) und/oder (Xllb) dargelegte Gruppe L höchstens 1 Stickstoffatom, bevorzugt höchstens 2 Heteroatome, insbesondere bevorzugt höchstens ein Heteroatom und besonders bevorzugt kein Heteroatom aufweist.

Bevorzugt sind Verbindungen umfassend Strukturen der Formeln (IIIa), (IIIb), (IVa), (IVb), (Va), (Vb), (VIa), (VIb), (Vlla), (Vllb), (Vllla), (Vlllb), (IXa), (IXb), (Xa), (Xb), (XIa), (XIb), (XIc), (XId), (XIe), (XIf), (Xlla) und/oder (Xllb), in denen die Gruppe L für eine Gruppe steht, die ausgewählt ist aus den Formeln (L-1) bis (L-15), 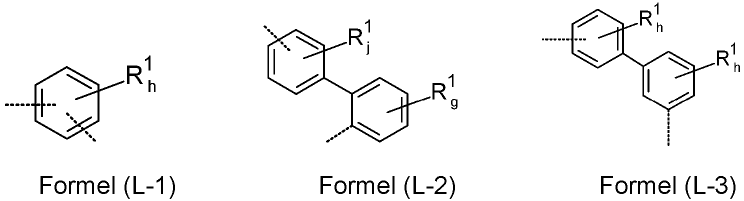 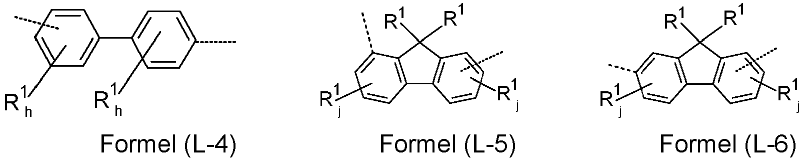 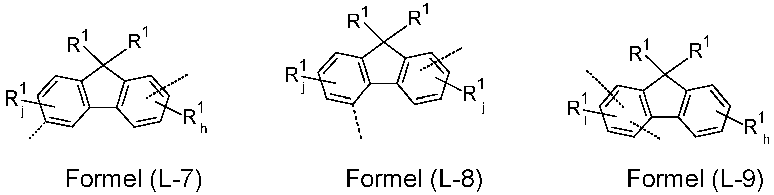 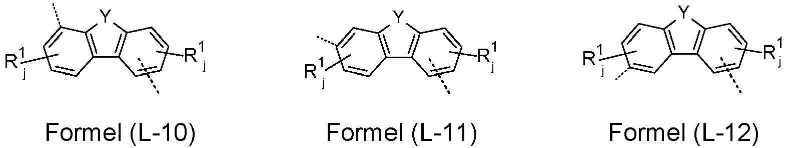 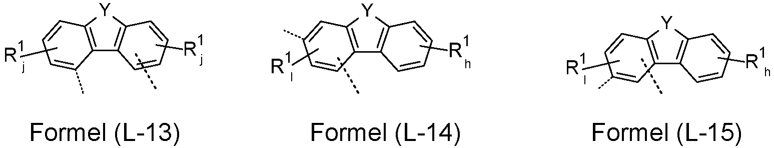 wobei die gestrichelten Bindungen jeweils die Anbindungspositionen markieren, der Index I 0, 1 oder 2 ist, der Index j bei jedem Auftreten unabhängig 0, 1, 2 oder 3 ist, der Index h bei jedem Auftreten unabhängig 0, 1, 2, 3 oder 4 ist, der Index g 0, 1, 2, 3, 4 oder 5 ist, das Symbol Y O, S oder NR², vorzugsweise O oder S ist, und das Symbol R¹ die zuvor, insbesondere für Formel (I) genannte Bedeutung aufweist.

Bevorzugt sind hierbei insbesondere die Formeln (L-1) bis (L-4) sowie (L-10) bis (L-15), wobei Y in den bevorzugten Formeln (L-10) bis (L-15) für O oder S steht. Vorzugsweise kann vorgesehen sein, dass die Summe der Indices k, l, g, h und j in den Strukturen der Formel (L-1) bis (L-15) jeweils höchstens 3, vorzugsweise höchstens 2 und besonders bevorzugt höchstens 1 beträgt.

In einer bevorzugten Ausgestaltung können die erfindungsgemäßen Verbindungen eine Struktur der Formel (XIII) enthalten, 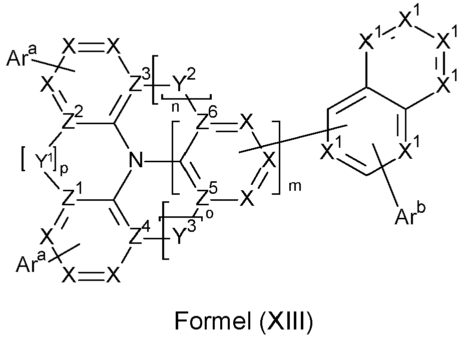 wobei Z⁵ und Z⁶ gleich oder verschieden für X oder C stehen, die verwendeten Symbole m, n, o, p, Ar^{a}, Ar^{b}, Y¹, Y², Y³, Z¹, Z², Z³, Z⁴, X und X¹ die zuvor genannte Bedeutung haben und mindestens zwei Gruppen X¹ für N stehen. Vorzugsweise stehen maximal zwei Gruppen X oder X¹ pro Ring für N.

Vorzugsweise können die erfindungsgemäßen Verbindungen mindestens eine Struktur der Formel (XIVa) und/oder (XIVb) umfassen, 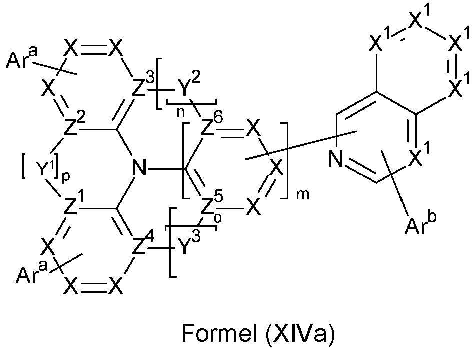 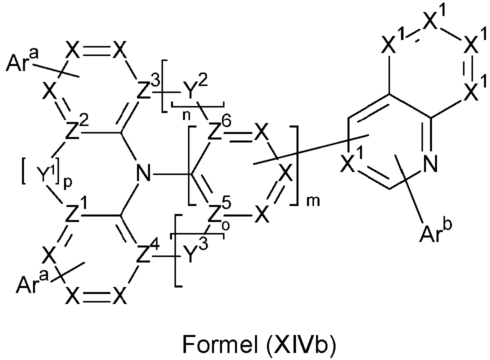 wobei die verwendeten Symbole m, n, o, p, Ar^{a}, Ar^{b}, Y¹, Y², Y³, Z¹, Z², Z³, Z⁴, Z⁵, Z⁶, X und X¹ die zuvor beziehungsweise Formel (XIII) genannte Bedeutung haben und mindestens eine Gruppe X¹ für N steht. Vorzugsweise stehen maximal zwei Gruppen X oder X¹ pro Ring für N.

Vorzugsweise können die erfindungsgemäßen Verbindungen Strukturen gemäß der Formel (XVa) und/oder (XVb) umfassen, 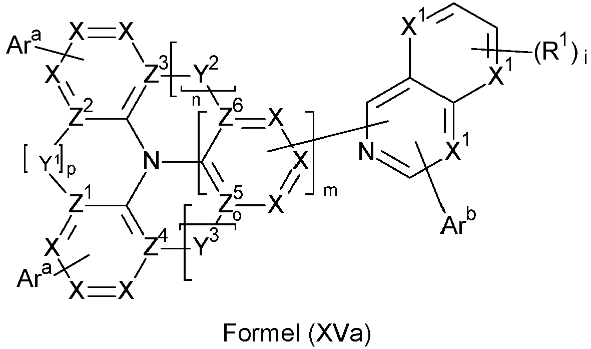 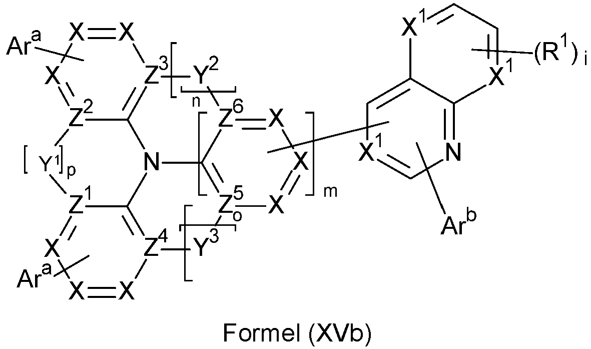 wobei die verwendeten Symbole m, n, o, p, Ar^{a}, Ar^{b}, Y¹, Y², Y³, Z¹, Z², Z³, Z⁴, Z⁵, Z⁶, X und X¹ die zuvor beziehungsweise Formel (XIII) genannte Bedeutung haben, mindestens eine Gruppe X¹ für N steht und i für 0, 1 oder 2, vorzugsweise für 0 oder 1 steht. Vorzugsweise stehen maximal zwei Gruppen X pro Ring für N.

Ferner sind Verbindungen mit Strukturen gemäß Formel (XIVa), (XIVb), (XVa) oder (XVb) bevorzugt, in der mindestens sechs, vorzugweise mindestens acht der Symbole X für CR¹ stehen, besonders bevorzugt mindestens sechs der Symbole X ausgewählt sind aus C-H und C-D.

Vorzugsweise können die erfindungsgemäßen Verbindungen mindestens eine Struktur der Formel (XVIa) und/oder (XVIb) umfassen, 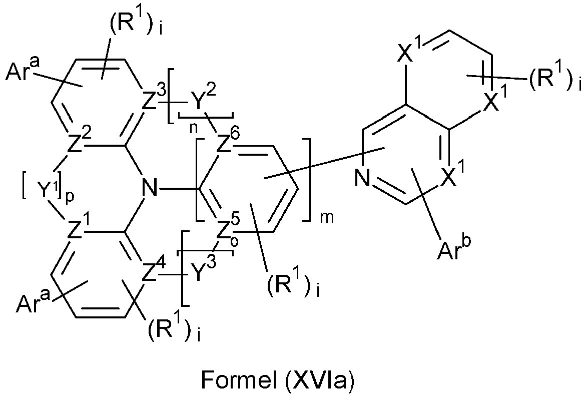 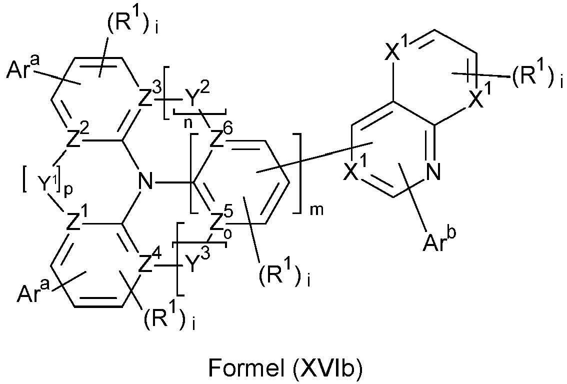 wobei die verwendeten Symbole m, n, o, p, Ar^{a}, Ar^{b}, Y¹, Y², Y³, Z¹, Z², Z³, Z⁴, Z⁵, Z⁶ und X¹ die zuvorbeziehungsweise Formel (XIII) genannte Bedeutung aufweisen, mindestens eine Gruppe X¹ für N steht und i für 0, 1 oder 2, vorzugsweise für 0 oder 1 steht.

Bevorzugt können die erfindungsgemäßen Verbindungen mindestens eine Struktur der Formel (XVIIa) und/oder (XVIIb) umfassen, 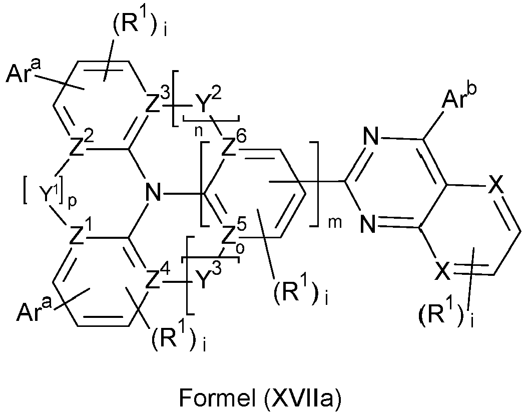 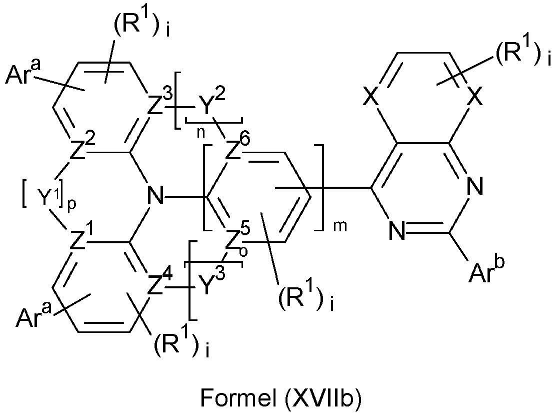 wobei die verwendeten Symbole m, n, o, p, Ar^{a}, Ar^{b}, Y¹, Y², Y³, Z¹, Z², Z³, Z⁴, Z⁵, Z⁶ und X die zuvor, insbesondere für Formel (XIII) genannte Bedeutung aufweisen und i für 0, 1 oder 2, vorzugsweise für 0 oder 1 steht.

In einer weiterhin bevorzugten Ausführungsform können die erfindungsgemäßen Verbindungen mindestens eine Struktur der Formel (XVIIIa) und/oder (XVIIIb) aufweisen, 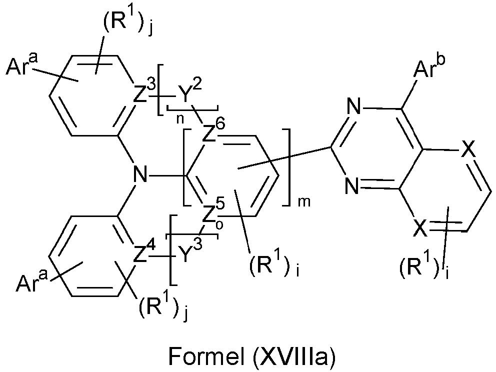 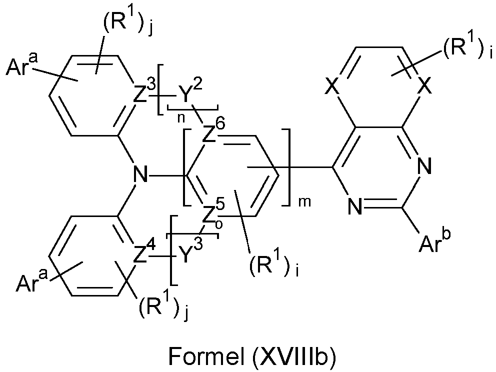 wobei die verwendeten Symbole m, n, o, Ar^{a}, Ar^{b}, Y¹, Y², Y³, Z³, Z⁴, Z⁵, Z⁶ und X die zuvor, insbesondere für Formel (XIII) genannte Bedeutung aufweisen, i für 0, 1 oder 2, vorzugsweise für 0 oder 1 und j für 0, 1, 2 oder 3, vorzugsweise für 0, 1 oder 2, besonders bevorzugt für 0 oder 1 steht.

Ferner kann vorgesehen sein, dass die erfindungsgemäßen Verbindungen mindestens eine Struktur der Formel (XIXa) und/oder (XIXb) umfassen, 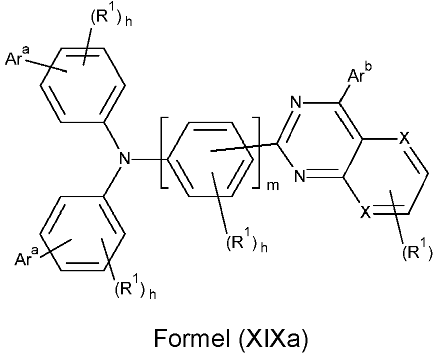 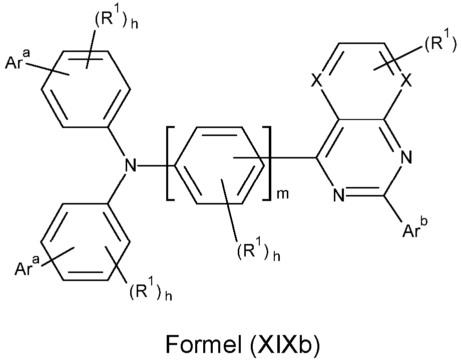 wobei die verwendeten Symbole m, Ar^{a}, Ar^{b}, R¹ und X die zuvor dargelegte Bedeutung aufweisen, i für 0, 1 oder 2, vorzugsweise für 0 oder 1 und h für 0, 1, 2, 3 oder 4, vorzugsweise für 0, 1 oder 2, besonders bevorzugt für 0 oder 1 steht.

Weiterhin sind Verbindungen, umfassend mindestens eine Struktur der Formel (XXa) und/oder (XXb), bevorzugt, 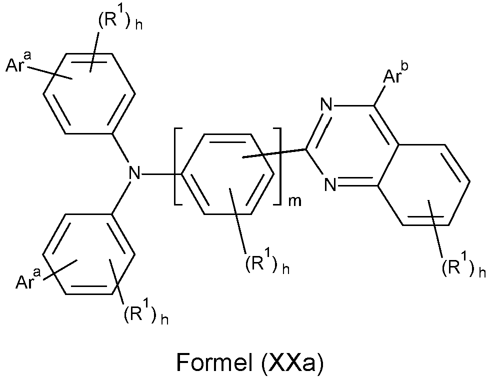 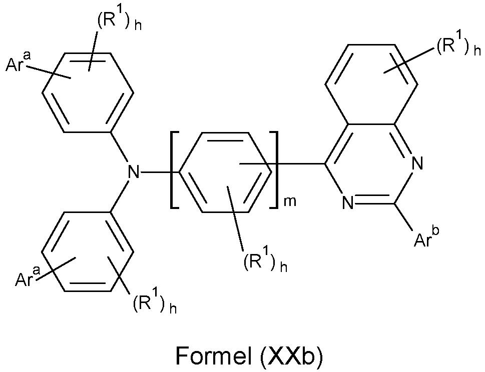 wobei die verwendeten Symbole m, Ar^{a}, Ar^{b} und R¹ die zuvordargelegte Bedeutung aufweisen und h für 0, 1, 2, 3 oder 4, vorzugsweise für 0, 1 oder 2, besonders bevorzugt für 0 oder 1 steht.

Vorzugsweise kann der Rest Ar^{a} gleich oder verschieden bei jedem Auftreten insbesondere in den Formeln (XIII), (XIVa), (XIVb), (XVa), (XVb), (XVIa), (XVIb), (XVIIa), (XVIIb), (XVIIIa), (XVIIIb), (XIXa), (XIXb), (XXa) und/oder (XXb) eine Spirobifluoren-, Fluoren-, Dibenzofuran- oder Dibenzothiophen-Gruppe umfassen oder mit dem Aryl- oder Heteroarylrest bilden, an den der Rest Ar^{a} bindet.

Weiterhin können die erfindungsgemäßen Verbindungen mindestens eine Struktur der Formel (XXIIa) oder (XXIIb) umfassen, 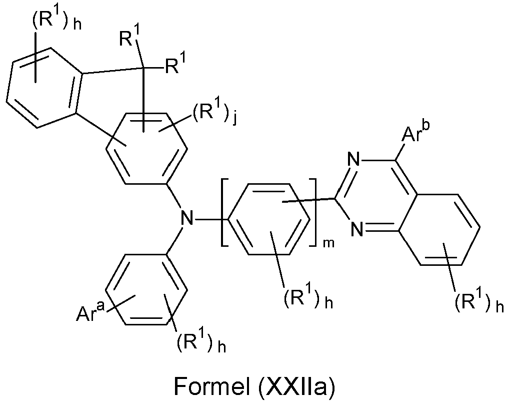 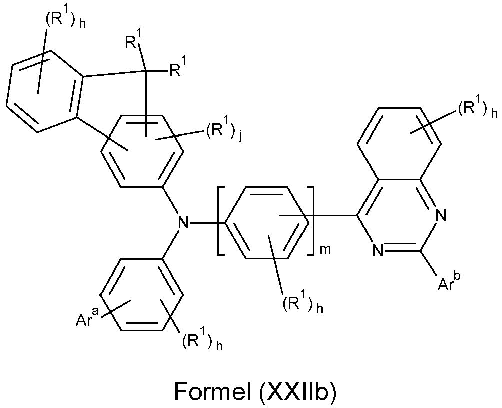 wobei die verwendeten Symbole m, Ar^{a}, Ar^{b} und R¹ die zuvor dargelegte Bedeutung aufweisen, j für 0, 1, 2 oder 3, vorzugsweise für 0, 1 oder 2, besonders bevorzugt für 0 oder 1 und h für 0, 1, 2, 3 oder 4, vorzugsweise für 0, 1 oder 2, besonders bevorzugt für 0 oder 1 steht.

In einer weiterhin bevorzugten Ausführungsform können die erfindungsgemäßen Verbindungen mindestens eine Struktur der Formel (XXIIIa), (XXIIIb), (XXIIIc), (XXIIId), (XXIIIe) oder (XXIIIf) aufweisen, 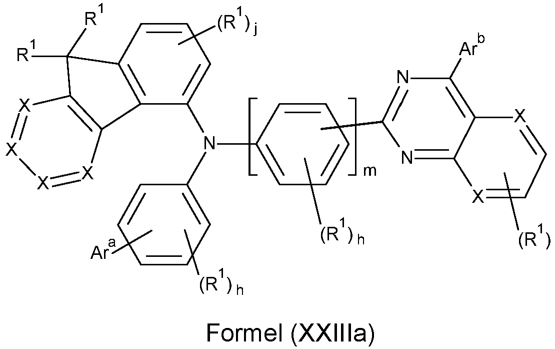 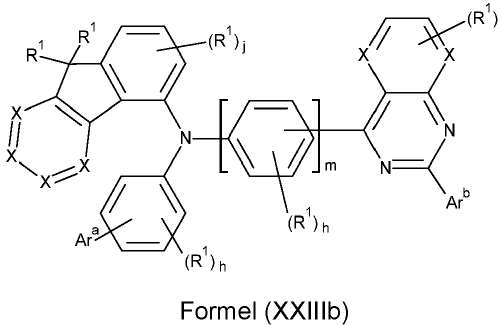 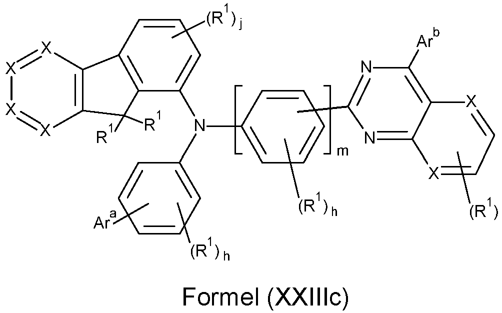 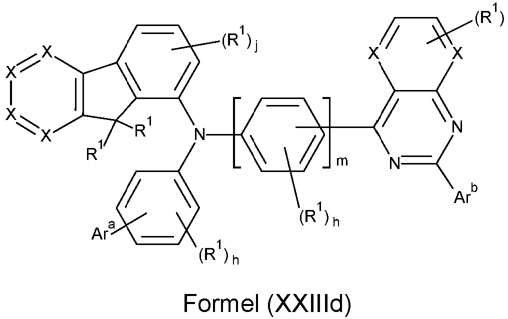 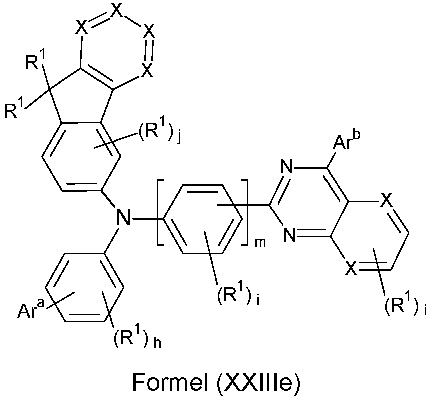 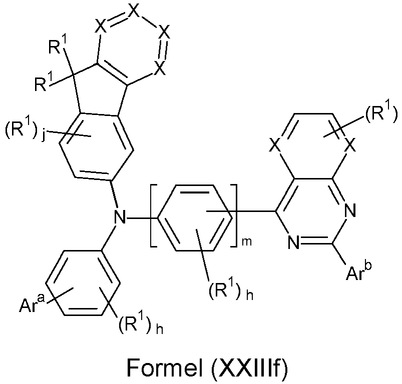 wobei die verwendeten Symbole m, Ar^{a}, Ar^{b}, R¹ und X die zuvor dargelegte Bedeutung aufweisen, i für 0, 1 oder 2, vorzugsweise für 0 oder 1, j für 0, 1, 2 oder 3, vorzugsweise für 0, 1 oder 2, besonders bevorzugt für 0 oder 1 und h für 0, 1, 2, 3 oder 4, vorzugsweise für 0, 1 oder 2, besonders bevorzugt für 0 oder 1 steht.

Weiterhin sind Verbindungen, umfassend mindestens eine Struktur der Formel (XXVa), (XXVb), (XXVc), (XXVd), (XXVe), (XXVf), (XXVg) oder (XXVh), bevorzugt, 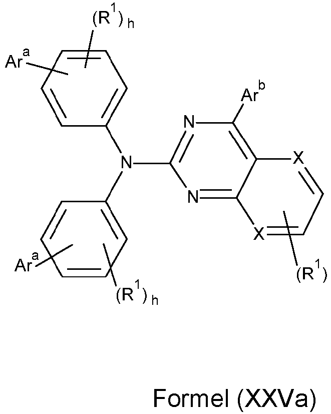 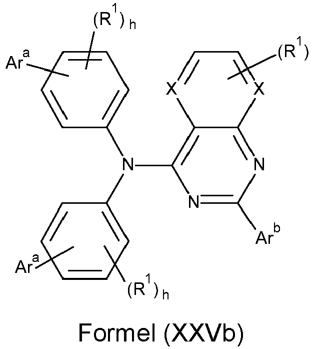 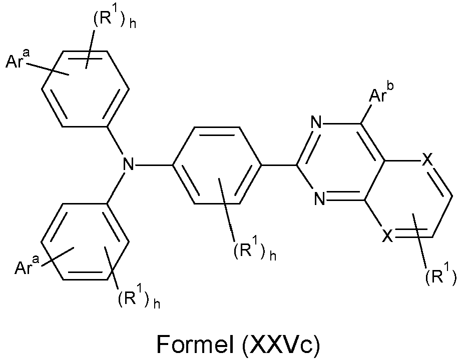 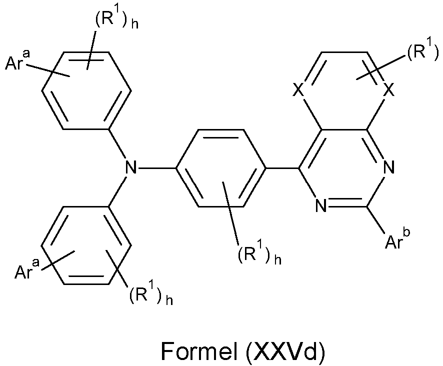 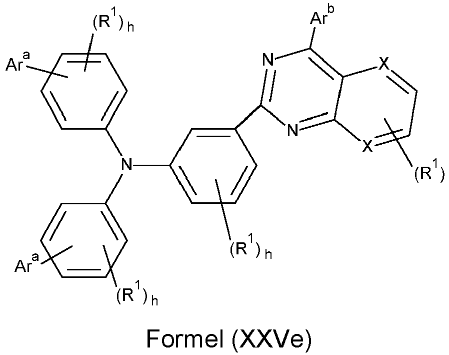 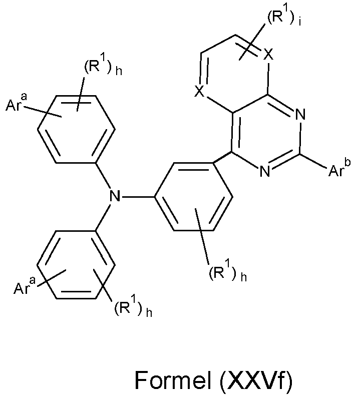 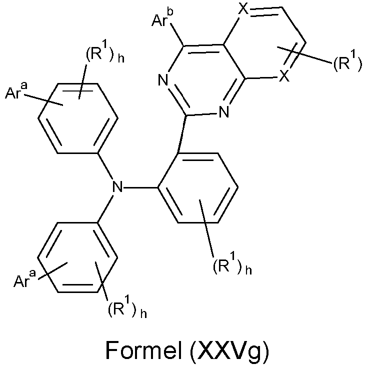 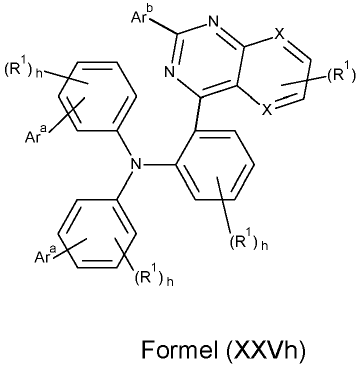 wobei die verwendeten Symbole Ar^{a}, Ar^{b}, R¹ und X die zuvor, dargelegte Bedeutung aufweisen, i für 0, 1 oder 2, vorzugsweise für 0 oder 1 und h für 0, 1, 2, 3 oder 4, vorzugsweise für 0, 1 oder 2, besonders bevorzugt für 0 oder 1 steht.

In einer bevorzugten Ausführungsform kann vorgesehen sein, dass der Index m 0 ist, so dass das Stickstoffatom der Diarylamingruppe direkt mit der Diazanaphthylgruppe verbunden ist. Daher sind insbesondere Verbindungen mit Strukturen der Formeln (XXVa) und/oder (XXVb) besonders bevorzugt.

In einer bevorzugten Ausführungsform der Erfindung stehen maximal vier Symbole X¹ für N pro Struktur der zuvor dargelegten Formeln. Besonders bevorzugt stehen maximal drei Symbole X¹ für N. Ganz besonders bevorzugt stehen genau zwei Symbole X¹ für N in Strukturen der zuvor dargelegten Formeln (IIIa), (IIIb) oder (XIII) und genau ein Symbol X¹ für N in Strukturen der zuvor dargelegten Formeln (IIIa), (IIIb), (IVa), (IVb), (XIVa), (XIVb), (XVa), (XVb), (XVIa) oder (XVIb).

In einer weiteren bevorzugten Ausführungsform der Erfindung stehen maximal vier Symbole X für N pro Struktur der zuvor dargelegten Formeln. Besonders bevorzugt stehen maximal zwei Symbole X für N. Ganz besonders bevorzugt steht keines der Symbole X für N in Strukturen der zuvor dargelegten Formeln.

Der Rest Ar^{a} stellt ein aromatisches oder heteroaromatisches Ringsystem mit 5 bis 30 aromatischen Ringatomen, welches durch einen oder mehrere Reste R¹ substituiert sein kann, dar. Aus Gründen der Vollständigkeit ist festzuhalten, dass der Rest Ar^{a} zusammen mit der Aryl- oder Heteroarylgruppe, an die der Rest Ar^{a} bindet, ein aromatisches oder heteroaromatisches Ringsystem bilden kann, wobei die Anzahl der Ringatome des gebildeten Ringsystems entsprechend steigen kann.

Vorzugsweise kann der Rest Ar^{a}, insbesondere in den Formeln (XXVa), (XXVb), (XXVc), (XXVd), (XXVe), (XXVf), (XXVg) oder (XXVh), eine Spirobifluoren-, Fluoren-, Dibenzofuran- oder Dibenzothiophen-Gruppe umfassen oder mit dem Aryl- oder Heteroarylrest bilden, an den der Rest Ar^{a} bindet.

Bevorzugt kann vorgesehen sein, dass der Rest Ar^{a} insbesondere in den Formeln (IIIa), (IIIb), (IVa), (IVb), (Va), (Vb), (VIa), (VIb), (Vlla), (Vllb), (Vllla), (Vlllb), (IXa), (IXb), (Xa), (Xb), (XIa), (XIb), (XIc), (XId), (XIe), (XIf), (XIIa), (XIIb), (XIII), (XIVa), (XIVb), (XVa), (XVb), (XVIa), (XVIb), (XVIIa), (XVIIb), (XVIIIa), (XVIIIb), (XIXa), (XIXb), (XXa), (XXb), (XXIa), (XXIb), (XXIIa), (XXIIb), (XXIIIa), (XXIIIb), (XXIIIc), (XXIIId), (XXIIIe), (XXIIIf), (XXIVa), (XXIVb), (XXVa), (XXVb), (XXVc), (XXVd), (XXVe), (XXVf), (XXVg) oder (XXVh) einen Aryl- oder Heteroarylrest mit 5 bis 30 aromatischen Ringatomen darstellt, welcher durch einen oder mehrere Reste R¹ substituiert sein kann, wobei der Rest Ar^{a} keine Carbazolgruppe umfasst oder mit der Aryl- oder Heteroarylgruppe bildet, an die Ar^{a} bindet, wobei dies Substituenten R¹, R² und R³, die an den Rest Ar^{a} gebunden sein können, einschließt. Besonders bevorzugt weist der Rest Ar^{a} höchstens 3, speziell bevorzugt höchstens 2 Reste R¹ auf und kann in einer ganz besonders bevorzugten Ausführungsform unsubstituiert sein.

Vorzugsweise ist die aromatische oder heteroaromatische Gruppe des durch das Symbol Ar^{a} dargestellten aromatischen oder heteroaromatischen Ringsystems direkt, d.h. über ein Atom der aromatischen oder heteroaromatischen Gruppe, an das jeweilige Atom der weiteren Gruppe gebunden, wobei das Symbol Ar^{a} besonders bevorzugt einen Aryl- oder Heteroarylrest darstellt.

In einer weiteren bevorzugten Ausführungsform der Erfindung steht Ar^{a} gleich oder verschieden bei jedem Auftreten für ein aromatisches oder heteroaromatisches Ringsystem, vorzugsweise einen Aryl- oder Heteroarylrest mit 5 bis 24 aromatischen Ringatomen, bevorzugt mit 6 bis 18 aromatischen Ringatomen, besonders bevorzugt für ein aromatisches Ringsystem, vorzugsweise einen Arylrest mit 6 bis 12 aromatischen Ringatomen bzw. ein heteroaromatisches Ringsystem, vorzugsweise eine Heteroarylgruppe mit 5 bis 13 aromatischen Ringatomen, das jeweils durch einen oder mehrere Reste R¹ substituiert sein kann, bevorzugt aber unsubstituiert ist, wobei R¹ die zuvor dargestellte Bedeutung aufweisen kann.

Der Rest Ar^{a} umfasst keine Carbazolgruppe oder bildet mit der Aryl- oder Heteroarylgruppe, an die Ar^{a} bindet, keine Carbazol-Gruppe, wobei dies Substituenten R¹, R² und R³, die an den Rest Ar^{a} gebunden sein können, einschließt. Vorzugsweise kann vorgesehen sein, dass Substituenten R¹, mit denen die Aryl- oder Heteroarylgruppe substituiert sein können, an die der Rest Ar^{a} bindet und die mit dem Stickstoffatom der Diarylamingruppe verbunden ist, keine Carbazol-Gruppe umfassen oder mit der Aryl- oder Heteroarylgruppe bilden, an die Ar^{a} bindet, wobei dies Substituenten R² und R³, die an den Rest R¹ gebunden sein können, einschließt.

Bevorzugt kann vorgesehen sein, dass der Rest Ar^{b} insbesondere in den Formeln (IIIa), (IIIb), (IVa), (IVb), (Va), (Vb), (VIa), (VIb), (Vlla), (Vllb), (Vllla), (Vlllb), (IXa), (IXb), (Xa), (Xb), (XIa), (XIb), (XIc), (XId), (XIe), (XIf), (XIIa), (XIIb), (XIII), (XIVa), (XIVb), (XVa), (XVb), (XVIa), (XVIb), (XVIIa), (XVIIb), (XVIIIa), (XVIIIb), (XIXa), (XIXb), (XXa), (XXb), (XXIa), (XXIb), (XXIIa), (XXIIb), (XXIIIa), (XXIIIb), (XXIIIc), (XXIIId), (XXIIIe), (XXIIIf), (XXIVa), (XXIVb), (XXVa), (XXVb), (XXVc), (XXVd), (XXVe), (XXVf), (XXVg) oder (XXVh) einen Aryl- oder Heteroarylrest mit 5 bis 30 aromatischen Ringatomen darstellt, welcher durch einen oder mehrere Reste R¹ substituiert sein kann. Besonders bevorzugt weist der Rest Ar^{b} höchstens 3, speziell bevorzugt höchstens 2 Reste R¹ auf und kann in einer ganz besonders bevorzugten Ausführungsform unsubstituiert sein.

Vorzugsweise ist die aromatische oder heteroaromatische Gruppe des durch das Symbol Ar^{b} dargestellten aromatischen oder heteroaromatischen Ringsystems direkt, d.h. über ein Atom der aromatischen oder heteroaromatischen Gruppe, an das jeweilige Atom der weiteren Gruppe gebunden, wobei das Symbol Ar^{b} besonders bevorzugt einen Aryl- oder Heteroarylrest darstellt.

In einer weiteren bevorzugten Ausführungsform der Erfindung steht Ar^{b} gleich oder verschieden bei jedem Auftreten für ein aromatisches oder heteroaromatisches Ringsystem, vorzugsweise einen Aryl- oder Heteroarylrest mit 5 bis 24 aromatischen Ringatomen, bevorzugt mit 6 bis 18 aromatischen Ringatomen, besonders bevorzugt für ein aromatisches Ringsystem, vorzugsweise einen Arylrest mit 6 bis 12 aromatischen Ringatomen bzw. ein heteroaromatisches Ringsystem, vorzugsweise eine Heteroarylgruppe mit 5 bis 13 aromatischen Ringatomen, das jeweils durch einen oder mehrere Reste R¹ substituiert sein kann, bevorzugt aber unsubstituiert ist, wobei R¹ die zuvordargestellte Bedeutung aufweisen kann.

Ferner kann vorgesehen sein, dass in den Strukturen gemäß Formeln (IIIa), (IIIb), (IVa), (IVb), (Va), (Vb), (VIa), (VIb), (Vlla), (Vllb), (Vllla), (Vlllb), (IXa), (IXb), (Xa), (Xb), (XIa), (XIb), (XIc), (XId), (XIe), (XIf), (Xlla), (XIIb), (XIII), (XIVa), (XIVb), (XVa), (XVb), (XVIa), (XVIb), (XVIIa), (XVIIb), (XVIIIa), (XVIIIb), (XIXa), (XIXb), (XXa), (XXb), (XXIa), (XXIb), (XXIIa), (XXIIb), (XXIIIa), (XXIIIb), (XXIIIc), (XXIIId), (XXIIIe), (XXIIIf), (XXIVa), (XXIVb), (XXVa), (XXVb), (XXVc), (XXVd), (XXVe), (XXVf), (XXVg) und/oder (XXVh) die Gruppe Ar^{b} eine Gruppe der Formel (Ar^{b}-1) darstellt, 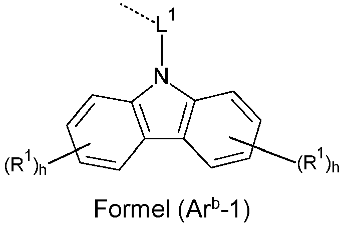 worin L' eine Bindung oder ein aromatisches oder heteroaromatisches Ringsystem mit 5 bis 30 aromatischen Ringatomen ist, welches durch einen oder mehrere Reste R¹ substituiert sein kann, das Symbol R¹ die zuvor dargelegte Bedeutung aufweist, h 0, 1, 2, 3 oder 4, vorzugsweise 0, 1 oder 2 ist und die gestrichelte Linie die Bindung darstellt.

Gemäß einer bevorzugten Ausgestaltung sind erfindungsgemäße Verbindungen durch Strukturen gemäß Formel (IIIa), (IIIb), (IVa), (IVb), (Va), (Vb), (VIa), (VIb), (Vlla), (Vllb), (Vllla), (Vlllb), (IXa), (IXb), (Xa), (Xb), (XIa), (XIb), (XIc), (XId), (XIe), (XIf), (Xlla), (Xllb), (XIII), (XIVa), (XIVb), (XVa), (XVb), (XVIa), (XVIb), (XVIIa), (XVIIb), (XVIIIa), (XVIIIb), (XIXa), (XIXb), (XXa), (XXb), (XXIa), (XXIb), (XXIIa), (XXIIb), (XXIIIa), (XXIIIb), (XXIIIc), (XXIIId), (XXIIIe), (XXIIIf), (XXIVa), (XXIVb), (XXVa), (XXVb), (XXVc), (XXVd), (XXVe), (XXVf), (XXVg) und/oder (XXVh) darstellbar. Vorzugsweise weisen erfindungsgemäße Verbindungen, insbesondere umfassend Strukturen gemäß Formel (IIIa), (IIIb), (IVa), (IVb), (Va), (Vb), (VIa), (VIb), (Vlla), (Vllb), (Vllla), (Vlllb), (IXa), (IXb), (Xa), (Xb), (XIa), (XIb), (XIc), (XId), (XIe), (XIf), (Xlla), (Xllb), (XIII), (XIVa), (XIVb), (XVa), (XVb), (XVIa), (XVIb), (XVIIa), (XVIIb), (XVIIIa), (XVIIIb), (XIXa), (XIXb), (XXa), (XXb), (XXIa), (XXIb), (XXIIa), (XXIIb), (XXIIIa), (XXIIIb), (XXIIIc), (XXIIId), (XXIIIe), (XXIIIf), (XXIVa), (XXIVb), (XXVa), (XXVb), (XXVc), (XXVd), (XXVe), (XXVf), (XXVg) und/oder (XXVh), ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Wenn X für CR¹ steht bzw. wenn die aromatischen und/oder heteroaromatischen Gruppen durch Substituenten R¹ substituiert sind, dann sind diese Substituenten R¹ bevorzugt gewählt aus der Gruppe bestehend aus H, D, CN, N(Ar¹)₂, einer geradkettigen Alkylgruppe mit 1, 2, 3 oder 4 C-Atomen oder einer verzweigten oder cyclischen Alkylgruppe mit 3 bis 8 C-Atomen einem aromatischen oder heteroaromatischen Ringsystem mit 5 bis 24 aromatischen Ringatomen, bevorzugt 6 bis 18 aromatischen Ringatomen, bevorzugter 6 bis 13 aromatischen Ringatomen, das jeweils mit einem oder mehreren Resten R² substituiert sein kann, bevorzugt aber unsubstituiert ist; dabei können optional zwei Substituenten R¹, die an dasselbe Kohlenstoffatom oder an benachbarte Kohlenstoffatome gebunden sind, ein monocyclisches, aliphatisches oder aromatisches Ringsystem bilden. Vorzugsweise stellt Ar¹ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten R² substituiert sein kann, vorzugsweise jedoch unsubstituiert ist.

Ganz besonders bevorzugt sind die Substituenten R¹ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten R² substituiert sein kann, bevorzugt aber unsubstituiert ist. Beispiele für geeignete Substituenten R¹ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R² substituiert sein können, bevorzugt aber unsubstituiert sind.

Bevorzugt bilden die Reste R¹ mit den Ringatomen der Arylgruppe oder Heteroarylgruppe, an die die Reste R¹ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten R² ein, die an die Reste R¹ gebunden sein können.

Ferner kann vorgesehen sein, dass die Reste R¹ mit weiteren Gruppen, kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten R² ein, die an die Reste R¹ gebunden sein können. Diese weiteren Gruppen können räumlich benachbart oder entfernt sein, wobei diese Gruppen die in den Formeln (IIIa) oder (IIIb) sowie deren bevorzugte Ausführungsformen dargestellten Ringsysteme und Reste einschließt.

Vorzugsweise ist die aromatische oder heteroaromatische Gruppe des durch das Symbol Ar¹ dargestellten aromatischen oder heteroaromatischen Ringsystems direkt, d. h. über ein Atom der aromatischen oder heteroaromatischen Gruppe, an das jeweilige Atom der weiteren Gruppe gebunden, wobei das Symbol Ar¹ besonders bevorzugt einen Aryl- oder Heteroarylrest darstellt.

In einer weiteren bevorzugten Ausführungsform der Erfindung steht Ar¹ gleich oder verschieden bei jedem Auftreten für ein aromatisches oder heteroaromatisches Ringsystem, vorzugsweise einen Aryl- oder Heteroarylrest mit 5 bis 24 aromatischen Ringatomen, bevorzugt mit 6 bis 18 aromatischen Ringatomen, besonders bevorzugt für ein aromatisches Ringsystem, vorzugsweise einen Arylrest mit 6 bis 12 aromatischen Ringatomen bzw. ein heteroaromatisches Ringsystem, vorzugsweise eine Heteroarylgruppe mit 5 bis 13 aromatischen Ringatomen, das jeweils durch einen oder mehrere Reste R² substituiert sein kann, bevorzugt aber unsubstituiert ist, wobei R² die zuvor dargestellte Bedeutung aufweisen kann.

Beispiele für geeignete Gruppen Ar¹ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R² substituiert sein können, bevorzugt aber unsubstituiert sind.

In einer weiteren Ausführungsform kann vorgesehen sein, dass die Reste Ar^{a} oder der Rest Ar^{b} jeweils durch Reste R² anstatt durch Reste R¹ substituiert sind.

Weiterhin kann vorgesehen sein, dass in einer Struktur gemäß Formel (IIIa), (IIIb), (IVa), (IVb), (Va), (Vb), (VIa), (VIb), (Vlla), (Vllb), (Vllla), (Vlllb), (IXa), (IXb), (Xa), (Xb), (XIa), (XIb), (XIc), (XId), (XIe), (XIf), (Xlla), (XIIb), (XIII), (XIVa), (XIVb), (XVa), (XVb), (XVIa), (XVIb), (XVIIa), (XVIIb), (XVIIIa), (XVIIIb), (XIXa), (XIXb), (XXa), (XXb), (XXIa), (XXIb), (XXIIa), (XXIIb), (XXIIIa), (XXIIIb), (XXIIIc), (XXIIId), (XXIIIe), (XXIIIf), (XXIVa), (XXIVb), (XXVa), (XXVb), (XXVc), (XXVd), (XXVe), (XXVf), (XXVg) und/oder (XXVh) mindestens ein Rest R¹, Ar^{a} oder Ar^{b} für eine Gruppe steht, die ausgewählt ist aus den Formeln (R¹-1) bis (R¹- 80) 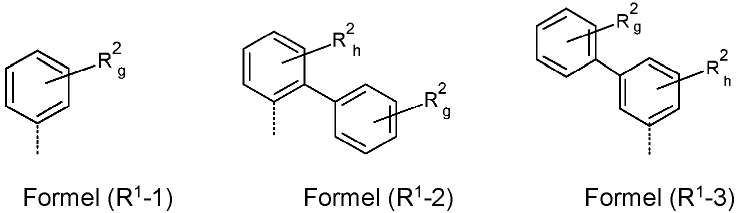 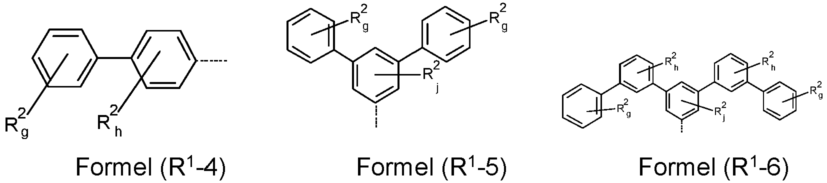 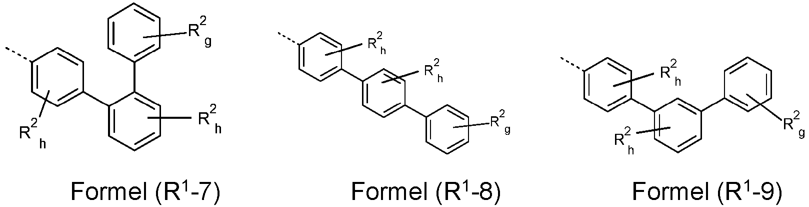 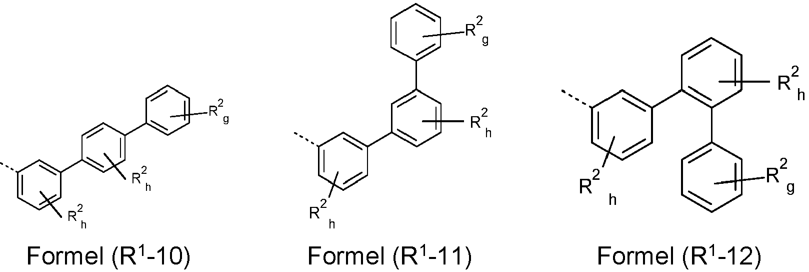 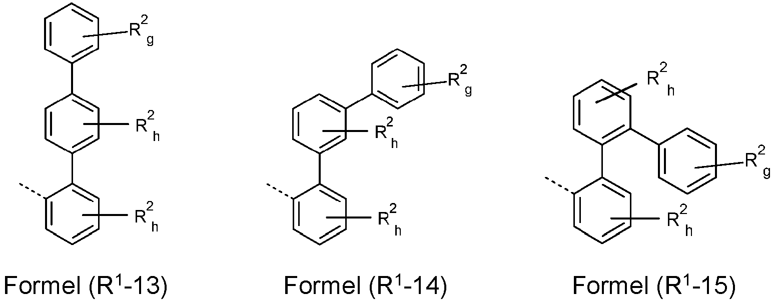 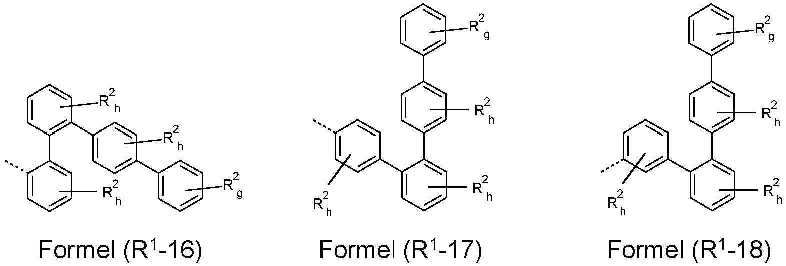 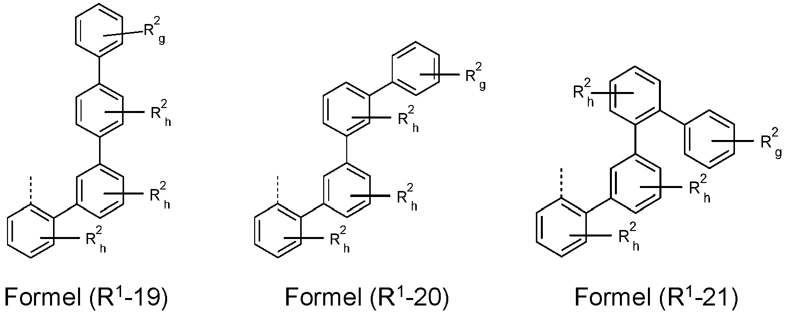 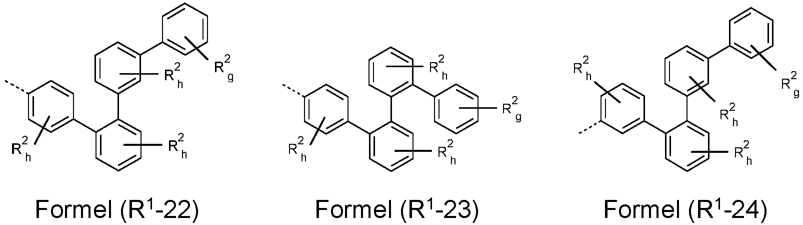 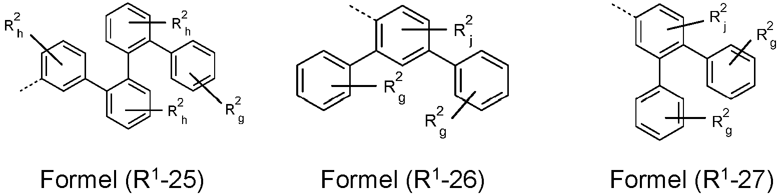 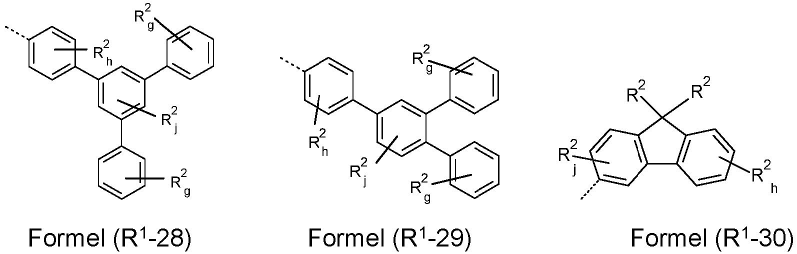 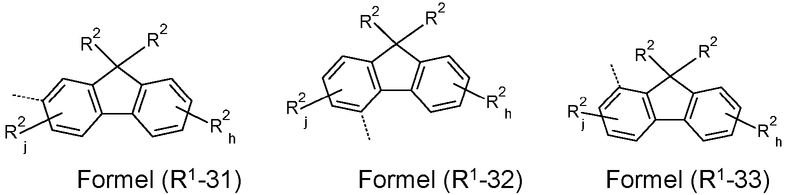 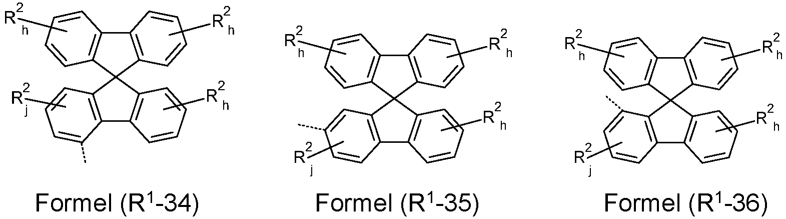 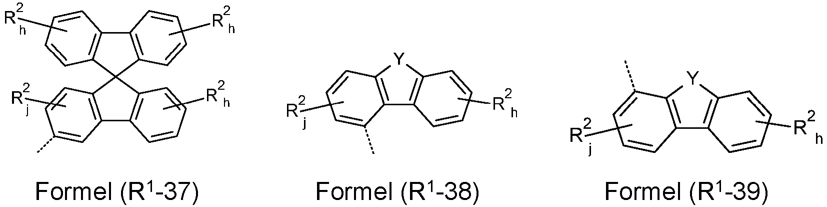 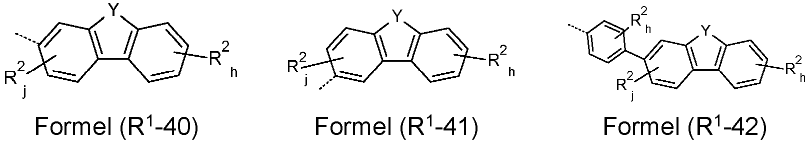 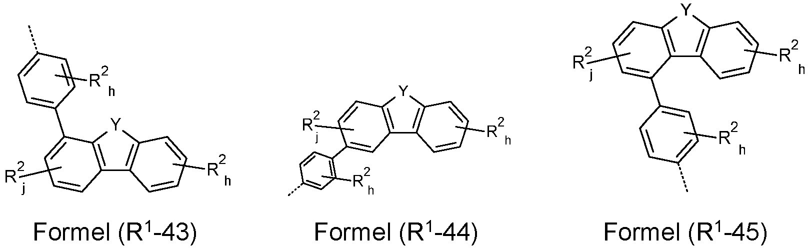 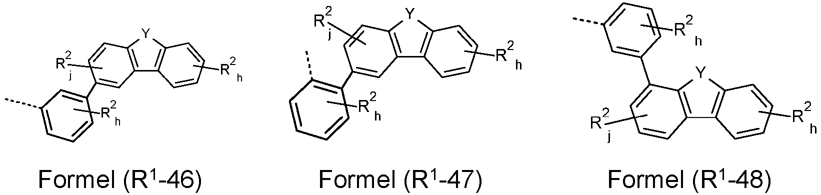 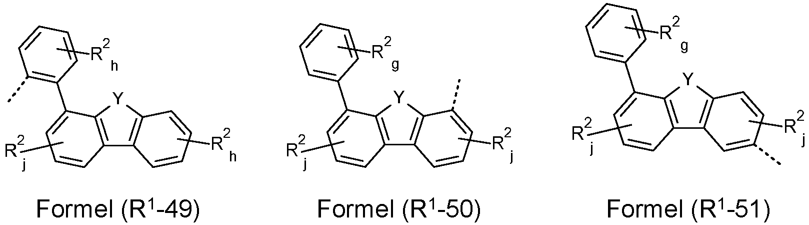 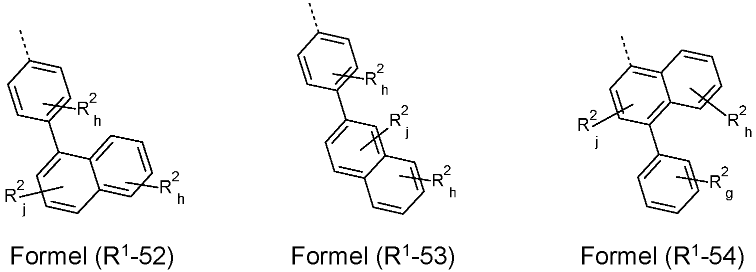 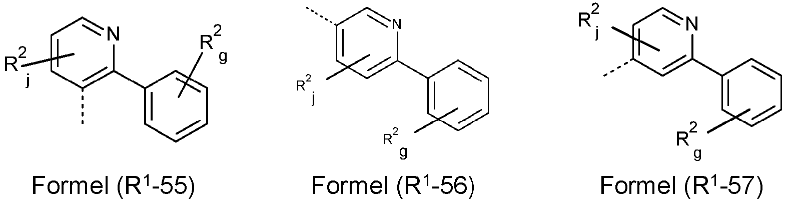 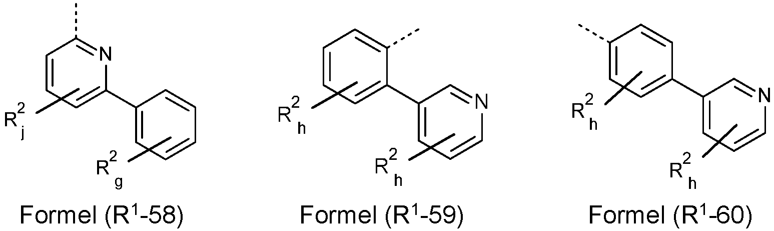 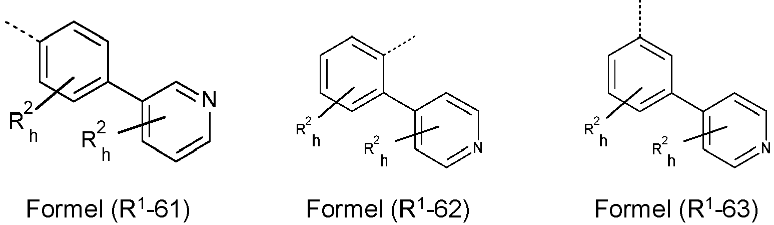 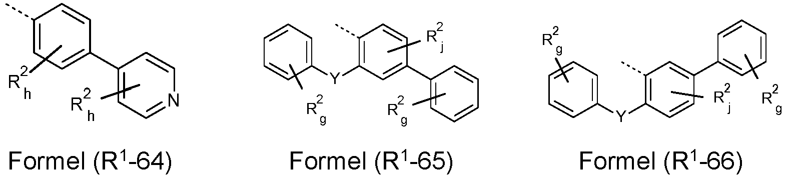 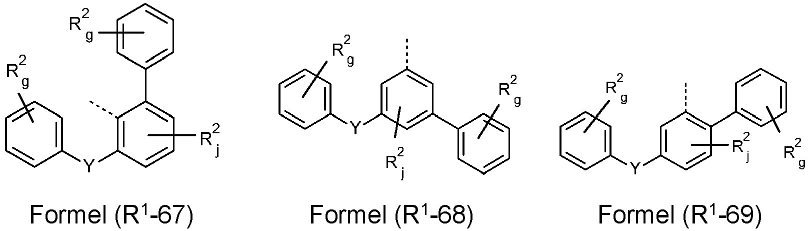 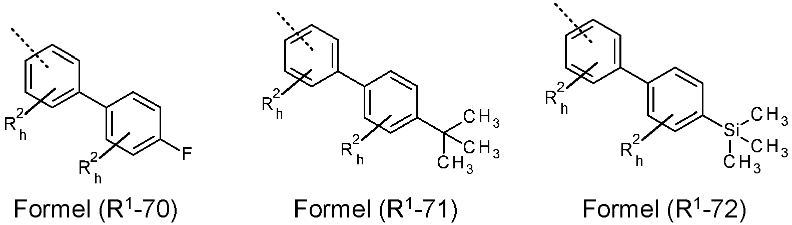 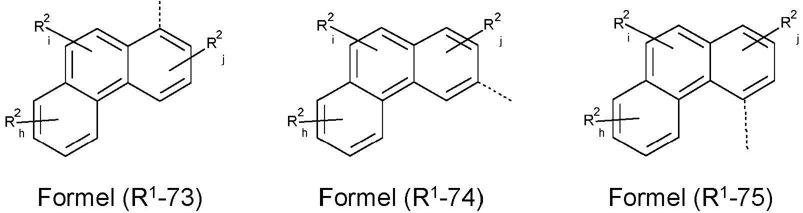 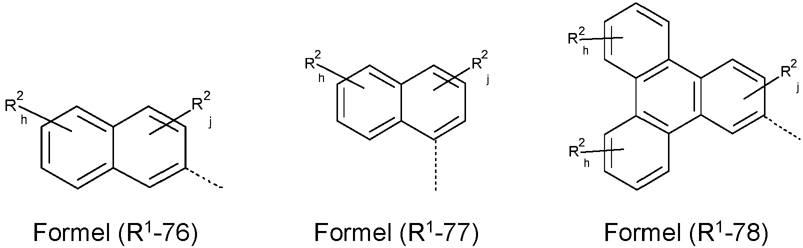 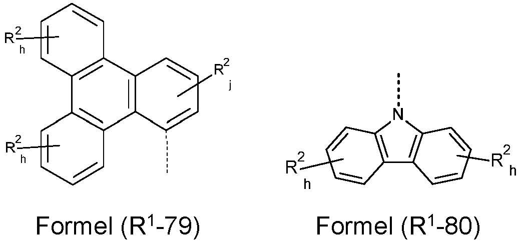
wobei für die verwendeten Symbole gilt:
   - Y: ist O, S oder NR², vorzugsweise O oder S;
   - i: ist bei jedem Auftreten unabhängig 0, 1 oder 2;
   - j: ist bei jedem Auftreten unabhängig 0, 1, 2 oder 3;
   - h: ist bei jedem Auftreten unabhängig 0, 1, 2, 3 oder 4;
   - g: ist bei jedem Auftreten unabhängig 0, 1, 2, 3, 4 oder 5;
   - R²: kann die zuvor genannte, insbesondere für Formel (I) genannte Bedeutung aufweisen, und
die gestrichelte Bindung markiert die Anbindungsposition.

Hierbei sind die Gruppen der Formeln R¹-1 bis R¹-51 bevorzugt, wobei die Gruppen R¹-1, R¹-3, R¹-5, R¹-6, R¹-15, R¹-29, R¹-30, R¹-31, R¹-32, R¹-33, R¹-38, R¹-39, R¹-40, R¹-41, R¹-42, R¹-43, R¹-44 und/oder R¹-45 besonders bevorzugt sind.

Vorzugsweise kann vorgesehen sein, dass die Summe der Indices i, j, h und g in den Strukturen der Formeln (R¹-1) bis (R¹-80) jeweils höchstens 3, vorzugsweise höchstens 2 und besonders bevorzugt höchstens 1 beträgt.

Bevorzugt bilden die Reste R² in den Formeln (R¹-1) bis (R¹-80) mit den Ringatomen der Arylgruppe oder Heteroarylgruppe, an die die Reste R² gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten R³ ein, die an die Reste R² gebunden sein können.

In den Formeln R¹-1 bis R¹-80 werden bevorzugte Gruppen Ar^{a} und Ar^{b} mit Resten R² dargestellt. Selbstverständlich stellen Reste, die im Wesentlichen den Formeln R¹-1 bis R¹-80 entsprechen, jedoch Reste R¹ anstatt R² als Substituenten aufweisen, bevorzugte Gruppen Ar^{a} und Ar^{b} dar. Hierbei gelten die zuvor genannten Bevorzugungen für diese Formeln R¹-1 bis R¹-80, die jedoch Reste R¹ anstatt R² als Substituenten aufweisen, ebenfalls.

Bevorzugt kann die Gruppe L' mit dem Diazanaphthalin-Rest, an den die Gruppe L' gemäß Formel (Ar^{b}-1) gebunden ist, und mit der Carbazol-gruppe der Formel (Ar^{b}-1) eine durchgängige Konjugation ausbilden. Weitere Bevorzugungen der Gruppe L' in Formel (Ar^{b}-1) wurden zuvor im Zusammenhang mit der unter anderem in Formeln (I), (IIa), (IIb), (IIIa), (IIIb), (IVa), (IVb), (Va), (Vb), (VIa), (VIb), (Vlla), (Vllb), (Vllla), (Vlllb), (IXa), (IXb), (Xa), (Xb), (XIa), (XIb), (XIc), (XId), (XIe), (XIf), (Xlla), (Xllb) dargelegte Gruppe L' beschrieben, die auch für die Formel (Ar^{b}-1) gelten.

Ferner kann vorgesehen sein, dass die Gruppe L durch Reste R² anstatt durch Reste R¹ substituiert ist.

Bevorzugt sind Verbindungen umfassend Strukturen der Formeln (I), (IIa), (IIb), (IIIa), (IIIb), (IVa), (IVb), (Va), (Vb), (VIa), (VIb), (Vlla), (Vllb), (Vllla), (Vlllb), (IXa), (IXb), (Xa), (Xb), (XIa), (XIb), (XIc), (XId), (XIe), (XIf), (Xlla), (XIIb) und (Ar^{b}-1), in denen die Gruppe L beziehungsweise L' für eine Bindung oder für eine Gruppe steht, die ausgewählt ist aus den Formeln (L¹-1) bis (L¹-108), 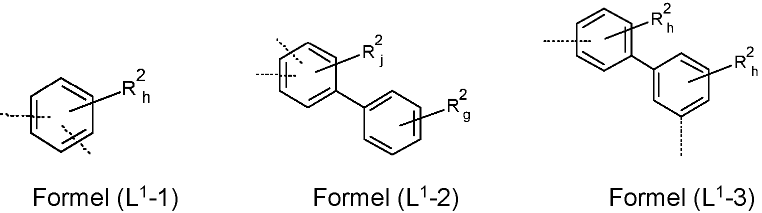 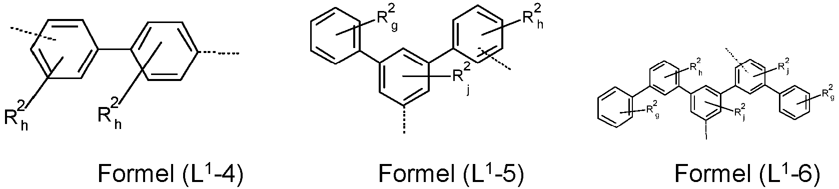 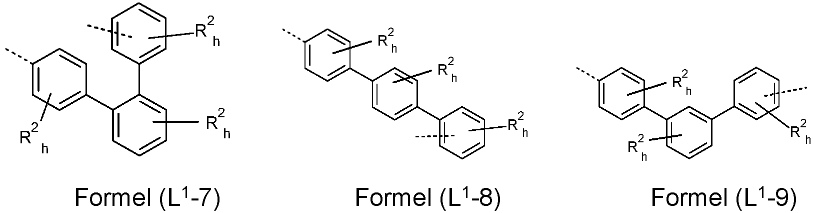 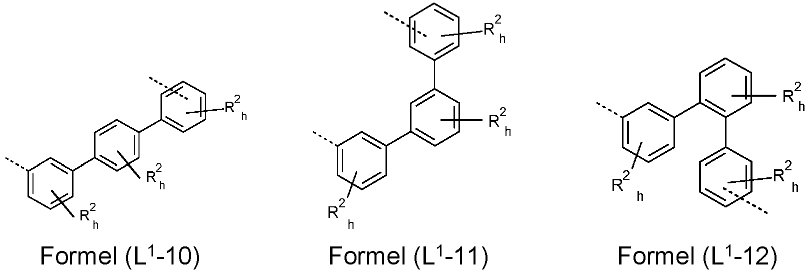 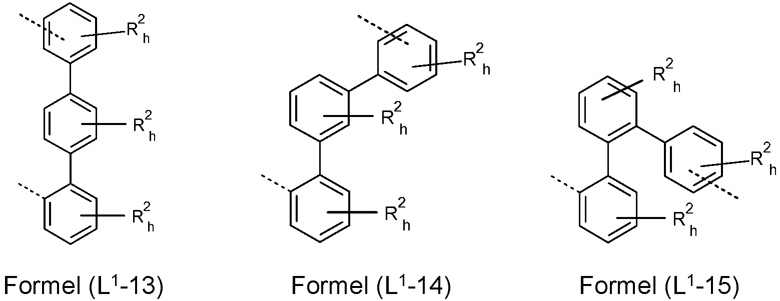 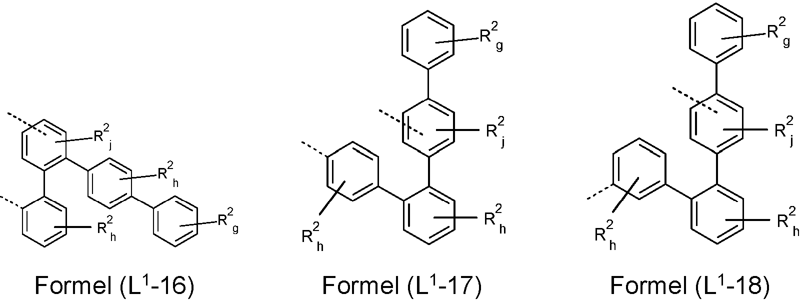 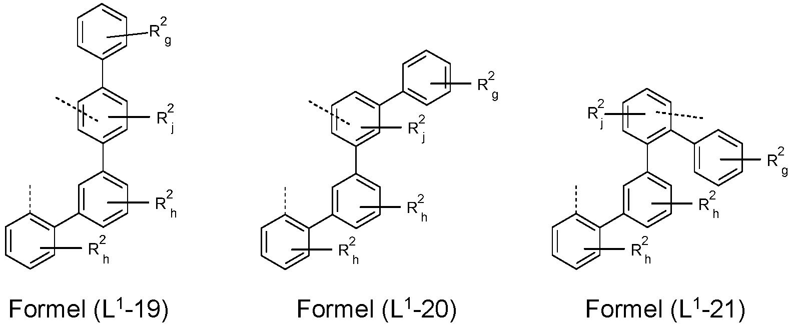 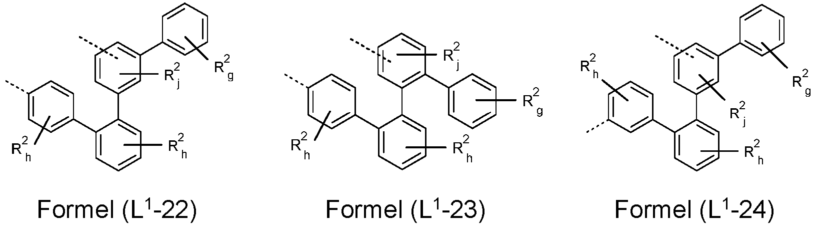 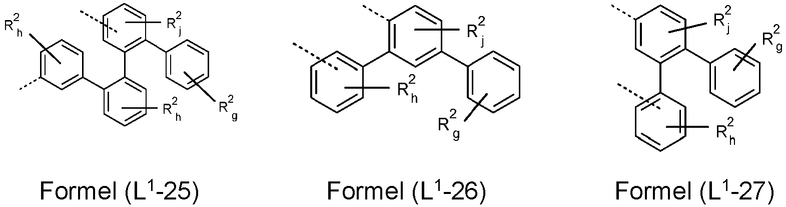 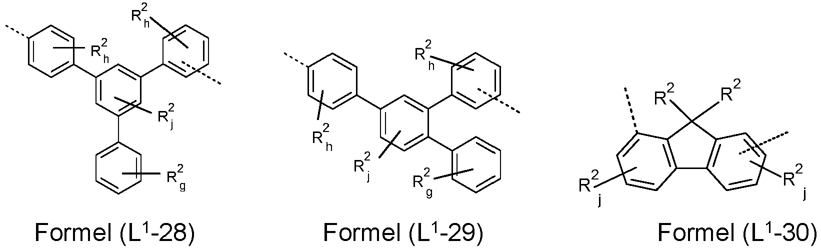 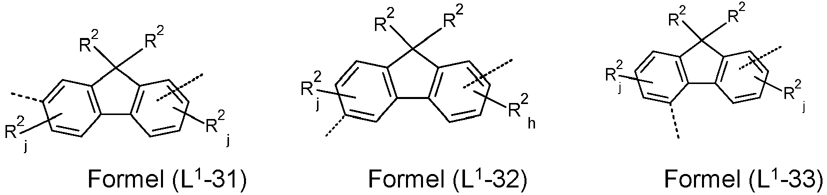 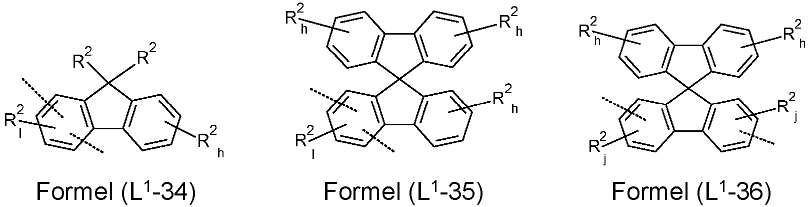 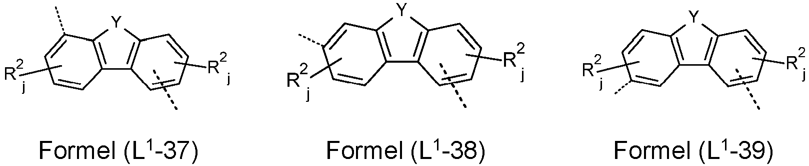 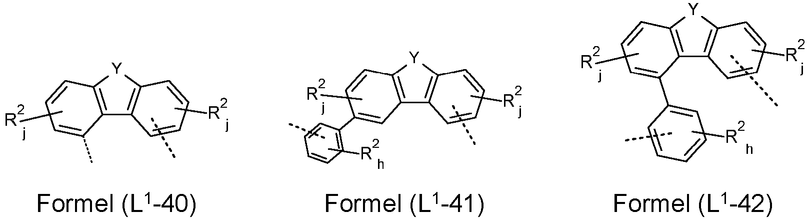 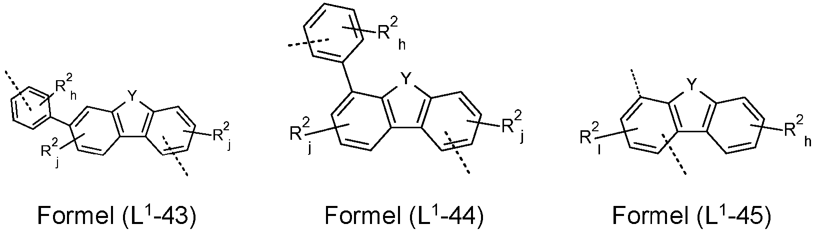 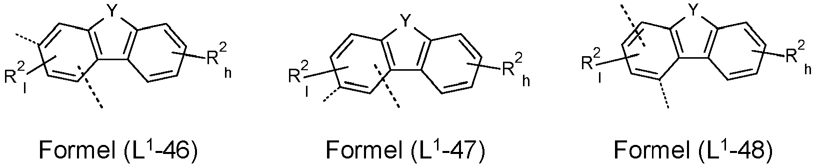 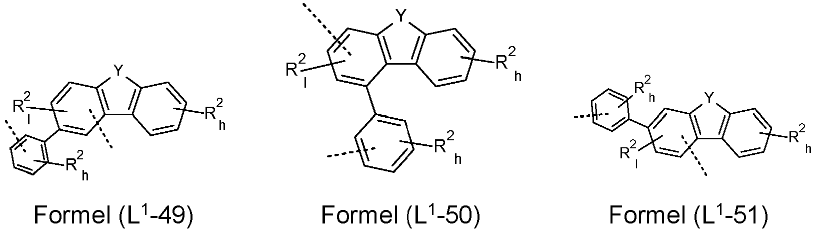 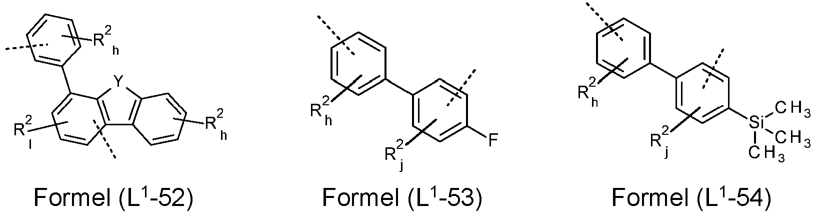 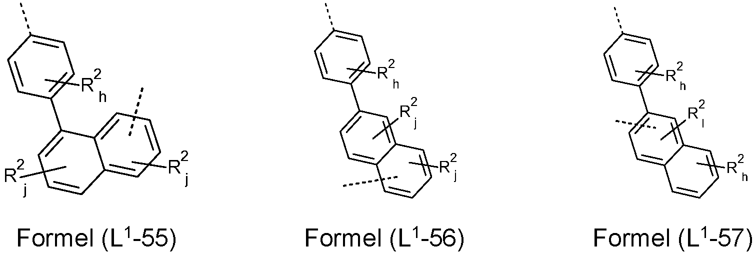 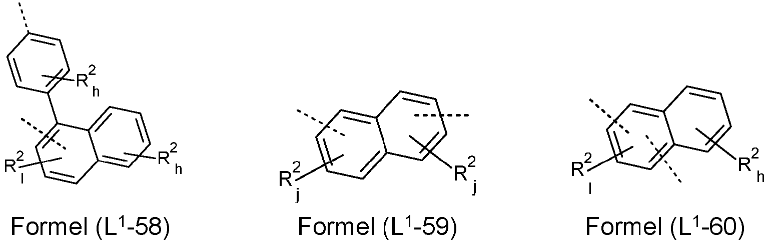 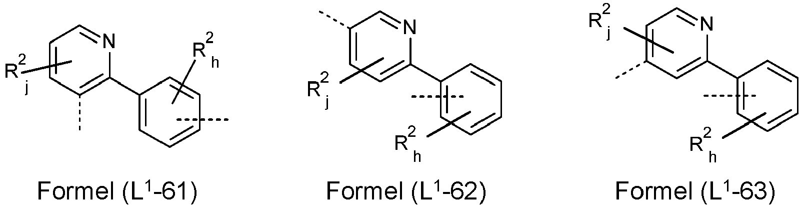 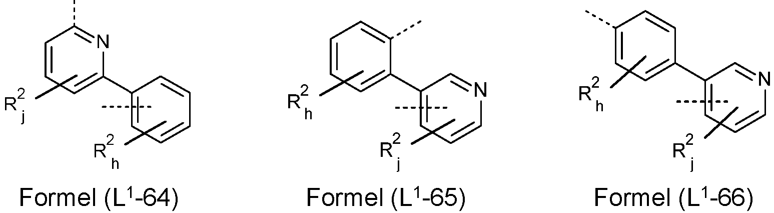 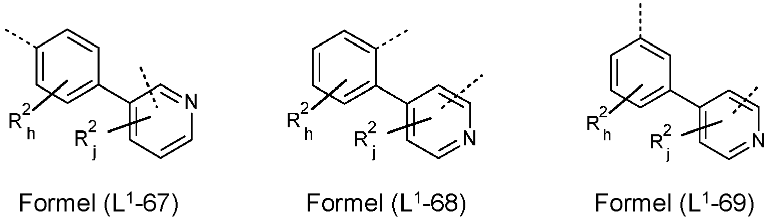 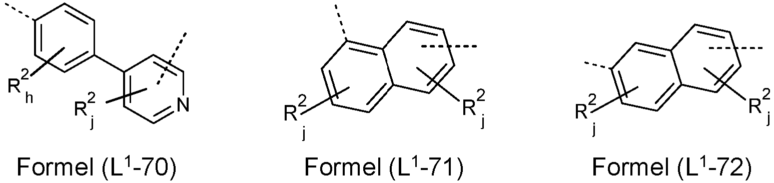 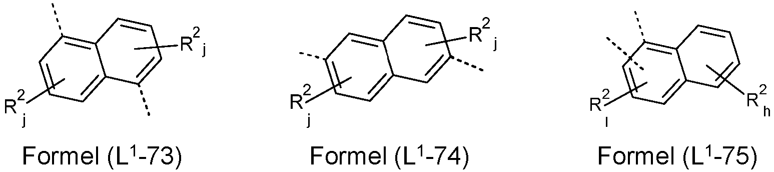 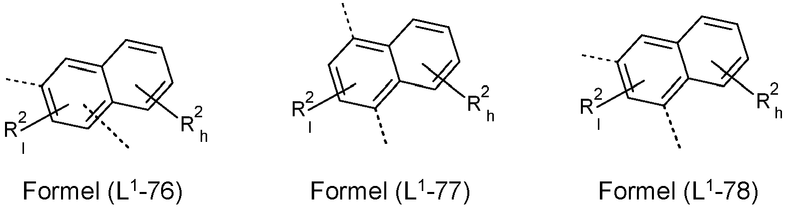 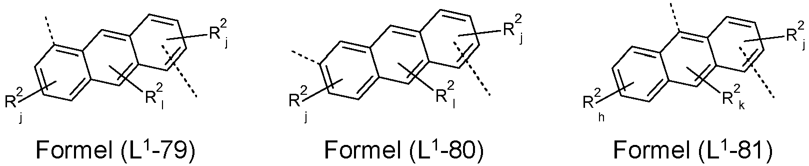 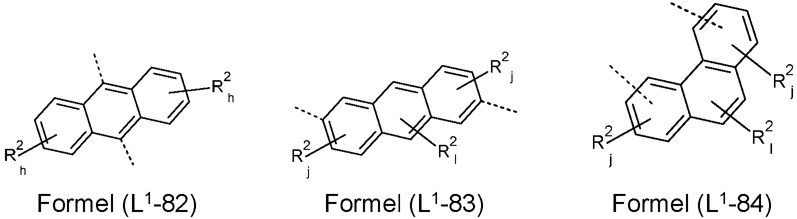 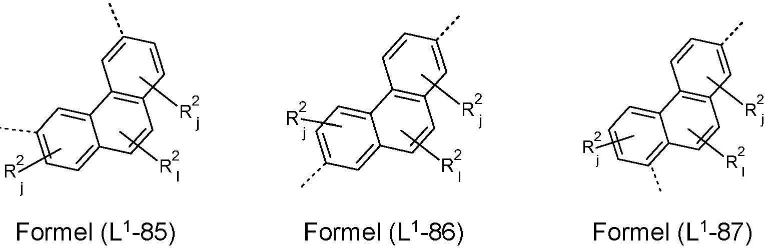 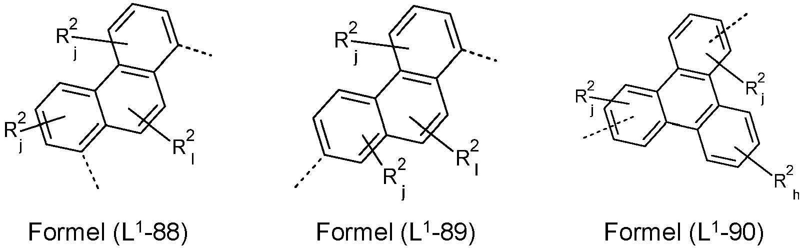 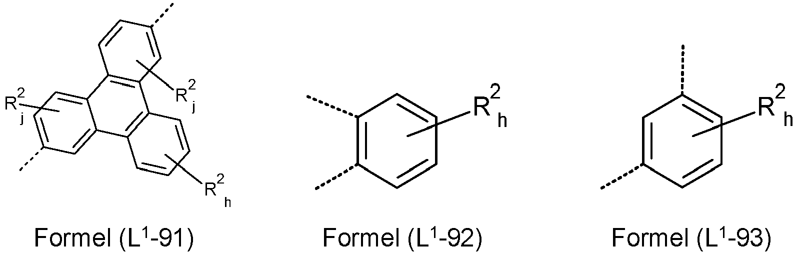 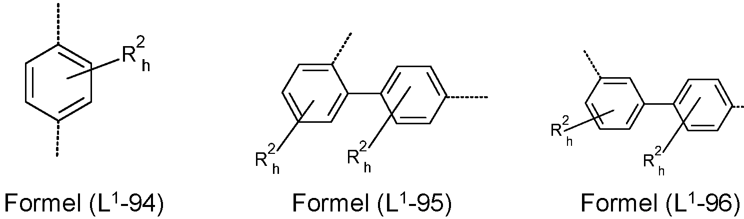 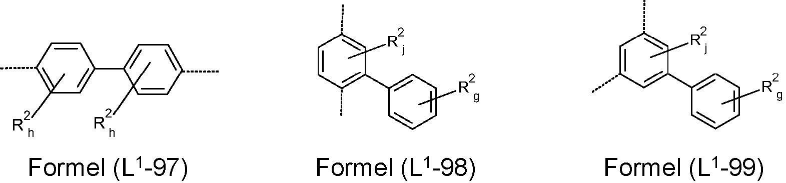 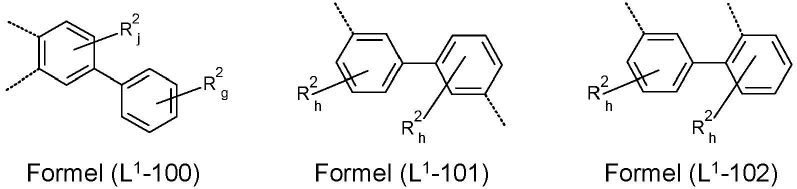 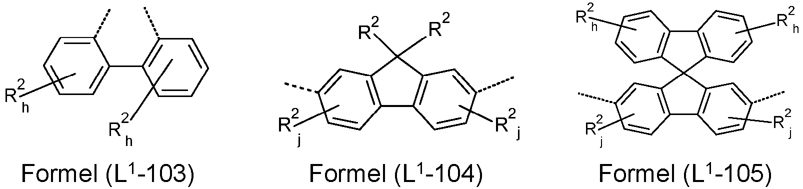 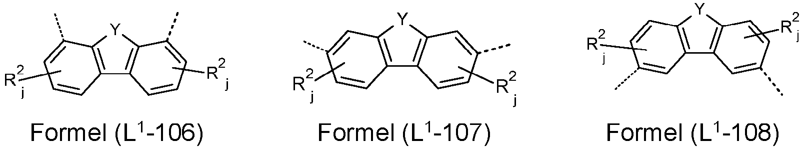 wobei die gestrichelten Bindungen jeweils die Anbindungspositionen markieren, der Index k 0 oder 1 ist, der Index I 0, 1 oder 2 ist, der Index j bei jedem Auftreten unabhängig 0, 1, 2 oder 3 ist; der Index h bei jedem Auftreten unabhängig 0, 1, 2, 3 oder 4 ist, der Index g 0, 1, 2, 3, 4 oder 5 ist; das Symbol Y O, S oder NR², vorzugsweise O oder S ist; und das Symbol R² die zuvor genannte Bedeutung aufweist.

Vorzugsweise kann vorgesehen sein, dass die Summe der Indices k, l, g, h und j in den Strukturen der Formel (L¹-1) bis (L¹-108) jeweils höchstens 3, vorzugsweise höchstens 2 und besonders bevorzugt höchstens 1 beträgt.

Bevorzugte erfindungsgemäße Verbindungen umfassen eine Gruppe L, die eine Bindung darstellt, in denen also m = 0 ist, oder die ausgewählt ist aus einer der Formeln (L¹-1) bis (L¹-78) und/oder (L¹-92) bis (L¹-108), bevorzugt der Formeln (L¹-1) bis (L¹-54) und/oder (L¹-92) bis (L¹-108), speziell bevorzugt der Formeln (L¹-1) bis (L¹-29) und/oder (L¹-92) bis (L¹-103). Mit Vorteil kann die Summe der Indices k, l, g, h und j in den Strukturen der Formeln (L¹-1) bis (L¹-78) und/oder (L¹-92) bis (L¹-108), bevorzugt der Formeln (L¹-1) bis (L¹-54) und/oder (L¹-92) bis (L¹-108), speziell bevorzugt der Formeln (L¹-1) bis (L¹-29) und/oder (L¹-92) bis (L¹-103) jeweils höchstens 3, vorzugsweise höchstens 2 und besonders bevorzugt höchstens 1 betragen.

Bevorzugt bilden die Reste R² in den Formeln (L¹-1) bis (L¹-108) mit den Ringatomen der Arylgruppe oder Heteroarylgruppe, an die die Reste R² gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten R³ ein, die an die Reste R² gebunden sein können.

In den Formeln L¹-1 bis L¹-108 werden bevorzugte Gruppen L mit Resten R² dargestellt. Selbstverständlich stellen Reste, die im Wesentlichen den Formeln L¹-1 bis L¹-108 entsprechen, jedoch Reste R¹ anstatt R² als Substituenten aufweisen, bevorzugte Gruppen L dar. Hierbei gelten die zuvor genannten Bevorzugungen für diese Formeln L¹-1 bis L¹-108, die jedoch Reste R¹ anstatt R² als Substituenten aufweisen, ebenfalls.

Wenn die erfindungsgemäße Verbindung mit aromatischen oder heteroaromatischen Gruppen R¹ bzw. R² substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

In einer weiteren bevorzugten Ausführungsform der Erfindung ist R², beispielsweise bei einer Struktur gemäß Formel (IIIa) oder (IIIb) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, einem aliphatischen Kohlenwasserstoffrest mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Bevorzugt bilden die Reste R² mit den Ringatomen der Arylgruppe oder Heteroarylgruppe, an die die Reste R² gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten R³ ein, die an die Reste R² gebunden sein können.

In einer weiteren bevorzugten Ausführungsform der Erfindung ist R³, beispielsweise bei einer Struktur gemäß Formel (IIIa) oder (IIIb) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, CN, einem aliphatischen Kohlenwasserstoffrest mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Erfindungsgemäß sind Verbindungen der Formel (A) 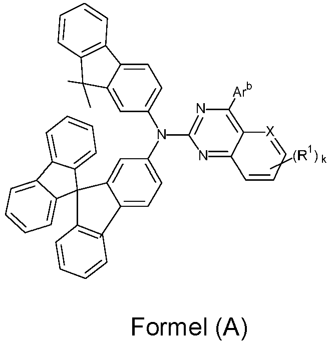 ausgenommen, wobei die verwendeten Symbole die zuvor, insbesondere für Formel (I) dargelegte Bedeutungen aufweisen.

Vorzugsweise sind Verbindungen der Formel (A-1) 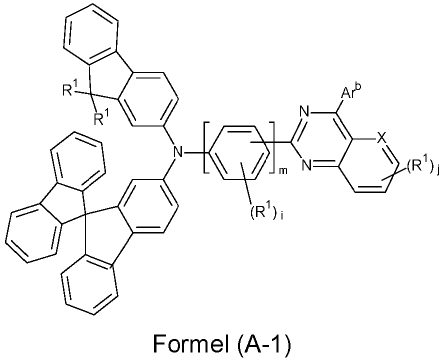 ausgenommen, wobei die verwendeten Symbole die zuvor, insbesondere für Formel (I) beziehungsweise Formeln (XXIa) und (XXIb) dargelegte Bedeutungen aufweisen.

Besonders bevorzugt sind Verbindungen der Formel (A-2) 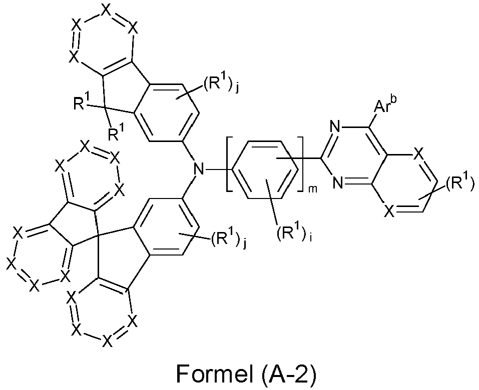 ausgenommen, wobei die verwendeten Symbole die zuvor dargelegte Bedeutungen aufweisen.

In einer besonderen Ausgestaltung der vorliegenden Erfindung weist eine Verbindung gemäß Formel (IIIa) oder (IIIb), oder einer bevorzugten Ausgestaltung dieser Formel höchstens eine Gruppe Ar^{a} auf, die eine 4-Spirobifluoren-, 4-Fluoren-, 1-Dibenzofuran- oder 1-Dibenzothiophen-Gruppe umfasst oder mit dem Aryl- oder Heteroarylrest bildet, an den der Rest Ar^{a} bindet. Insbesondere bevorzugt weist eine Verbindung gemäß Formel (IIIa) oder (IIIb), oder einer bevorzugten Ausgestaltung dieser Formel höchstens eine Gruppe Ar^{a} auf, die eine Spirobifluoren-, Fluoren-, Dibenzofuran- oder Dibenzothiophen-Gruppe umfasst oder mit dem Aryl- oder Heteroarylrest bildet, an den der Rest Ar^{a} bindet.

Ferner kann bevorzugt vorgesehen sein, dass mindestens einer der Reste Ar^{a} gemäß den Formeln(IIIa), (IIIb), (XXIa), (XXIb), (XXIVa) oder (XXIVb) oder einer bevorzugten Ausgestaltung dieser Formel unverbrückt ist, so dass genau eine Bindung zum Arylrest vorhanden ist, der mit dem Stickstoffatom der Diarylgruppe verbunden ist.

Beispiele für geeignete erfindungsgemäße Verbindungen sind die nachstehend gezeigten Strukturen gemäß den folgenden Formeln 1 bis 131:

| | | |
|---|---|---|
| 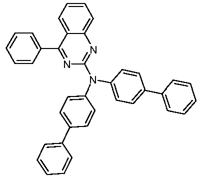 | 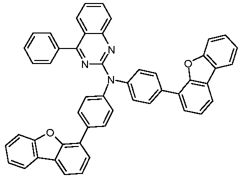 | 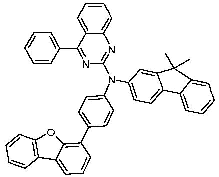 |
| Formel 1 | Formel 2 | Formel 3 |
| 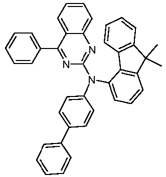 | 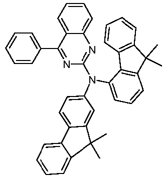 | 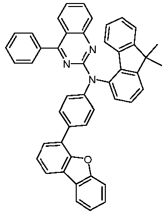 |
| Formel 4 | Formel 5 | Formel 6 |
| 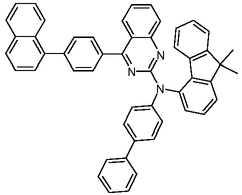 | 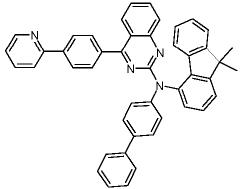 | 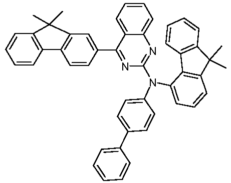 |
| Formel 7 | Formel 8 | Formel 9 |
| 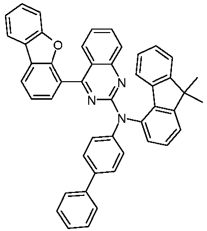 | 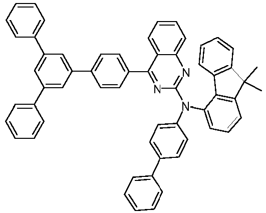 | 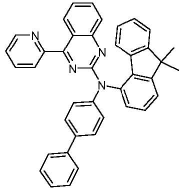 |
| Formel 10 | Formel 11 | Formel 12 |
| 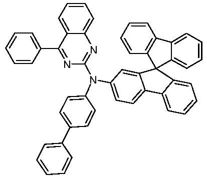 | 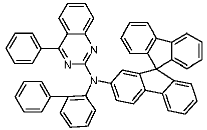 | 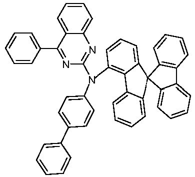 |
| Formel 13 | Formel 14 | Formel 15 |
| 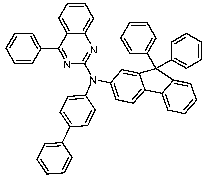 | 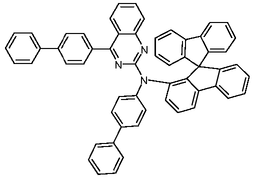 | 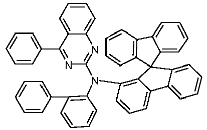 |
| Formel 16 | Formel 17 | Formel 18 |
| 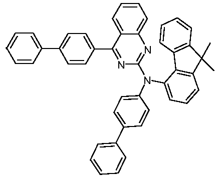 | 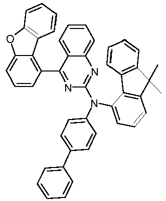 | 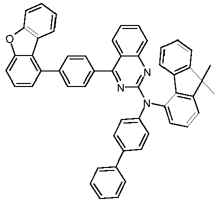 |
| Formel 19 | Formel 20 | Formel 21 |
| 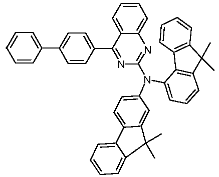 | 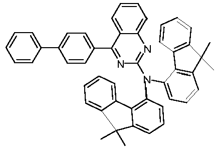 | 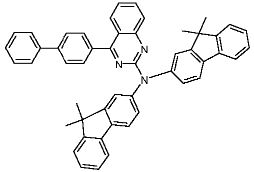 |
| Formel 22 | Formel 23 | Formel 24 |
| | 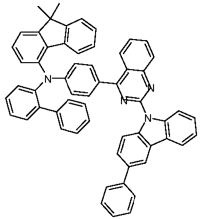 | 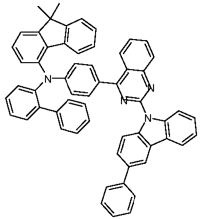 |
| | Formel 26 | Formel 27 |
| 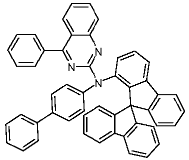 | 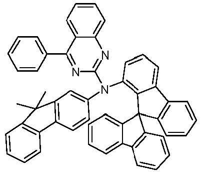 | 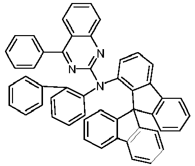 |
| Formel 28 | Formel 29 | Formel 30 |
| 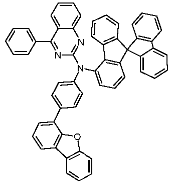 | 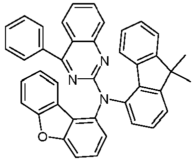 | 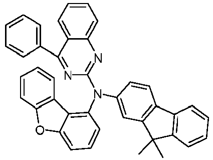 |
| Formel 31 | Formel 32 | Formel 33 |
| 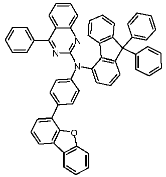 | 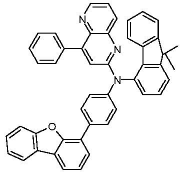 | 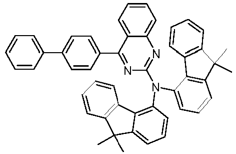 |
| Formel 34 | Formel 35 | Formel 36 |
| 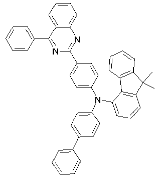 | 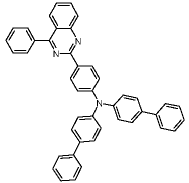 | 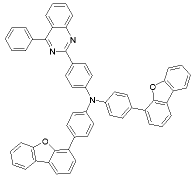 |
| Formel 37 | Formel 38 | Formel 39 |
| 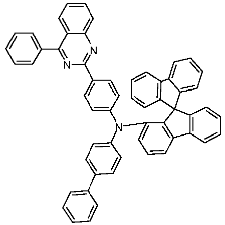 | 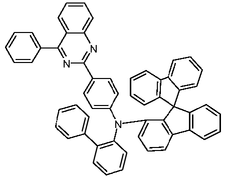 | 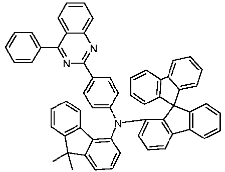 |
| Formel 40 | Formel 41 | Formel 42 |
| 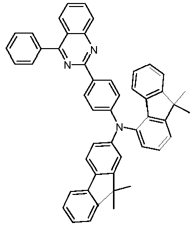 | 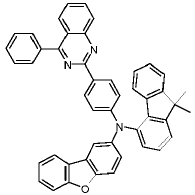 | 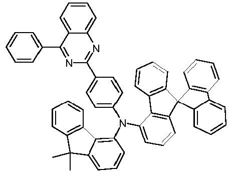 |
| Formel 43 | Formel 44 | Formel 45 |
| 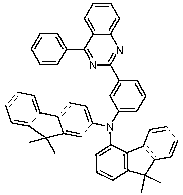 | 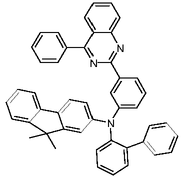 | 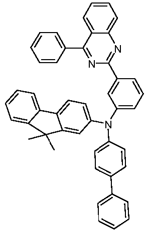 |
| Formel 46 | Formel 47 | Formel 48 |
| 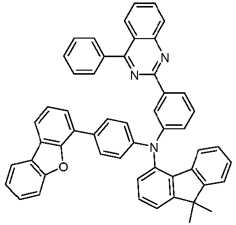 | 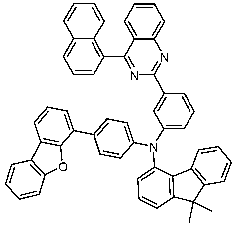 | 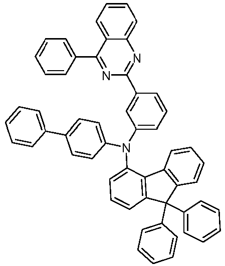 |
| Formel 49 | Formel 50 | Formel 51 |
| 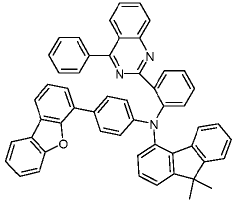 | 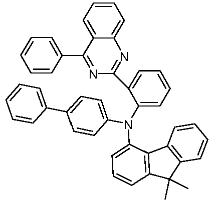 | 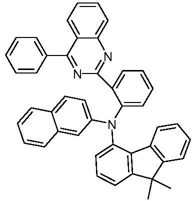 |
| Formel 52 | Formel 53 | Formel 54 |
| 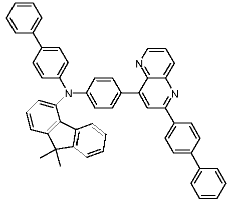 | 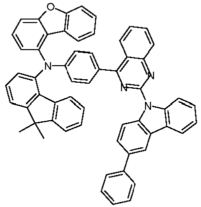 | 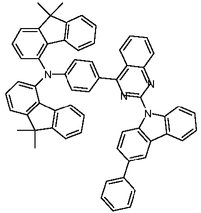 |
| Formel 55 | Formel 56 | Formel 57 |
| 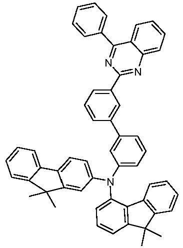 | 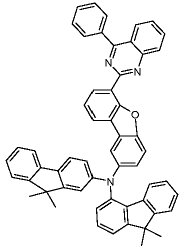 | 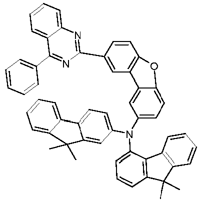 |
| Formel 58 | Formel 59 | Formel 60 |
| 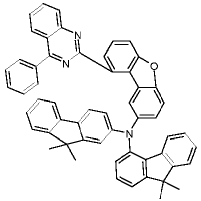 | 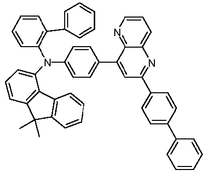 | 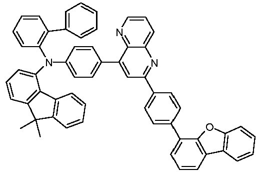 |
| Formel 61 | Formel 62 | Formel 63 |
| 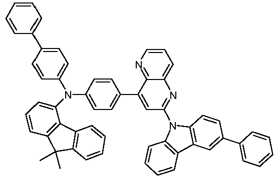 | 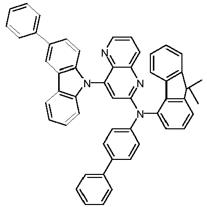 | 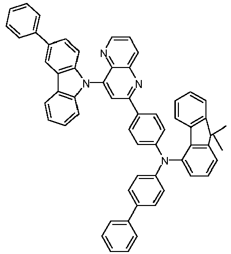 |
| Formel 64 | Formel 65 | Formel 66 |
| 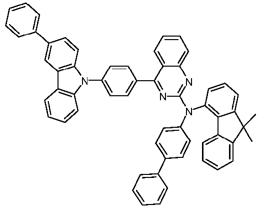 | 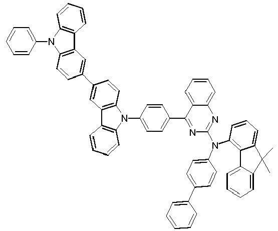 | 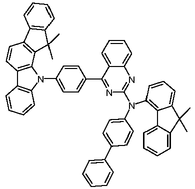 |
| Formel 67 | Formel 68 | Formel 69 |
| 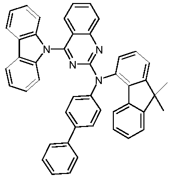 | 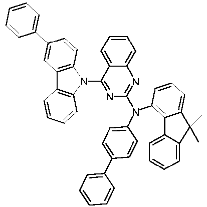 | 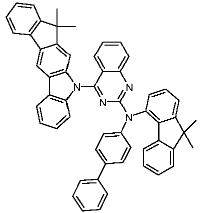 |
| Formel 70 | Formel 71 | Formel 72 |
| 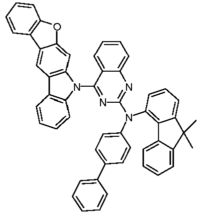 | 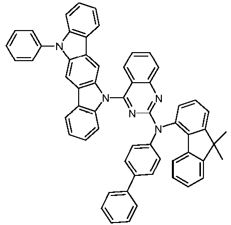 | 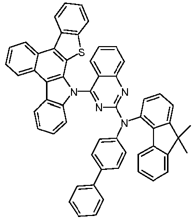 |
| Formel 73 | Formel 74 | Formel 75 |
| 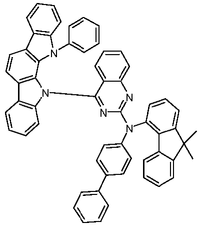 | 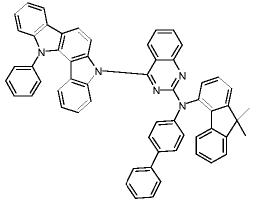 | 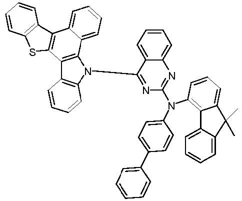 |
| Formel 76 | Formel 77 | Formel 78 |
| 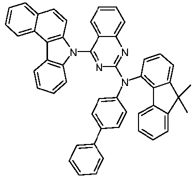 | 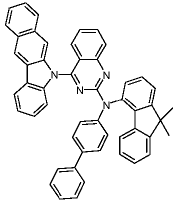 | 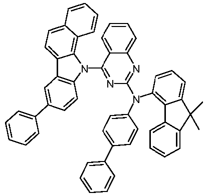 |
| Formel 79 | Formel 80 | Formel 81 |
| 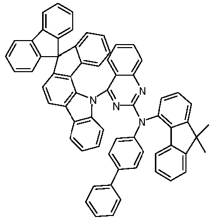 | 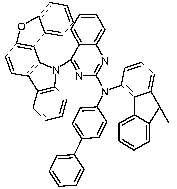 | 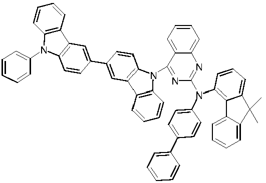 |
| Formel 82 | Formel 83 | Formel 84 |
| 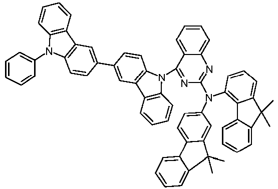 | 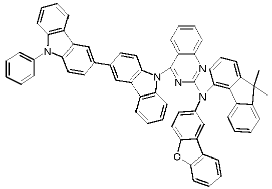 | 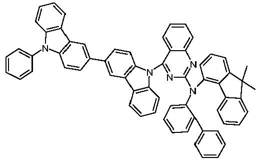 |
| Formel 85 | Formel 86 | Formel 87 |
| 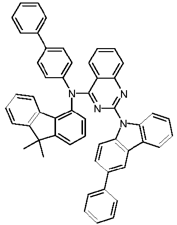 | 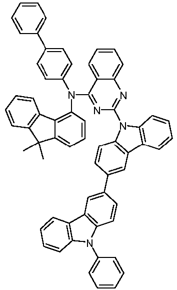 | 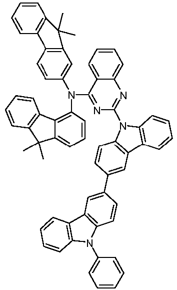 |
| Formel 88 | Formel 89 | Formel 90 |
| 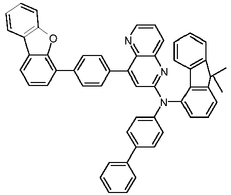 | 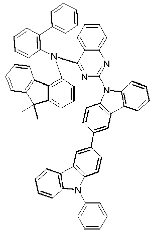 | 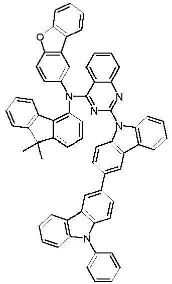 |
| Formel 91 | Formel 92 | Formel 93 |
| 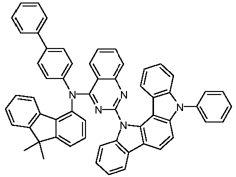 | 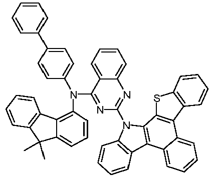 | 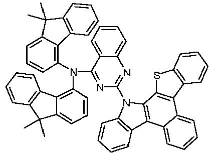 |
| Formel 94 | Formel 95 | Formel 96 |
| 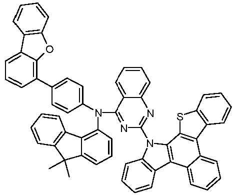 | 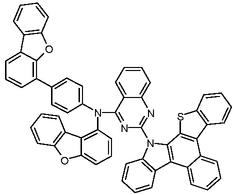 | 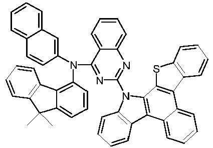 |
| Formel 97 | Formel 98 | Formel 99 |
| 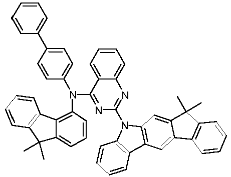 | 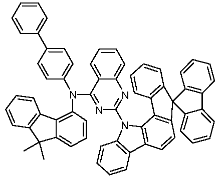 | 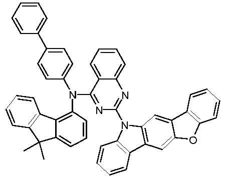 |
| Formel 100 | Formel 101 | Formel 102 |
| 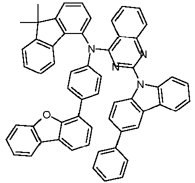 | 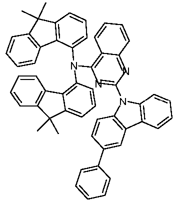 | 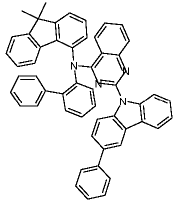 |
| Formel 103 | Formel 104 | Formel 105 |
| 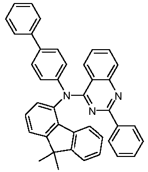 | 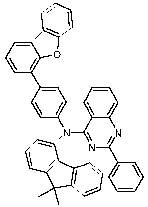 | 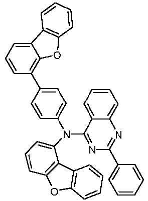 |
| Formel 106 | Formel 107 | Formel 108 |
| 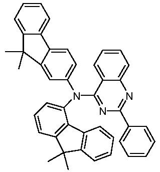 | 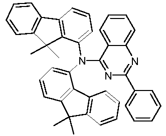 | 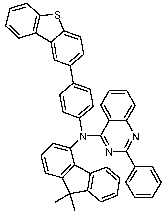 |
| Formel 109 | Formel 110 | Formel 111 |
| 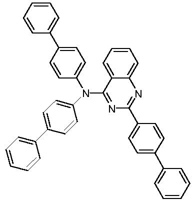 | 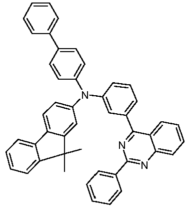 | 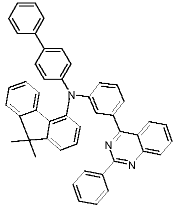 |
| Formel 112 | Formel 113 | Formel 114 |
| 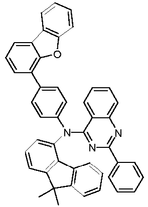 | 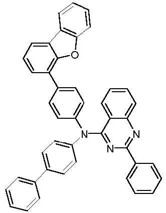 | 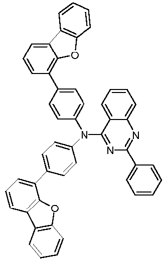 |
| Formel 115 | Formel 116 | Formel 117 |
| 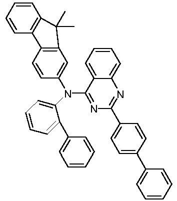 | 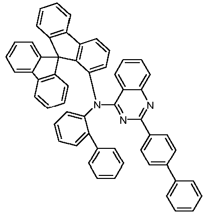 | 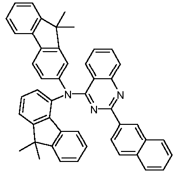 |
| Formel 118 | Formel 119 | Formel 120 |
| 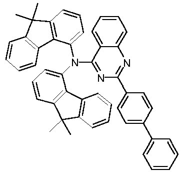 | 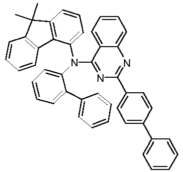 | 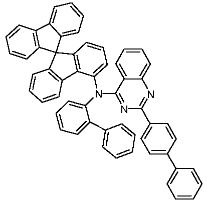 |
| Formel 121 | Formel 122 | Formel 123 |
| 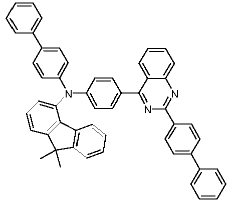 | 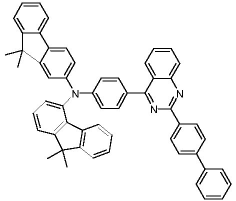 | 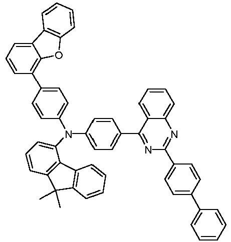 |
| Formel 124 | Formel 125 | Formel 126 |
| 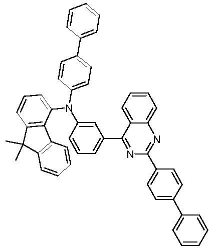 | 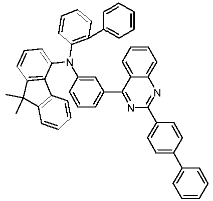 | 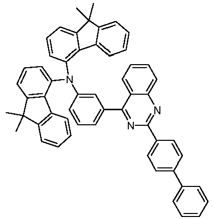 |
| Formel 127 | Formel 128 | Formel 129 |
| 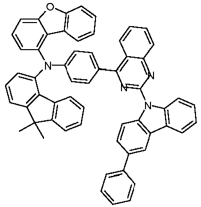 | 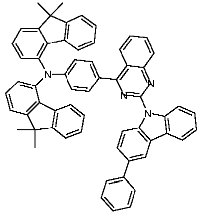 | 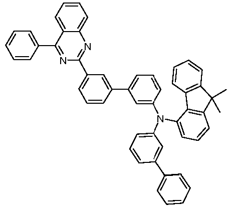 |
| Formel 130 | Formel 131 | Formel 132 |
| 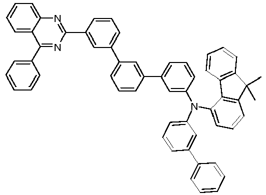 | 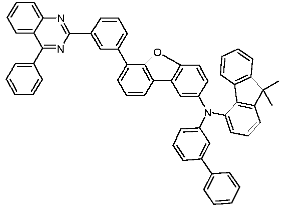 | 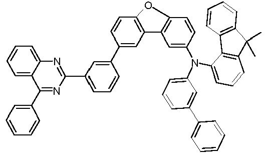 |
| Formel 133 | Formel 134 | Formel 135 |
| 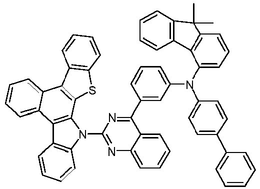 | 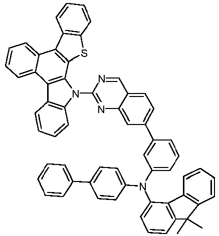 | 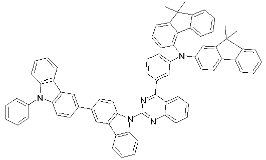 |
| Formel 136 | Formel 137 | Formel 138 |

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der erfindungsgemäßen Verbindungen bei dem in einer Kupplungsreaktion eine Diarylamin-Verbindung, mit einer Verbindung, umfassend mindestens eine Diazanaphthyl-Gruppe, verbunden wird.

Geeignete Verbindungen mit einer Diazanaphthyl-Gruppe oder Diarylamin-Verbindungen können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Arylverbindungen umgesetzt werden, wobei die notwendigen Bedingungen hierfür dem Fachmann bekannt sind und ausführliche Angaben in den Beispielen den Fachmann zur Durchführung dieser Umsetzungen unterstützen. Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Im allen folgenden Syntheseschemata sind die Verbindungen zur Vereinfachung der Strukturen mit einer geringen Anzahl an Substituenten gezeigt. Dies schließt das Vorhandensein von beliebigen weiteren Substituenten in den Verfahren nicht aus.

Eine Umsetzung ergibt sich beispielhaft gemäß folgenden Schemata, ohne dass hierdurch eine Beschränkung erfolgen soll. Die Teilschritte der einzelnen Schemata können hierbei beliebig kombiniert werden.

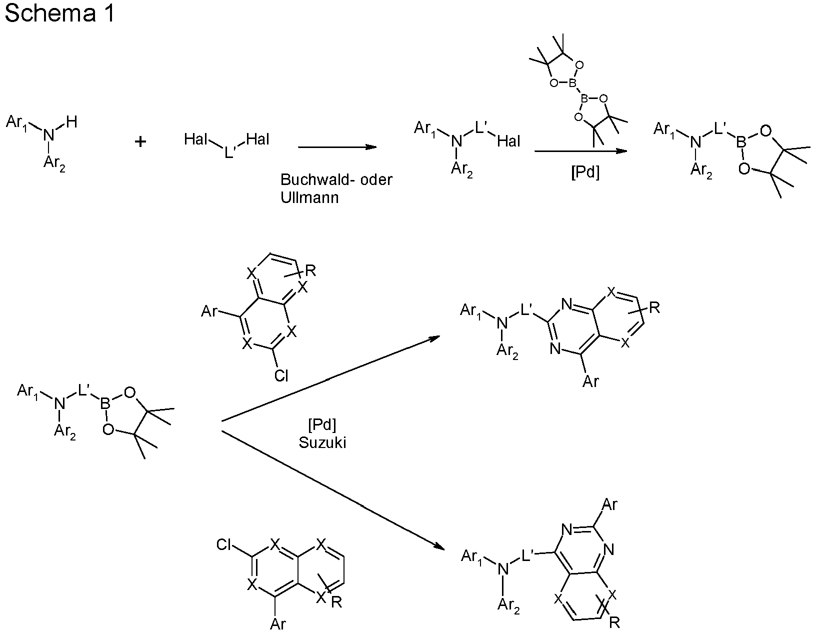

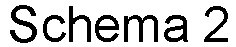

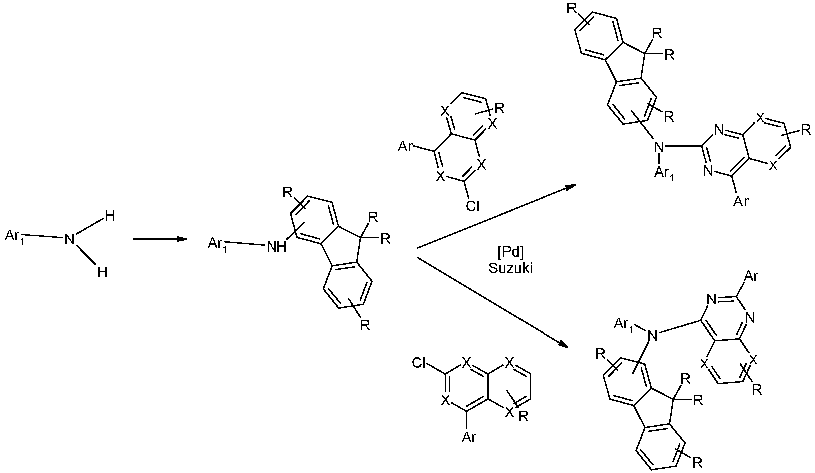

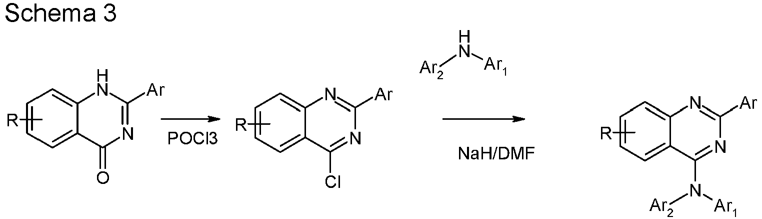

Journal of Materials Chemistry, 22(14), 6878-6884; 2012.

Die Bedeutung der in den Schemata 1 bis 3 verwendeten Symbole entspricht im Wesentlichen denen, die für Formel (I) definiert wurde, wobei aus Gründen der Übersichtlichkeit vielfach auf eine Nummerierung verzichtet wurde, jedoch die Diaryl- bzw. Triarylaminstruktur mit den Symbolen Ar₁, Ar₂ und teilweise L' zur besseren Leserlichkeit dargelegt wurde, um deutlich zu machen, dass die Arylgruppen verschieden sein können.

Die gezeigten Verfahren zur Synthese der erfindungsgemäßen Verbindungen sind exemplarisch zu verstehen. Der Fachmann kann alternative Synthesewege im Rahmen seines allgemeinen Fachwissens entwickeln. Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen, umfassend Strukturen gemäß Formel (I) in hoher Reinheit, bevorzugt mehr als 99 % (bestimmt mittels ¹H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, wie beispielsweise Toluol oder Xylol bei Raumtemperatur in ausreichender Konzentration löslich, um die Verbindungen aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen, umfassend mindestens eine Struktur der Formeln (IIIa), (IIIb), (XXIa), (XXIb), (XXIVa), (XXIVb) bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formeln (IIIa), (IIIb), (XXIa), (XXIb), (XXIVa), (XXIVb) oder erfindungsgemäße Verbindungen, wobei ein oder mehrere Bindungen der erfindungsgemäßen Verbindungen oder der Strukturen der Formeln (IIIa), (IIIb), (XXIa), (XXIb), (XXIVa), (XXIVb) zum Polymer, Oligomer oder Dendrimer vorhanden sind. Je nach Verknüpfungder Strukturen der Formeln (IIIa), (IIIb), (XXIa), (XXIb), (XXIVa), (XXIVb) bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß den Formeln (IIIa), (IIIb), (XXIa), (XXIb), (XXIVa), (XXIVb) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen umfassend Strukturen der allgemeinen Formeln (IIIa), (IIIb), (XXIa), (XXIb), (XXIVa), (XXIVb) bzw. die zuvor und nachfolgend ausgeführten bevorzugten Ausführungsformen bevorzugt, die eine Glasübergangstemperatur von mindestens 70 °C, besonders bevorzugt von mindestens 110 °C, ganz besonders bevorzugt von mindestens 125 °C und insbesondere bevorzugt von mindestens 150 °C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)-ethan, Hexamethylindan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, insbesondere ein phosphoreszierender Dotand, und/oder ein weiteres Matrixmaterial. Diese weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Emittern, die TADF (thermally activated delayed fluorescence) zeigen, Host-materialien, Elektronentransportmaterialien, Elektroneninjektionsmaterialien, Lochleitermaterialien, Lochinjektionsmaterialien, Elektronenblockiermaterialien, Lochblockiermaterialien, Wide-Band-Gap-Materialien und n-Dotanden.

Die vorliegenden Erfindung betrifft daher auch eine Zusammensetzung enthaltend wenigstens eine Verbindung umfassend Strukuren gemäß der Formeln (IIIa), (IIIb), (XXIa), (XXIb), (XXIVa), (XXIVb) bzw. die zuvor und nachfolgend ausgeführten bevorzugten Ausführungsformen sowie wenigstens ein weiteres Matrixmaterial. Gemäß einem besonderen Aspekt der vorliegenden Erfindung weist das weitere Matrixmaterial lochtransportierende Eigenschaften auf.

Ein weiterer Gegenstand der vorliegenden Erfindung stellt eine Zusammensetzung dar, enthaltend wenigstens eine Verbindung, umfassend mindestens eine Struktur gemäß den Formel (IIIa), (IIIb), (XXIa), (XXIb), (XXIVa), (XXIVb) bzw. die zuvor und nachfolgend ausgeführten bevorzugten Ausführungsformen sowie wenigstens ein Wide-Band-Gap-Material, wobei unter Wide-Band-Gap-Material ein Material im Sinne der Offenbarung von US 7,294,849 verstanden wird. Diese Systeme zeigen besondere vorteilhafte Leistungsdaten in elektrolumineszierenden Vorrichtungen.

Vorzugsweise kann die zusätzliche Verbindung eine Bandlücke (band gap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands T₁ bzw. des niedrigsten angeregten Singulettzustands S₁ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31 G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:
HOMO(eV) = ((HEh*27.212)-0.9899)/1.1206
LUMO(eV) = ((LEh*27.212)-2.0041)/1.385

Diese Werte sind im Sinne dieser Anmeldung als HOMO- bzw. LUMO-Energieniveaus der Materialien anzusehen.

Der niedrigste Triplettzustand T₁ ist definiert als die Energie des Triplettzustands mit der niedrigsten Energie, der sich aus der beschriebenen quantenchemischen Rechnung ergibt.

Der niedrigste angeregte Singulettzustand S₁ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die vorliegende Erfindung betrifft auch eine Zusammensetzung umfassend wenigstens eine Verbindung umfassend Strukuren gemäß den Formeln (IIIa), (IIIb), (XXIa), (XXIb), (XXIVa), (XXIVb) bzw. die zuvor und nachfolgend ausgeführten bevorzugten Ausführungsformen sowie wenigstens einen phosphoreszierenden Emitter, wobei unter dem Begriff phosphoreszierende Emitter auch phosphoreszierende Dotanden verstanden werden.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Matrix-Systemen, vorzugsweise Mixed-Matrix-Systemen sind die im Folgenden angebenen bevorzugten phosphoreszierenden Dotanden.

Vom Begriff phosphoreszierende Dotanden sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439 und den noch nicht offen gelegten Anmeldungen EP16179378.1 und EP16186313.9 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für phosphoreszierende Dotanden sind nachfolgend aufgeführt.

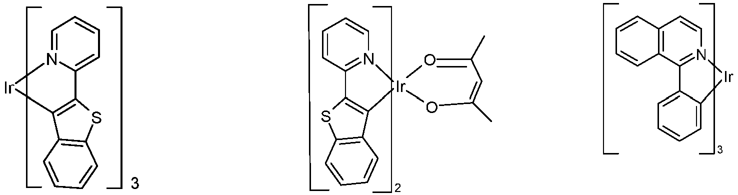

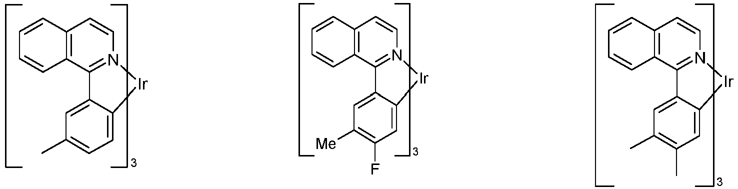

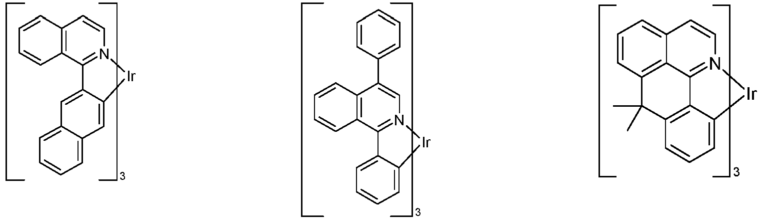

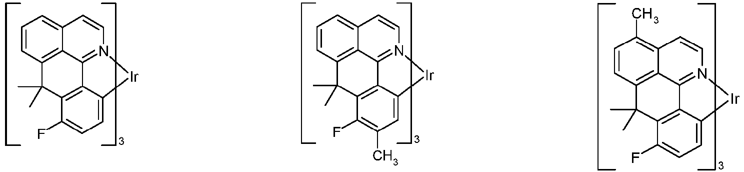

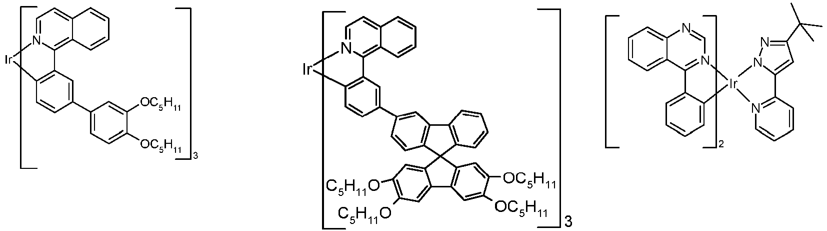

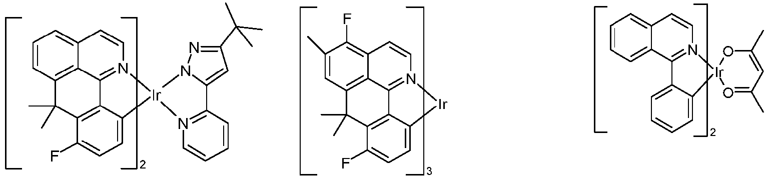

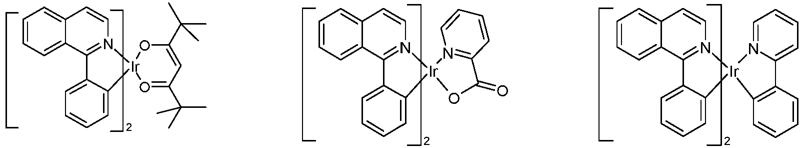

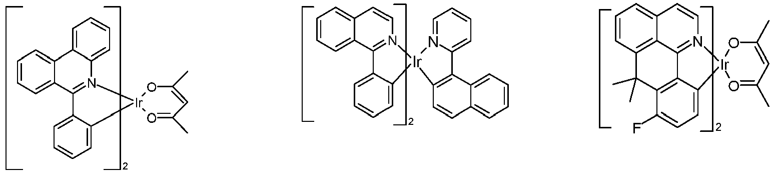

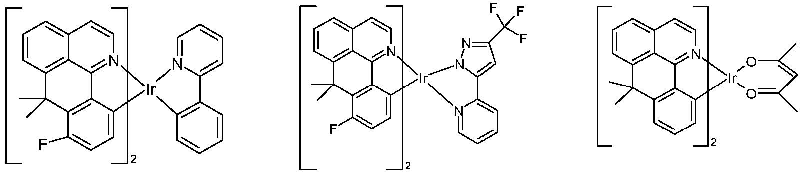

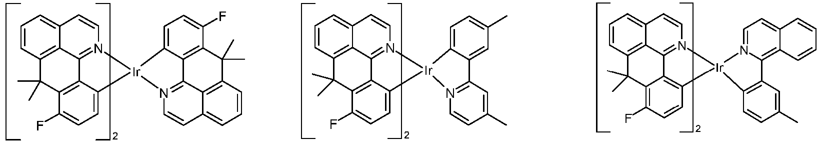

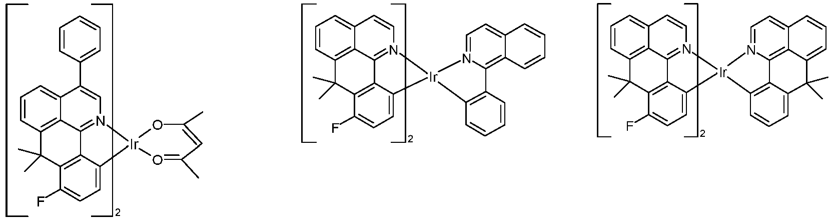

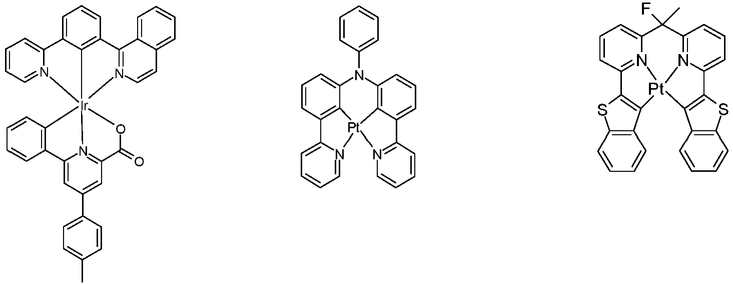

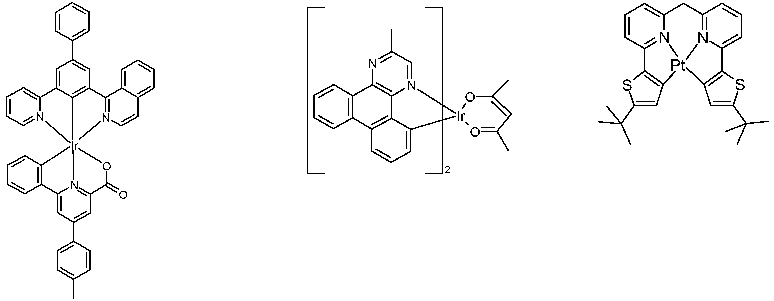

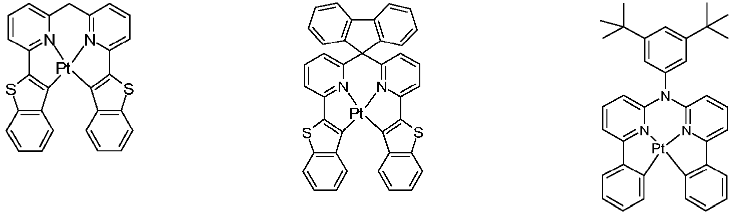

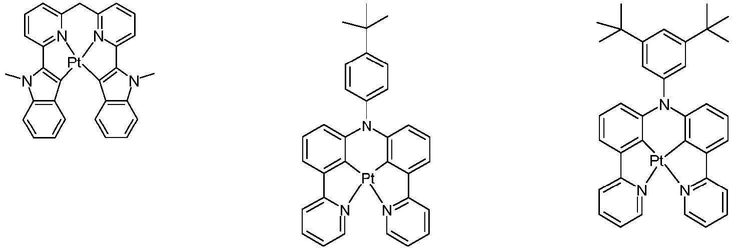

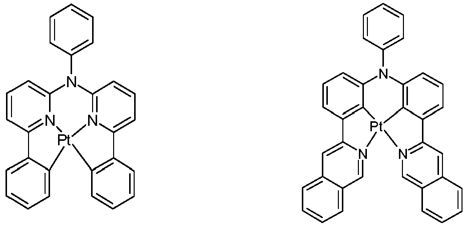

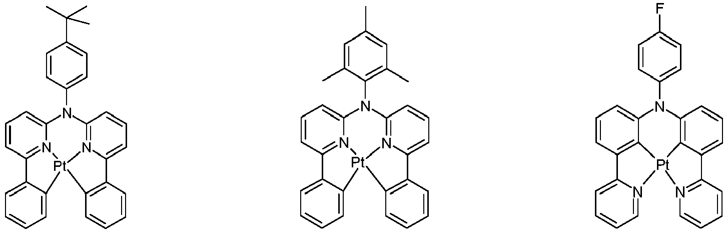

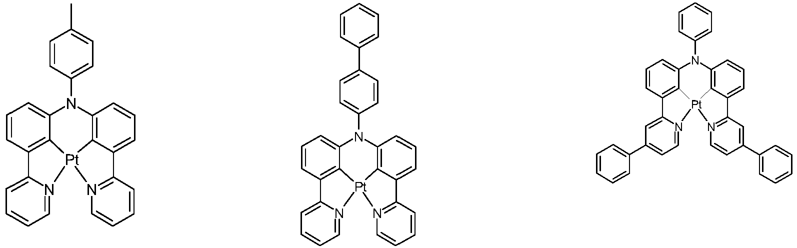

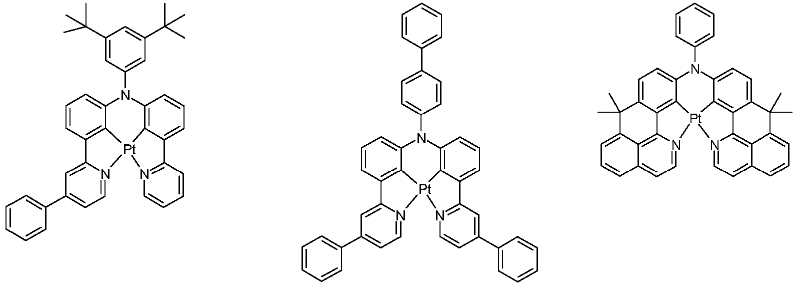

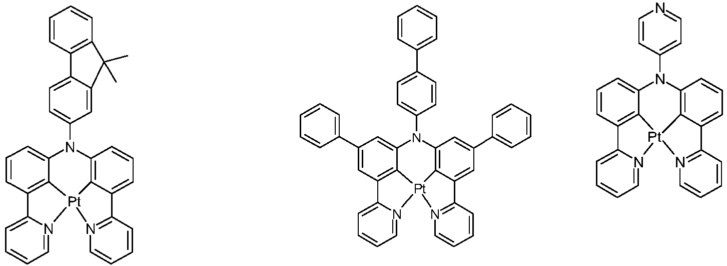

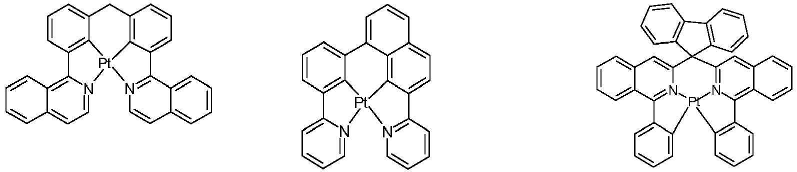

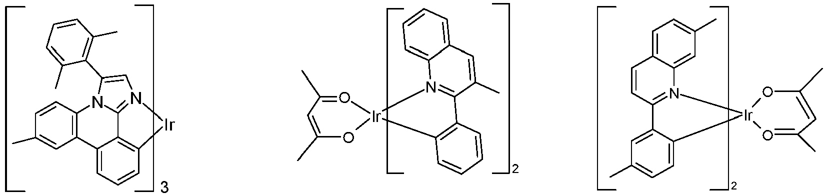

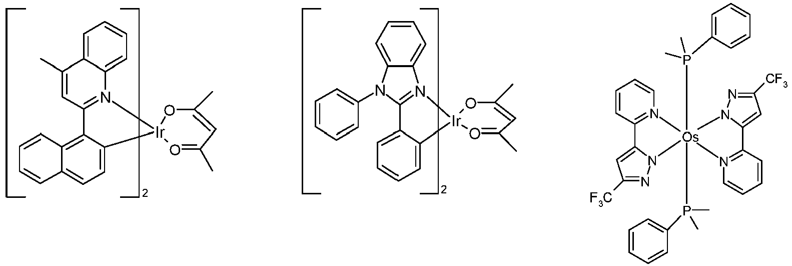

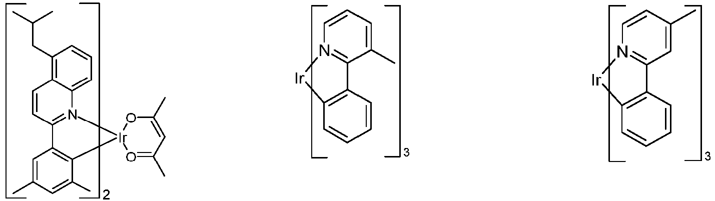

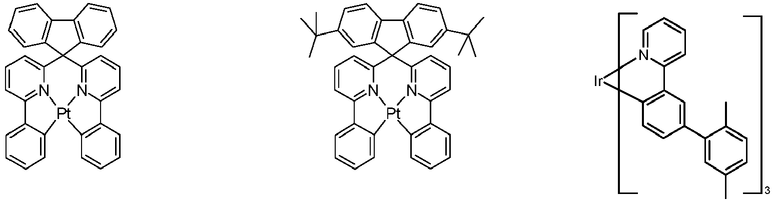

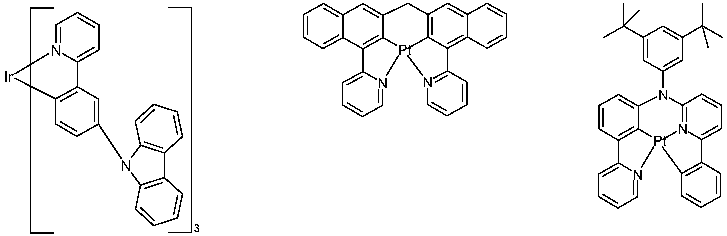

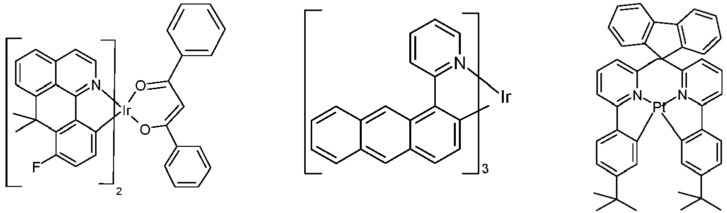

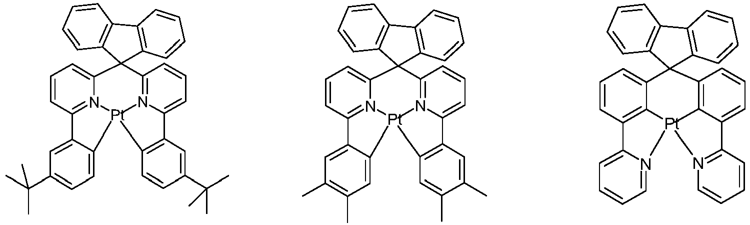

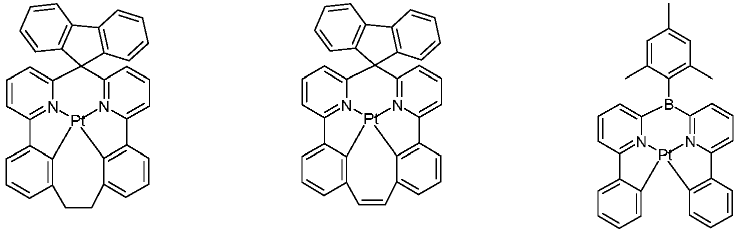

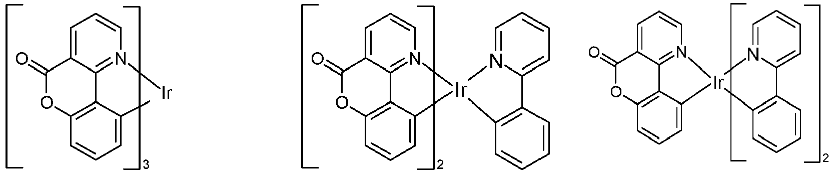

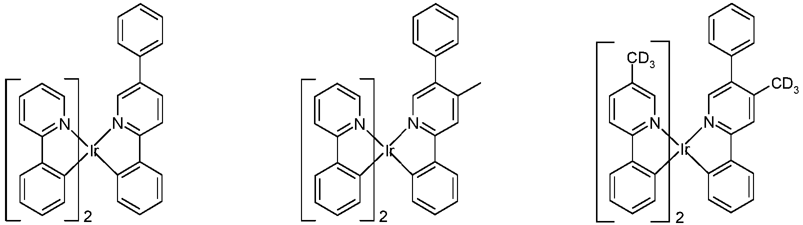

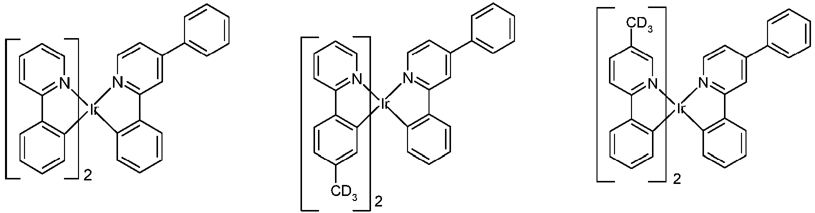

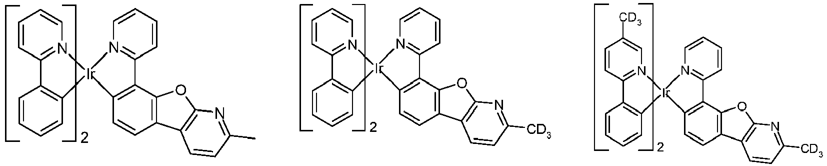

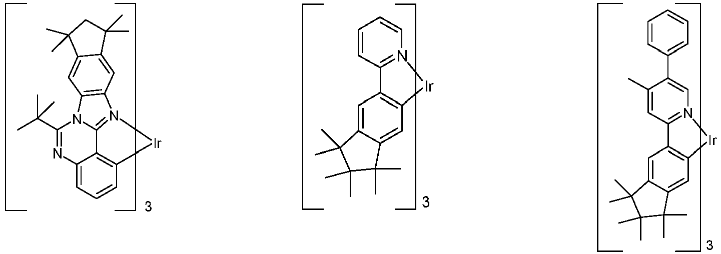

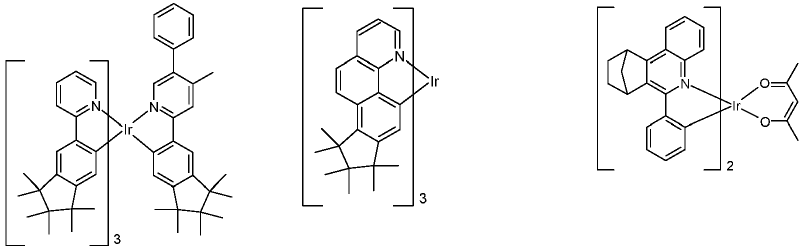

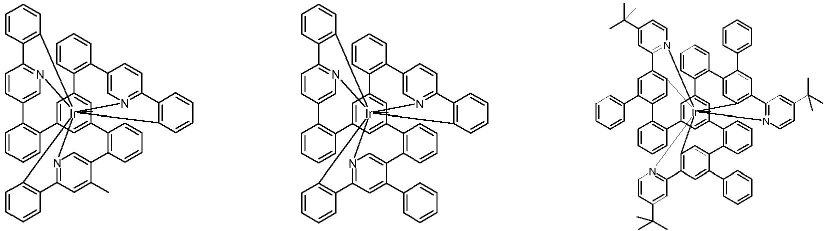

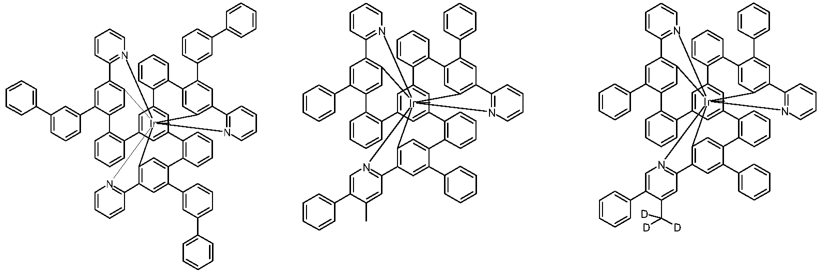

Die oben beschriebenen Verbindungen, umfassend Strukturen der Formel (IIIa), (IIIb), (XXIa), (XXIb), XXIVa), (XXIVb) bzw. die oben aufgeführten bevorzugten Ausführungsformen, können in einer elektronischen Vorrichtung bevorzugt als aktive Komponente verwendet werden. Unter einer elektronischen Vorrichtung wird eine Vorrichtung verstanden, welche Anode, Kathode und mindestens eine zwischen Anode und Kathode liegende Schicht enthält, wobei diese Schicht mindestens eine organische bzw. metallorganische Verbindung enthält. Die erfindungsgemäße elektronische Vorrichtung enthält also Anode, Kathode und mindestens eine dazwischen liegende Schicht, welche mindestens eine Verbindung, umfassend Strukturen der Formel (I), enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices", bevorzugt organischen Elektrolumineszenzvorrichtungen (OLEDs, PLEDs), insbesondere phosphoreszierenden OLEDs,enthaltend in mindestens einer Schicht mindestens eine Verbindung, umfassend Strukturen der Formel (I). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie MoOs oder WOs oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonenblockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Weiterhin bevorzugt sind Tandem-OLEDs. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die efindungsgemäße Verbindung umfassend Strukturen gemäß den Formeln (IIIa), (IIIb), (XXIa), (XXIb), (XXIVa), (XXIVb) bzw. die oben aufgeführten bevorzugten Ausführungsformen als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist. Eine emittierende Schicht umfasst mindestens eine emittierende Verbindung.

Geeignete Matrixmaterialien, welche in Kombination mit den Verbindungen gemäß den Formeln (IIIa), (IIIb), (XXIa), (XXIb), (XXIVa), (XXIVb) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, insbesondere Monoamine, z. B. gemäß WO 2014/015935, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivate, z. B. gemäß WO 2012/048781, Lactame, z. B. gemäß WO 2011/116865, WO 2011/137951 oder WO 2013/064206, 4-SpirocarbazolDerivate, z. B. gemäß WO 2014/094963 oder WO 2015/192939, oder Dibenzofuran-Derivate, z. B. gemäß WO 2015/169412, WO 2016/015810, WO 2016/023608, WO 2017/148564 oder WO 2017/148565. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame und Carbazolderivate.

Bevorzugte Triarylaminderivate, die als Co-Host-Materialien zusammen mit den erfindungsgemäßen Verbindungen eingesetzt werden, sind ausgewählt aus den Verbindungen der folgenden Formel (TA-1), 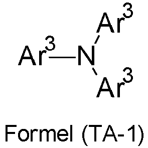 wobei Ar³ gleich oder verschieden bei jedem Auftreten ein aromatisches oder heteroaromatisches Ringsystem mit 6 bis 40 C-Atomen, das jeweils mit einem oder mehreren Resten R² substituiert sein kann, darstellt, wobei optional zwei oder mehr benachbarte Substituenten R² ein mono- oder polycyclisches, aliphatisches Ringsystem bilden können, das mit einem oder mehreren Resten R³ substituiert sein kann, wobei das Symbol R² die zuvorgenannte Bedeutung aufweist. Vorzugsweise stellt Ar³ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten R² substituiert sein kann, vorzugsweise jedoch unsubstituiert ist.

Beispiele für geeignete Gruppen Ar³ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R² substituiert sein können, bevorzugt aber unsubstituiert sind.

Bevorzugt sind die Gruppen Ar³ gleich oder verschieden bei jedem Auftreten ausgewählt aus den oben genannten Gruppen R¹-1 bis R¹-80, besonders bevorzugt R¹-1 bis R¹-51.

In einer bevorzugten Ausführungsform der Verbindungen der Formel (TA-1) ist mindestens eine Gruppe Ar¹ ausgewählt aus einer Biphenylgruppe, wobei es sich um eine ortho-, meta- oder para-Biphenylgruppe handeln kann. In einer weiteren bevorzugten Ausführungsform der Verbindungen der Formel (TA-1) ist mindestens eine Gruppe Ar¹ ausgewählt aus einer Fluorengruppe oder Spirobifluorengruppe, wobei diese Gruppen jeweils in 1-, 2-, 3- oder 4-Position an das Stickstoffatom gebunden sein können. In nochmals einer weiteren bevorzugten Ausführungsform der Verbindungen der Formel (TA-1) ist mindestens eine Gruppe Ar³ ausgewählt aus einer Phenylen- oder Biphenylgruppe, wobei es sich um eine ortho-, meta- oder para-verknüpfte Gruppe handelt, die mit einer Dibenzofurangruppe, einer Dibenzothiophengruppe oder einer Carbazolgruppe, insbesondere einer Dibenzofurangruppe, substituiert ist, wobei die Dibenzofuran- bzw. Dibenzothiophengruppe über die 1-, 2-, 3- oder 4-Position mit der Phenylen- bzw. Biphenylgruppe verknüpft ist und wobei die Carbazolgruppe über die 1-, 2-, 3- oder 4-Position oder über das Stickstoffatom mit der Phenylen- bzw. Biphenylgruppe verknüpft ist.

In einer besonders bevorzugten Ausführungsform der Verbindungen der Formel (TA-1) ist eine Gruppe Ar³ ausgewählt aus einer Fluoren- oder Spirobifluorengruppe, insbesondere einer 4-Fluoren- bzw. 4-Spirobifluorengruppe, und eine Gruppe Ar³ ist ausgewählt aus einer Biphenylgruppe, insbesondere einer para-Biphenylgruppe, oder einer Fluorengruppe, insbesondere einer 2-Fluorengruppe, und die dritte Gruppe Ar³ ist ausgewählt aus einer para-Phenylengruppe oder einer para-Biphenylgruppe, die mit einer Dibenzofurangruppe, insbesondere einer 4-Dibenzofurangruppe, oder einer Carbazolgruppe, insbesondere einer N-Carbazolgruppe oder einer 3-Carbazolgruppe, substituiert ist.

Bevorzugte Indenocarbazolderivate, die als Co-Host-Materialien zusammen mit den erfindungsgemäßen Verbindungen eingesetzt werden, sind ausgewählt aus den Verbindungen der folgenden Formel (TA-2), 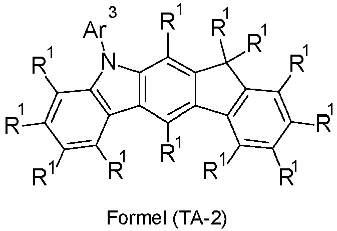 wobei Ar³ und R¹ die oben insbesondere für Formeln (I) und/oder (TA-3) aufgeführten Bedeutungen aufweisen. Dabei sind bevorzugte Ausführungsformen der Gruppe Ar³ die oben aufgeführten Strukturen R¹-1 bis R¹-80, besonders bevorzugt R¹-1 bis R¹-51.

Eine bevorzugte Ausführungsform der Verbindungen der Formel (TA-2) sind die Verbindungen der folgenden Formel (TA-2a), 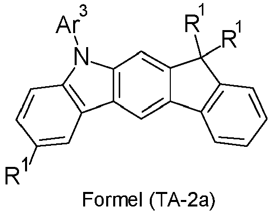 wobei Ar³ und R¹ die oben, insbesondere für Formeln (I) und/oder (TA-1) aufgeführten Bedeutungen aufweisen. Dabei stehen die beiden Gruppen R¹, die an das Indenokohlenstoffatom gebunden sind, bevorzugt gleich oder verschieden für eine Alkylgruppe mit 1 bis 4 C-Atomen, insbesondere für Methylgruppen, oder für ein aromatisches Ringsystem mit 6 bis 12 C-Atomen, insbesondere für Phenylgruppen. Besonders bevorzugt stehen die beiden Gruppen R¹, die an das Indenokohlenstoffatom gebunden sind, für Methylgruppen. Weiterhin bevorzugt steht der Substituent R¹, der in Formel (TA-2a) an den Indenocarbazolgrundkörper gebunden ist, für H oder für eine Carbazolgruppe, die über die 1-, 2-, 3- oder 4-Position oder über das N-Atom an den Indenocarbazolgrundkörper gebunden sein kann, insbesondere über die 3-Position.

Bevorzugte 4-Spirocarbazolderivate, die als Co-Host-Materialien zusammen mit den erfindungsgemäßen Verbindungen eingesetzt werden, sind ausgewählt aus den Verbindungen der folgenden Formel (TA-3), 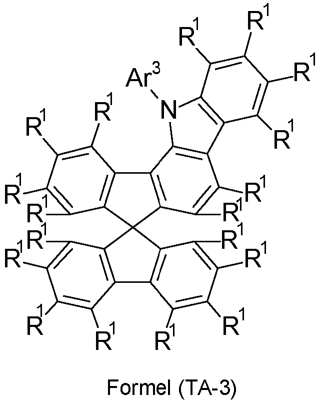 wobei Ar³ und R¹ die oben, insbesondere für Formeln (TA-1), (I), (II) und/oder (Q-1) aufgeführten Bedeutungen aufweisen. Dabei sind bevorzugte Ausführungsformen der Gruppe Ar³ die oben aufgeführten Strukturen R¹-1 bis R¹-80, besonders bevorzugt R¹-1 bis R¹-51.

Eine bevorzugte Ausführungsform der Verbindungen der Formel (TA-3) sind die Verbindungen der folgenden Formel (TA-3a), 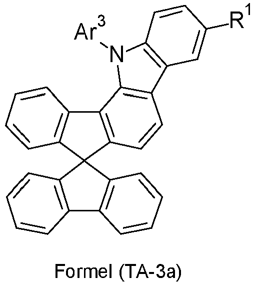 wobei Ar³ und R¹ die oben, insbesondere für Formeln (TA-1), (I), (II) und/oder (Q-1) aufgeführten Bedeutungen aufweisen. Dabei sind bevorzugte Ausführungsformen der Gruppe Ar³ die oben aufgeführten Strukturen R¹-1 bis R¹-80, besonders bevorzugt R¹-1 bis R¹-51.

Bevorzugte Lactame, die als Co-Host-Materialien zusammen mit den erfindungsgemäßen Verbindungen eingesetzt werden, sind ausgewählt aus den Verbindungen der folgenden Formel (LAC-1), 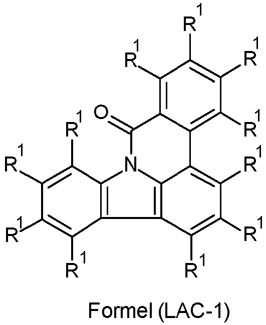 wobei R¹ die oben, insbesondere für Formel (I) aufgeführte Bedeutung aufweist.

Eine bevorzugte Ausführungsform der Verbindungen der Formel (LAC-1) sind die Verbindungen der folgenden Formel (LAC-1a), 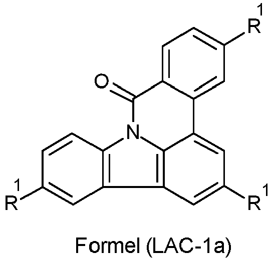 wobei R¹ die obengenannte Bedeutung aufweist. Dabei steht R¹ bevorzugt gleich oder verschieden bei jedem Auftreten für H oder ein aromatisches oder heteroaromatisches Ringsystem mit 5 bis 40 aromatischen Ringatomen, das mit einem oder mehreren Resten R² substituiert sein kann, wobei R² die zuvor genannte Bedeutung aufweisen kann. Ganz besonders bevorzugt sind die Substituenten R¹ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten R² substituiert sein kann, bevorzugt aber unsubstituiert ist. Beispiele für geeignete Substituenten R¹ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R² substituiert sein können, bevorzugt aber unsubstituiert sind. Dabei sind geeignete Strukturen R¹ die gleichen Strukturen, wie sie zuvor für R-1 bis R-79 abgebildet sind, besonders bevorzugt R¹-1 bis R¹-51.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung umfassend Strukuren gemäß den Formeln (IIIa), (IIIb), (XXIa), (XXIb), (XXIVa), (XXIVb) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend Verbindung umfassend Strukuren gemäß den Formeln (IIIa), (IIIb), (XXIa), (XXIb), (XXIVa), (XXIVb) bzw. die zuvor und nachfolgend ausgeführten bevorzugten Ausführungsformen in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen umfassend Strukuren gemäß Formel (I) bzw. die zuvor und nachfolgend ausgeführten bevorzugten Ausführungsformen als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung, Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren elektronenleitenden Schichten als elektronenleitende Verbindung umfasst.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise MoO₃ oder WO₃, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren aufgetragen werden. Dabei werden die Materialien in Vakuum-Sublimationsanlagen bei einem Anfangsdruck von üblicherweise kleiner 10⁻⁵ mbar, bevorzugt kleiner 10⁻⁶ mbar aufgedampft. Es ist auch möglich, dass der Anfangsdruck noch geringer oder noch höher ist, beispielsweise kleiner 10⁻⁷ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation aufgetragen werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine erfindungsgemäße Verbindung umfassend Strukturen gemäß den Formeln (IIIa), (IIIb), (XXIa), (XXIb), (XXIVa), (XXIVb) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronischen Vorrichtungen, insbesondere organische Elektrolumineszenzvorrichtungen enthaltend erfindungsgemäße Verbindungen umfassend Strukturen gemäß den Formeln (IIIa), (IIIb), (XXIa), (XXIb), (XXIVa), (XXIVb) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:
1. Elektronische Vorrichtungen, insbesondere organische Elektrolumineszenzvorrichtungen enthaltend Verbindungen, Oligomere, Polymere oder Dendrimere mit Strukturen gemäß den Formel (IIIa), (IIIb), (XXIa), (XXIb), (XXIVa), (XXIVb) bzw. die zuvor und nachfolgend ausgeführten bevorzugten Ausführungsformen, insbesondere als elektronenleitende Materialien und/oder Lochleitermaterialien und/oder als Matrixmaterialien, weisen eine sehr gute Lebensdauer auf.
2. Elektronische Vorrichtungen, insbesondere organische Elektrolumineszenzvorrichtungen enthaltend Verbindungen, Oligomere, Polymere oder Dendrimere mit Strukturen gemäß den Formeln (IIIa), (IIIb), (XXIa), (XXIb), (XXIVa), (XXIVb) bzw. die zuvor und nachfolgend ausgeführten bevorzugten Ausführungsformen insbesondere als Elektronentransport-Materialien, Lochleitermaterialien und/oder als Hostmaterialien weisen eine hervorragende Effizienz auf. Insbesondere ist die Effizienz deutlich höher gegenüber analogen Verbindungen, die keine Struktureinheit gemäß den Formeln (IIIa), (IIIb), (XXIa), (XXIb), (XXIVa), (XXIVb) enthalten. Hierbei bewirken erfindungsgemäßen Verbindungen, Oligomere, Polymere oder Dendrimere mit Strukturen gemäß den Formeln (IIIa), (IIIb), (XXIa), (XXIb), (XXIVa), (XXIVb) bzw. die zuvor und nachfolgend ausgeführten bevorzugten Ausführungsformen eine geringe Betriebsspannung bei Verwendung in elektronischen Vorrichtungen. Hierbei bewirken diese Verbindungen insbesondere einen geringen Roll-off, d.h. einen geringen Abfall der Leistungseffizienz der Vorrichtung bei hohen Leuchtdichten.
3. Elektronische Vorrichtungen, insbesondere organische Elektrolumineszenzvorrichtungen enthaltend Verbindungen, Oligomere, Polymere oder Dendrimere mit Strukturen gemäß den Formeln (IIIa), (IIIb), (XXIa), (XXIb), (XXIVa), (XXIVb) bzw. die zuvor und nachfolgend ausgeführten bevorzugten Ausführungsformen als Elektronentransport-Materialien, Lochleitermaterialien und/oder als Hostmaterialien weisen eine hervorragende Farbreinheit auf.
4. Die erfindungsgemäßen Verbindungen, Oligomere, Polymere oder Dendrimere mit Strukturen gemäß den Formeln (IIIa), (IIIb), (XXIa), (XXIb), (XXIVa), (XXIVb) bzw. die zuvor und nachfolgend ausgeführten bevorzugten Ausführungsformen zeigen eine sehr hohe thermische und photochemische Stabilität und führen zu Verbindungen mit einer sehr hohen Lebensdauer.
5. Die Verwendung von Verbindungen, Oligomeren, Polymeren oder Dendrimeren mit Strukturen gemäß den Formeln (IIIa), (IIIb), (XXIa), (XXIb), (XXIVa), (XXIVb) bzw. den zuvor und nachfolgend ausgeführten bevorzugten Ausführungsformen in Schichten elektronischer Vorrichtungen, insbesondere organischer Elektrolumineszenzvorrichtungen führt zu einer hohen Mobilität der Elektronleiterstrukturen.
6. Verbindungen, Oligomere, Polymere oder Dendrimere mit Strukturen gemäß den Formel (IIIa), (IIIb), (XXIa), (XXIb), (XXIVa), (XXIVb) bzw. die zuvor und nachfolgend ausgeführten bevozugten Ausführungsformen zeichnen sich durch eine ausgezeichnete thermische Stabilität aus, wobei Verbindungen mit einer Molmasse von weniger als ca. 1200 g/mol gut sublimierbar sind.
7. Verbindungen, Oligomere, Polymere oder Dendrimere mit Strukturen gemäß den Formeln (IIIa), (IIIb), (XXIa), (XXIb), (XXIVa), (XXIVb) bzw. die zuvor und nachfolgend ausgeführten bevorzugten Ausführungsformen weisen eine ausgezeichnete Glasfilmbildung auf.
8. Verbindungen, Oligomere, Polymere oder Dendrimere mit Strukturen gemäß den Formeln (IIIa), (IIIb), (XXIa), (XXIb), (XXIVa), (XXIVb) bzw. die zuvor und nachfolgend ausgeführten bevorzugten Ausführungsformen bilden aus Lösungen sehr gute Filme.

Diese oben genannten Vorteile gehen nicht mit einer Verschlechterung der weiteren elektronischen Eigenschaften einher.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Ein nochmals weiterer Gegenstand der vorliegenden Erfindung ist die Verwendung einer erfindungsgemäßen Verbindung und/oder eines erfindungsgemäßen Oligomers, Polymers oder Dendrimers in einer elektronischen Vorrichtung als Hostmaterial, Lochleitmaterial, Elektroneninjektionsmaterial und/oder Elektronentransportmaterial, vorzugsweise als Hostmaterial und/oder Elektronentransportmaterial.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen. Besonders bevorzugt ist elektronische Vorrichtung ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices", bevorzugt organischen Elektrolumineszenzvorrichtungen (OLEDs, PLEDs), insbesondere phosphoreszierenden OLEDs.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß den Formeln (IIIa), (IIIb), (XXIa), (XXIb), (XXIVa), (XXIVb) bzw. gemäß den bevorzugten Ausführungsformen einsetzen.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen. Der Fachmann kann aus den Schilderungen ohne erfinderisches Zutun weitere erfindungsgemäße elektronische Vorrichtungen herstellen und somit die Erfindung im gesamten beanspruchten Bereich ausführen.

### Beispiele

### Synthesebeispiele

Die nachfolgenden Synthesen werden, sofern nicht anders angegeben, unter einer Schutzgasatmosphäre in getrockneten Lösungsmitteln durchgeführt. Die erfindungsgemäßen Verbindungen können mittels dem Fachmann bekannten Syntheseverfahren dargestellt werden.

### a) Biphenyl-4-yl-(9,9-dimethyl-9H-fluoren-1-yl)-amin

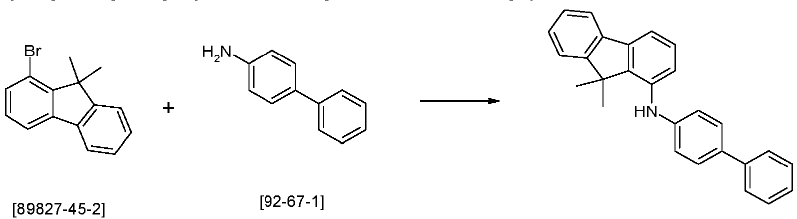

36 g (212 mmol, 1,0 eq) 4-Aminobiphenyl werden zusammen mit 57,8 g (177 mmol, 1,0 eq) 1-Bromdimethylfluoren und 2,4 g (212 mmol, 1,20 eq) Natrium-t-pentoxid [14593-46-5] in 600 ml absolutem Toluol vorgelegt und für 30 Minuten entgast. Anschließend werden 398 mg (1,77 mmol, 0,01 eq) Palladium(II)-acetat [3375-31-3] und 1,46 g (3,56 mmol, 0,02eq) 2-Dicyclohexylphosphino-2',6'-dimethoxybiphenyl (SPhos) [657408-07-6] zugegeben und der Ansatz über Nacht unter Rückfluss erhitzt. Nach beendeter Reaktion wird der Ansatz auf Raumtemperatur abgekühlt und mit 500 ml Wasser extrahiert. Anschließend wird die wässrige Phase dreifach mit Toluol gewaschen, die vereinten organischen Phasen über Natriumsulfat getrocknet und das Lösungsmittel am Rotationsverdampfer entfernt. Der braune Rückstand wird in ca. 200 ml Toluol aufgenommen und über Silikagel filtriert. Zur weiteren Aufreinigung wird eine Umkristallisation aus Toluol/Heptan durchgeführt. Ausbeute: 59 g (164 mmol), 79 % der Theorie.

Analog können die folgenden Verbindungen dargestellt werden:

| | **Edukt 1** | **Edukt 2** | **Produkt** | **Ausbeute [%]** |
|---|---|---|---|---|
| 1a | 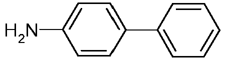 [92-67-1] | 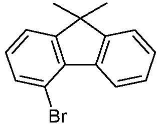 [942615-32-9] | 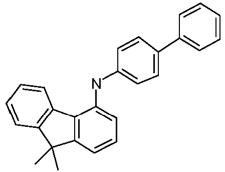 | 65 |
| 2a | 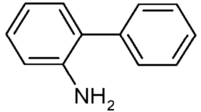 [90-41-5] | 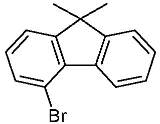 [942615-32-9] | 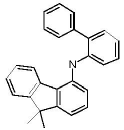 | 63 |
| 3a | 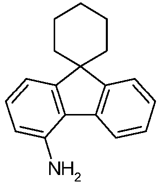 [18998-24-8] | 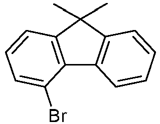 [942615-32-9] | 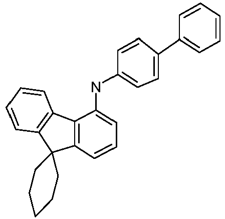 | 60 |
| 4a | 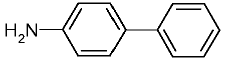 [92-67-1] | 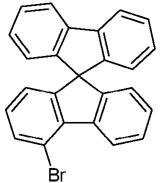 [1161009-88-6 | 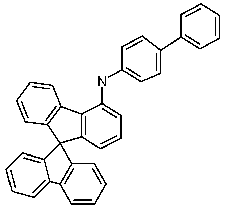 | 62 |
| 5a | 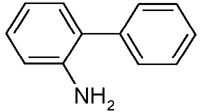 [90-41-5] | 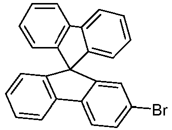 [171408-76-7] | 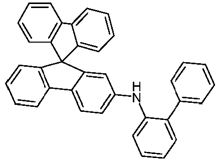 | 64 |
| 6a | 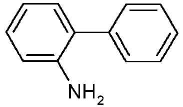 [90-41-5] | 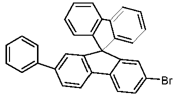 [1361305-36-3] | 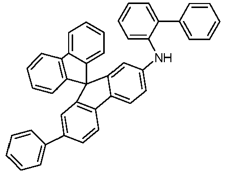 | 68 |
| 7a | 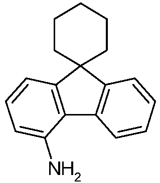 [18998-24-8] | 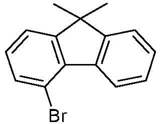 [942615-32-9] | 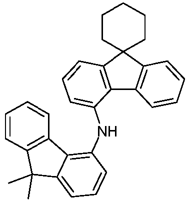 | 71 |
| 8a | 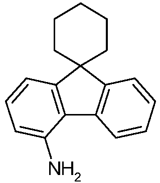 [18998-24-8] | 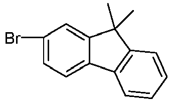 [320-31-2] | 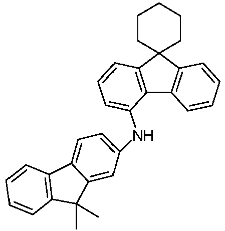 | 72 |
| 9a | 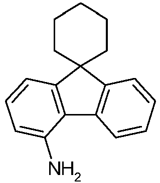 [18998-24-8] | 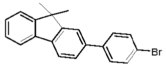 [574750-94-0] | 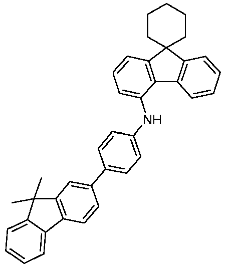 | 83 |
| 10a | 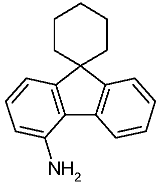 [18998-24-8] | 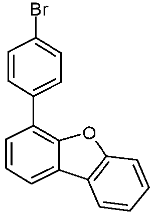 [1225053-54-2] | 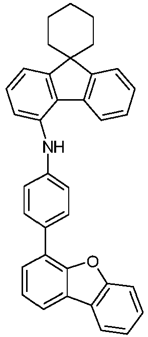 | 64 |
| 11a | 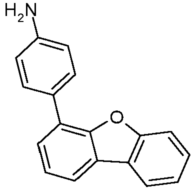 [578027-21-1] | 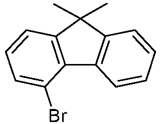 [942615-32-9] | 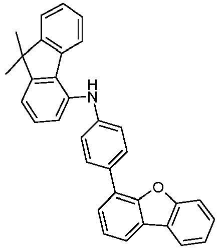 | 67 |
| 12a | 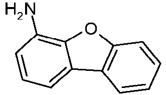 [50548-43-1] | 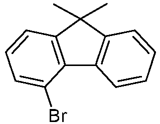 [942615-32-9] | 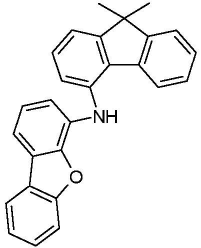 | 56 |
| 13a | 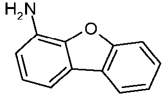 [50548-43-1] | 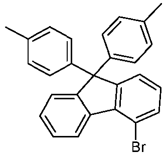 | 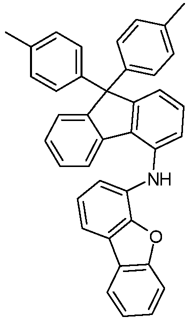 | 75 |
| 14a | 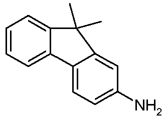 [108714-73-4] | 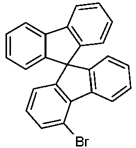 [1161009-88-6] | 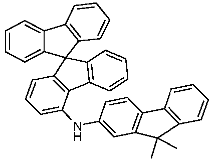 | 85 |
| 15a | 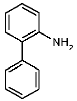 [90-41-5] | 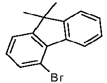 [942615-32-9] | 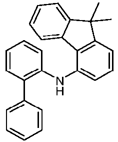 | 69 |
| 16a | 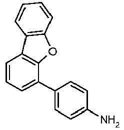 [578027-21-1] | 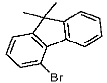 [942615-32-9] | 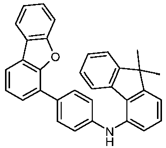 | 67 |
| 17a | 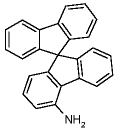 [1579281-06-3] | 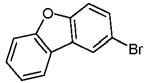 [86-76-0] | 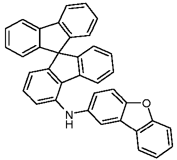 | 88 |
| 18a | 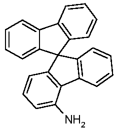 [1579281-06-3] | 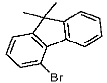 [942615-32-9] | 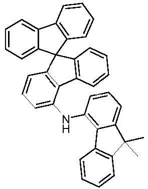 | 81 |
| 19a | 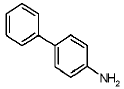 [92-67-1] | 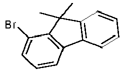 [1225053-54-2] | 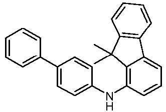 | 77 |
| 20a | 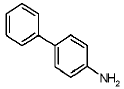 [92-67-1] | 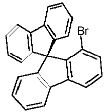 [1450933-18-2] | 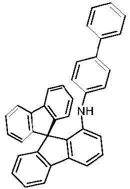 | 70 |
| 21a | 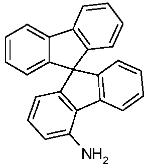 [1579281-06-3] | 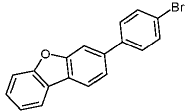 [86-76-0] | 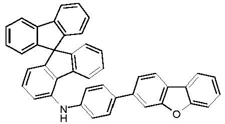 | 84 |
| 22a | 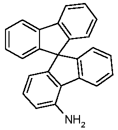 [1579281-06-3] | 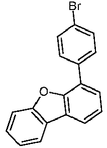 [955959-84-9] | 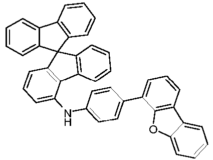 | 91 |
| 23a | 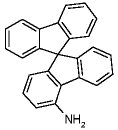 [1579281-06-3] | 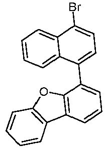 [1297532-85-4] | 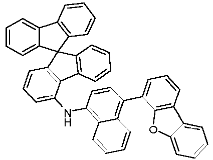 | 74 |
| 24a | 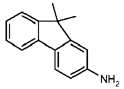 [108714-73-4] | 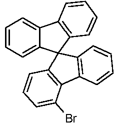 [1161009-88-6] | 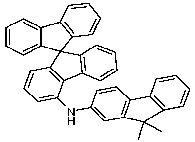 | 85 |
| 25a | 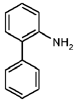 [90-41-5] | 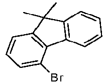 [942615-32-9] | 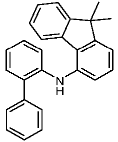 | 69 |
| 26a | 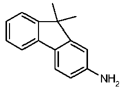 [108714-73-4] | 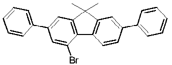 [1407005-83-8] | 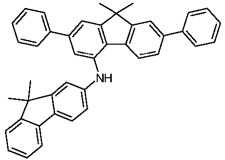 | 67 |
| 27a | 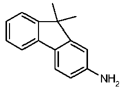 [108714-73-4] | 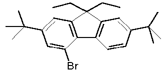 [942615-28-3] | 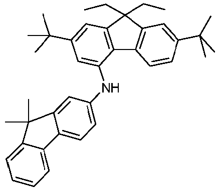 | 71 |
| 28a | 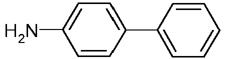 [92-67-1] | 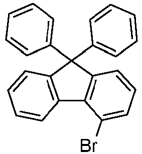 [713125-22-5] | 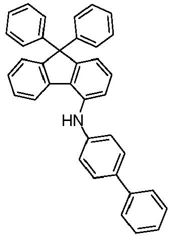 | 70 |

### b) Biphenyl-4-yl-(4-bromphenyl)-(9,9-dimethyl-9H-fluoren-4-yl)-amin

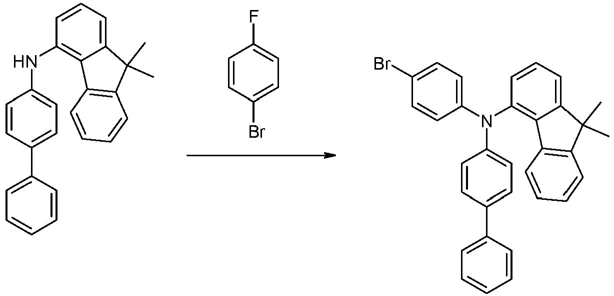

In einem 1L-Vierhalskolben werden 51,3 g (142 mmol, 1,00 eq) Biphenyl-4-yl-(9,9-dimethyl-9H-fluoren-4-yl)-amin, sowie 75,6 g (426 mmol, 3,00 eq) 1-Brom-4-fluorobenzol [460-00-4] und 92,5 g (284 mmol, 2,00 eq) Cäsiumcarbonat [534-17-8] vorgelegt und mit 500 ml Dimethylacetamid versetzt. Das Reaktionsgemisch wird für drei Tage bei 150 °C gerührt. Nach beendeter Reaktion wird der Ansatz auf Raumtemperatur abgekühlt und der Feststoff über Celite abfiltriert. Die Mutterlauge wird eingeengt und der ausgefallene Feststoff nach Filtration mit heißem Methanol ausgerührt. Ausbeute: 43 g (135 mmol), 95 % der Theorie.

Analog können die folgenden Verbindungen dargestellt werden:

| | **Edukt** | **Produkt** | **Ausbeute [%]** |
|---|---|---|---|
| 1b | 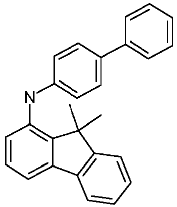 | 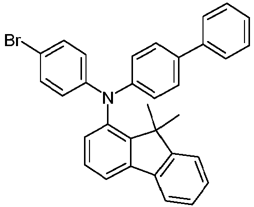 | 78 |
| 2b | 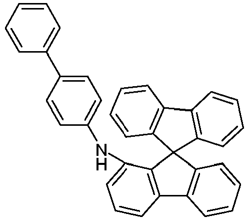 | 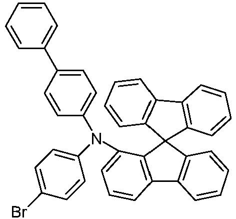 | 26 |
| 3b | 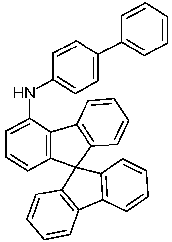 | 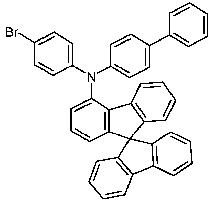 | 84 |
| 4b | 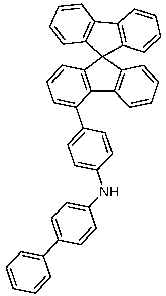 | 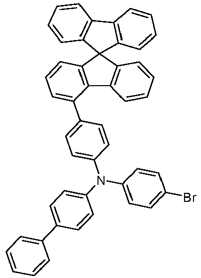 | 68 |
| 5b | 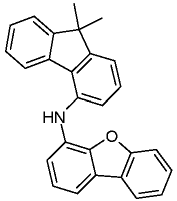 | 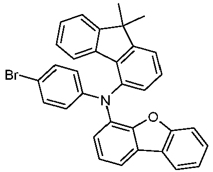 | 67 |
| 6b | 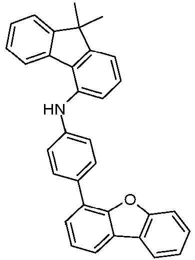 | 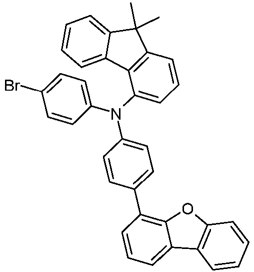 | 44 |
| 7b | 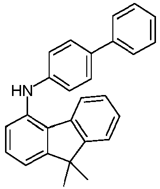 | 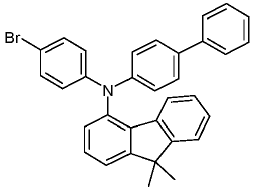 | 59 |
| 8b | 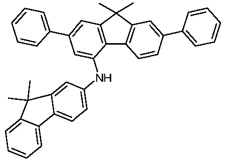 | 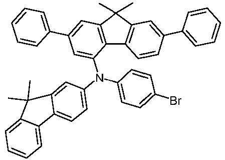 | 65 |
| 9b | 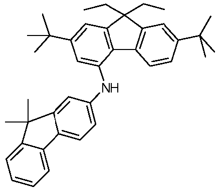 | 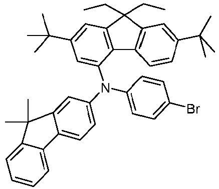 | 67 |
| 10b | 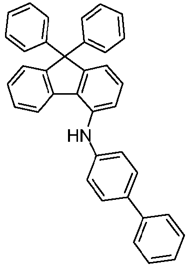 | 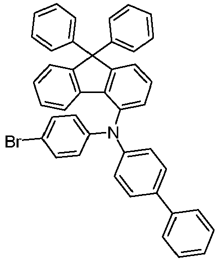 | 71 |
| 11b | 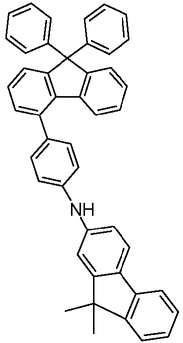 ]1852534-95-2] | 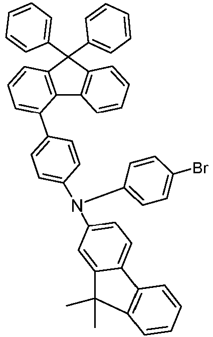 | 70 |

### c) Biphenyl-4-yl-(3'-brom-biphenyl-3-yl)-(9,9-dimethyl-9H-fluoren-2-yl)-amin

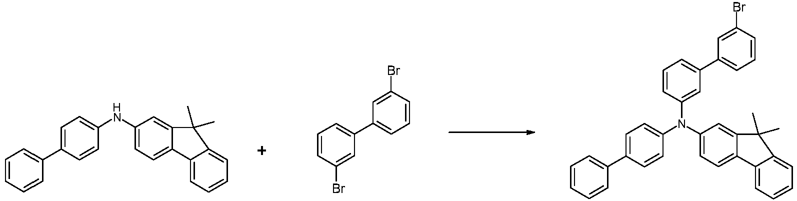

Es werden 29 g (80 mmol, 1,0 eq) des Zwischenprodukts aus Reaktion a) zusammen mit 25 g (80 mmol, 1,0 eq) 3,3'-Dibrom-1,1'-biphenyl (CAS 16400-51-4) in 600 ml Toluol gelöst und für 30 Minuten entgast. Anschließend werden 45 g (240 mmol, 3,0 eq) Natrium-tert-butylat, 890 mg (0,40 mmol, 0,050 eq) Palladium(II)-acetat und 8 ml (8,0 mmol, 0,10 eq.) einer 1M tri-tert-Butylphosphin-Lösung zugegeben. Der Ansatz wird über Nacht unter Rückfluss erhitzt und nach beendeter Reaktion zweimal über Aluminiumoxid mit Toluol filtriert. Nach Entfernung des Lösungsmittels am Rotationsverdampfer wird das Öl in etwas THF gelöst und in Heptan eingetragen. Der entstandene Feststoff wird abgesaugt und mittels Heißextraktion in Heptan/Toluol 1:1 aufgereinigt. Es werden 16,6 g (28 mmol, 35%) des gewünschten Produkts erhalten.

Analog können die folgenden Verbindungen dargestellt werden:

| | **Edukt 3** | **Edukt 4** | **Produkt** | **Ausbeute** |
|---|---|---|---|---|
| **1c** | 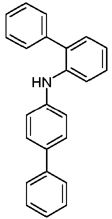 | 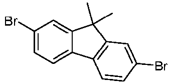 28320-32-3 | 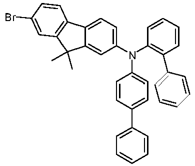 | 49% |
| **2c** | 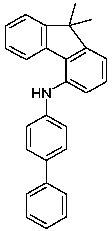 | 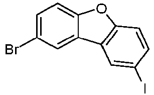 [1206544-88-8] | 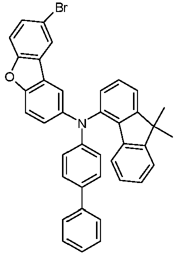 | 65% |
| **3c** | 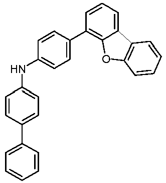 | 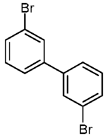 16400-51-4 | 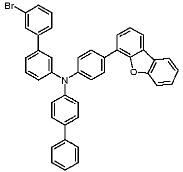 | 72% |
| **4c** | 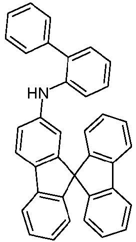 | 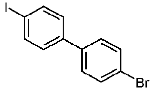 105946-82-5 | 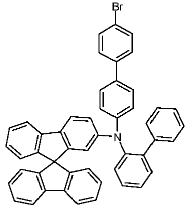 | 82% |
| **5c** | 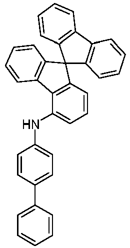 | 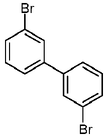 16400-51-4 | 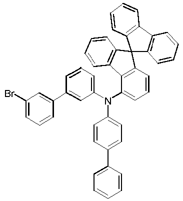 | 41% |
| **6c** | 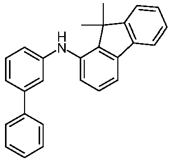 | 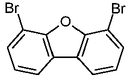 201138-91-2 | 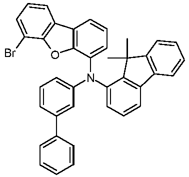 | 27% |
| **7c** | 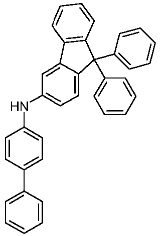 | 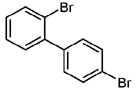 49602-91-7 | 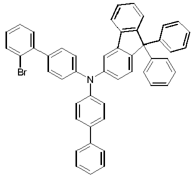 | 56% |
| **8c** | 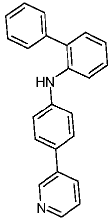 | 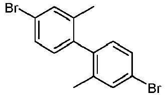 31458-17-0 | 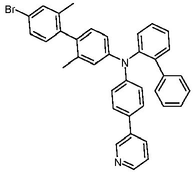 | 47% |
| **9c** | 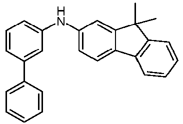 | 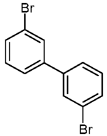 16400-51-4 | 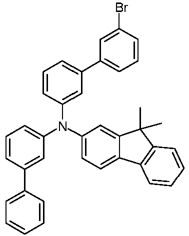 | 57% |
| **10c** | 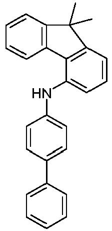 | 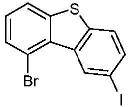 [1822311-12-5] | 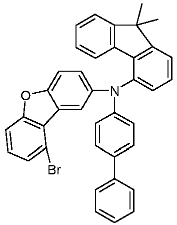 | 64 |
| **11c** | 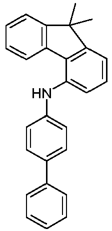 | 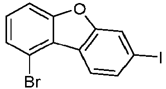 [1807910-82-2] | 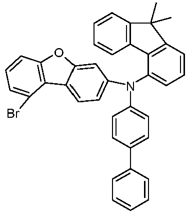 | 67 |
| **12c** | 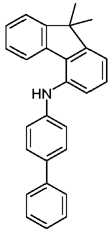 | 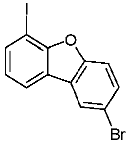 [1643716-58-8] | 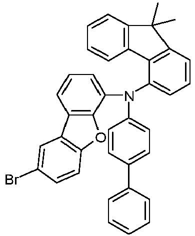 | 72 |
| **13c** | 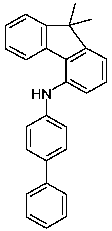 | 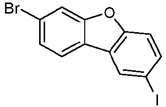 [1627589-28-9] | 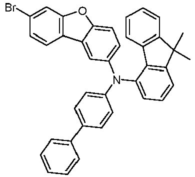 | 59 |

### d) Biphenyl-4-yl-(9,9-dimethyl-9H-fluoren-1-yl)-[4-(4-phenyl-chinazolin-2-yl)-phenyl]-amin

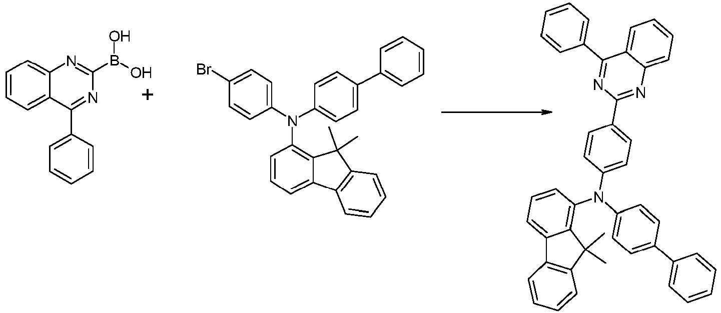

27,5 g (110,0 mmol) 4-Phenyl-chinazolin-2-boronsäure, 56 g (110,0 mmol) Biphenyl-4-yl-(4-bromphenyl)-(9,9-dimethyl-9H-fluoren-1-yl)-amin und 26 g (210,0 mmol) Natriumcarbonat werden in 500 mL Ethylenglycoldimethylether und 500 mL Wasser suspendiert. Zu dieser Suspension werden 913 mg (3,0 mmol) Tri-o-tolylphosphin und dann 112 mg (0,5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Das Produkt wird via Säulenchromatographie an Kieselgel mit Toluol/Heptan (1:2) gereinigt und abschließend im Hochvakuum (p = 5 x 10⁻⁷ mbar) sublimiert (Reinheit 99,9%). Die Ausbeute beträgt 53 g (83 mmol), entsprechend 73 % der Theorie.

Analog können die folgenden Verbindungen dargestellt werden:

| | **Edukt** 1 | **Edukt 2** | **Produkt** | **Ausbeute [%]** |
|---|---|---|---|---|
| 1d | 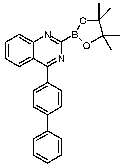 [1874309-53-1] | 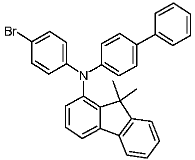 | 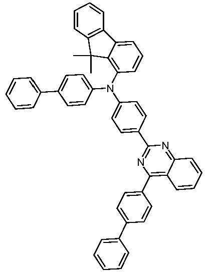 | 71 |
| 2d | 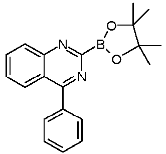 [1616841-71-4] | 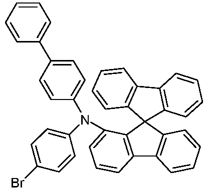 | 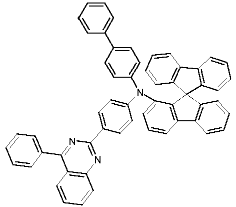 | 70 |
| 3d | 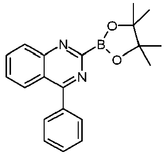 [1616841-71-4] | 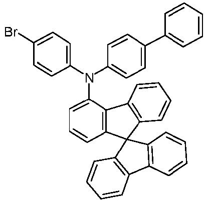 | 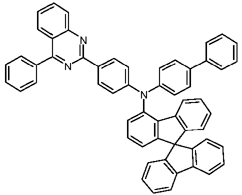 | 67 |
| 4d | 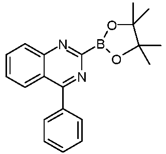 [1616841-71-4] | 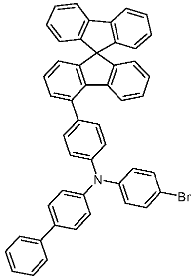 | 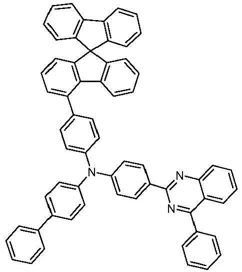 | 65 |
| 5d | 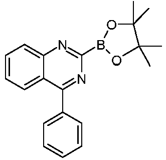 [1616841-71-4] | 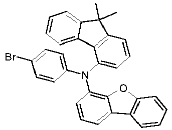 | 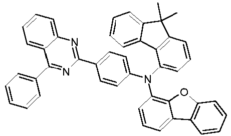 | 68 |
| 6d | 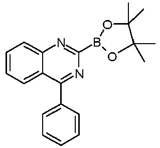 [1616841-71-4] | 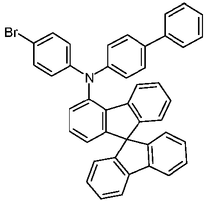 | 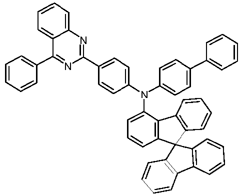 | 69 |
| 7d | 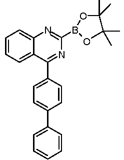 [1874309-53-1] | 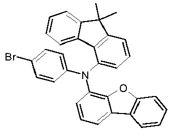 | 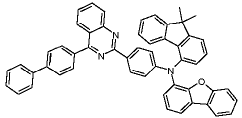 | 66 |
| 8d | 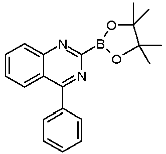 [1616841-71-4] | 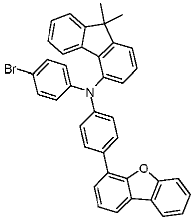 | 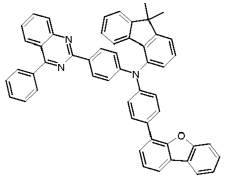 | 62 |
| 9d | 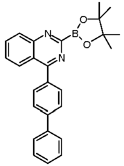 [1874309-53-1] | 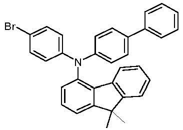 | 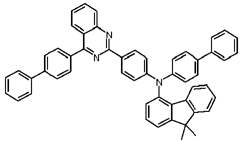 | 68 |
| 10d | 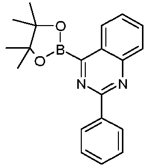 [1018833-74-3] | 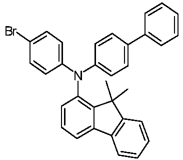 | 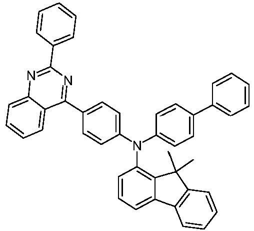 | 68 |
| 11d | 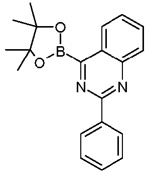 [1018833-74-3] | 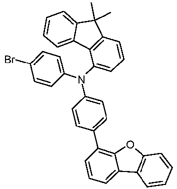 | 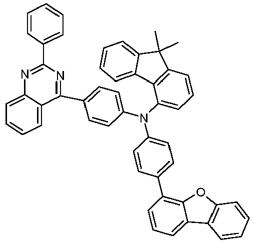 | 71 |
| 12d | 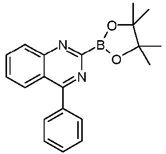 [1616841-71-4] | 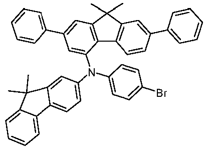 | 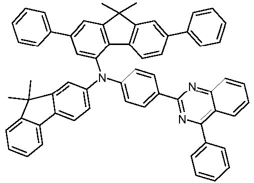 | 70 |
| 13d | 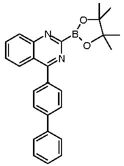 [1874309-53-1] | 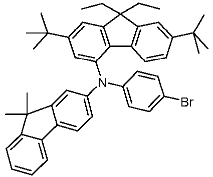 | 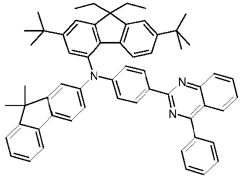 | 69 |
| 14d | 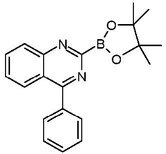 [1616841-71-4] | 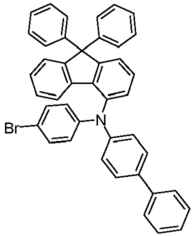 | 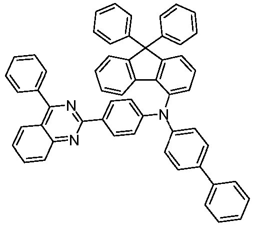 | 67 |
| 15d | 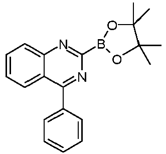 [1616841-71-4] | 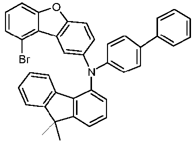 | 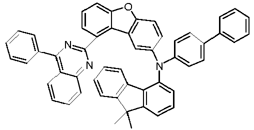 | 63 |
| 16d | 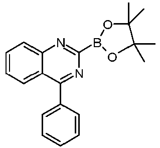 [1616841-71-4] | 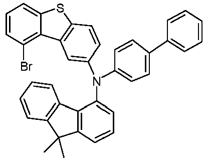 | 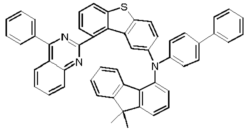 | 64 |
| 17d | 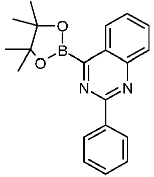 [1018833-74-3] | 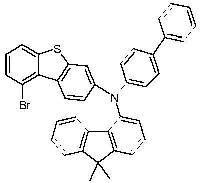 | 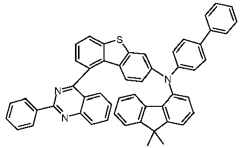 | 67 |
| 18d | 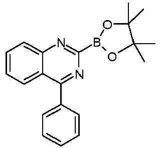 [1616841-71-4] | 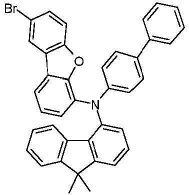 | 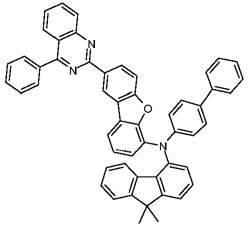 | 60 |
| 19d | 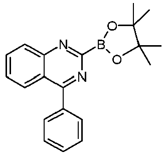 [1616841-71-4] | 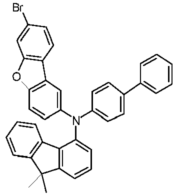 | 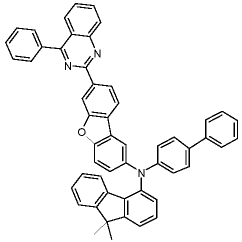 | 68 |
| 20d | 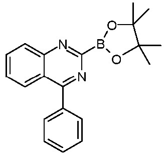 [1616841-71-4] | 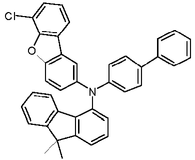 | 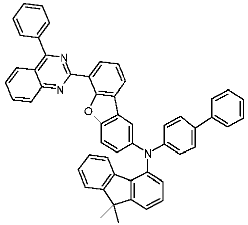 | 67 |
| 21d | 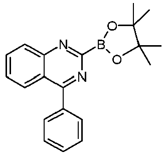 [1616841-71-4] | 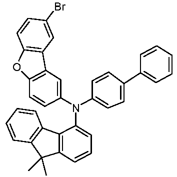 | 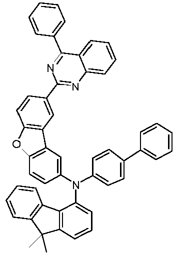 | 62 |
| 22d | 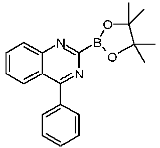 [1616841-71-4] | 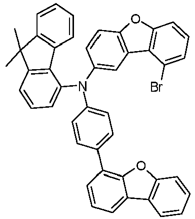 | 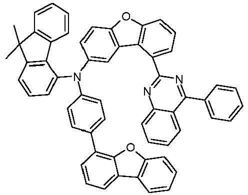 | 64 |
| 23d | 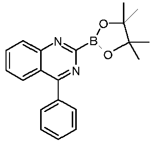 [1616841-71-4] | 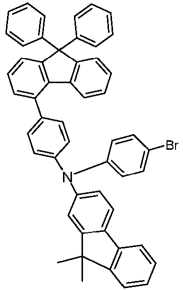 | 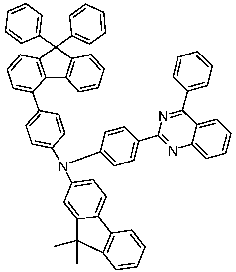 | 61 |
| 24d | 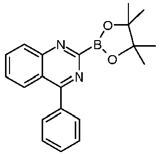 [1616841-71-4] | 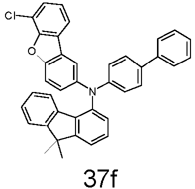 | 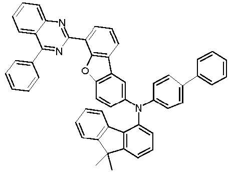 | 63 |
| 25d | 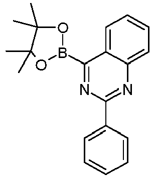 [1018833-74-3] | 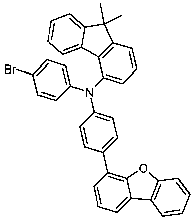 | 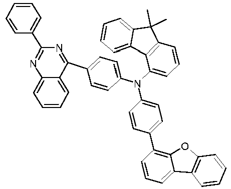 | 67 |
| 26d | 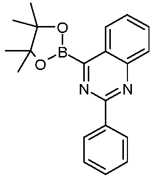 [1018833-74-3] | 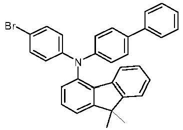 | 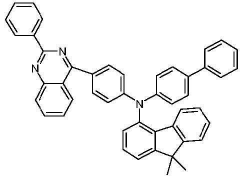 | 65 |
| 27d | 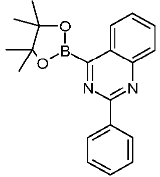 [1018833-74-3] | 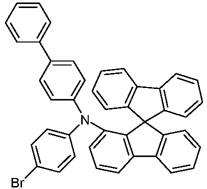 | 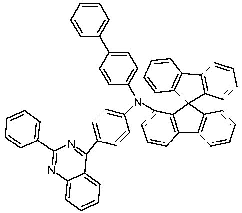 | 63 |
| 30d | 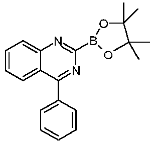 [1616841-71-4] | 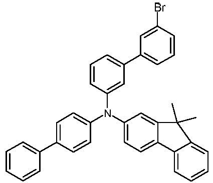 | 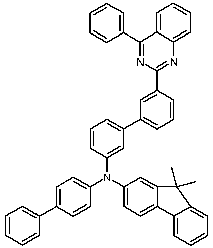 | 61 |
| 31d | 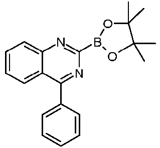 [1616841-71-4] | 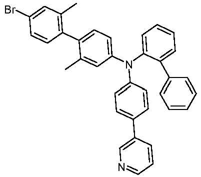 | 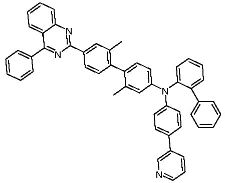 | 55 |

### e) Biphenyl-4-yl-(9,9-dimethyl-9H-fluoren-4-yl)-(4-phenyl-chinazolin-2-yl)-amin

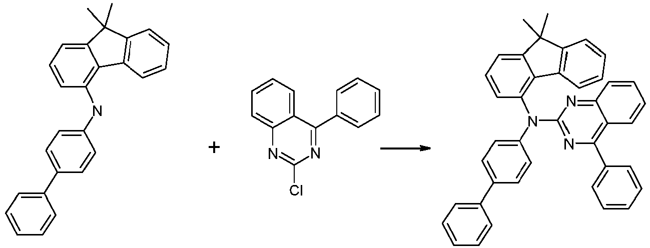

Ein Gemisch aus 14,4 g (60 mmol) 2-Chlor-4-phenyl-chinazolin, 18,7 g (60 mmol) Biphenyl-4-yl-(9,9-dimethyl-9H-fluoren-4-yl)-amin, 7,7 g (80 mmol) Natrium-tert-butylat, 1,4 g (5 mmol) Tricyclohexylamin, 561 mg (2,5 mmol) Palladium(II)acetat und 300 ml Mesitylen wird 24 h unter Rückfluss erhitzt. Nach Erkalten versetzt man mit 200 ml Wasser, rührt 30 min. nach, trennt die organische Phase ab, filtriert diese über ein kurzes Celite-Bett und entfernt dann das Lösungsmittel im Vakuum. Der Rückstand wird fünfmal aus DMF umkristallisiert und abschließend zweimal fraktioniert sublimiert (p ca. 10⁻⁶ mbar, T = 350 - 380 °C). Ausbeute: 23 g (40 mmol), 69 % der Theorie: 99,9 % n. HPLC.

Analog können die folgenden Verbindungen dargestellt werden:

| **Bsp.** | **Edukt 1** | **Edukt 2** | **Produkt** | **Ausbeute [%]** |
|---|---|---|---|---|
| 1e | 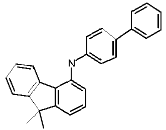 | 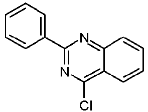 [6484-25-9] | 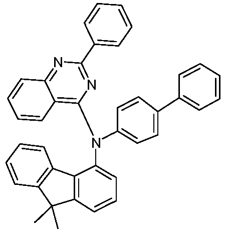 | 65 |
| 2e | 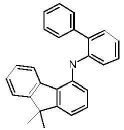 | 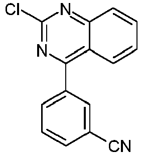 [1292317-90-8] | 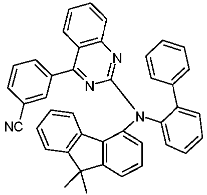 | 71 |
| 3e | 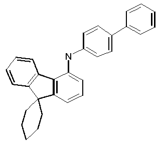 | 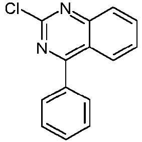 [29874-83-7] | 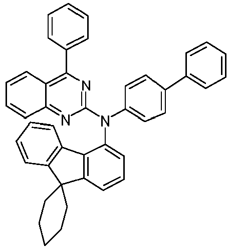 | 70 |
| 4e | 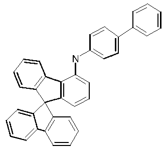 | 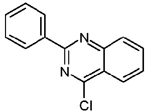 [6484-25-9] | 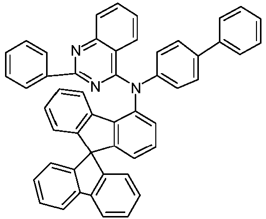 | 64 |
| 5e | 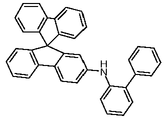 | 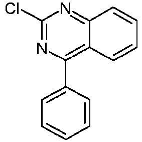 [29874-83-7] | 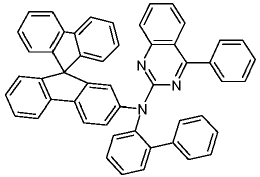 | 64 |
| 6e | 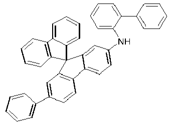 | 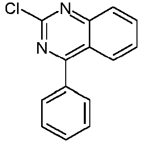 [29874-83-7] | 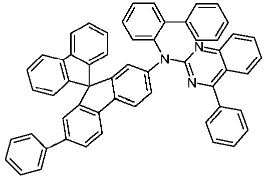 | 71 |
| 7e | 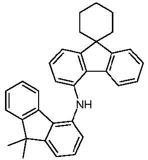 | 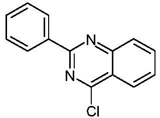 [6484-25-9] | 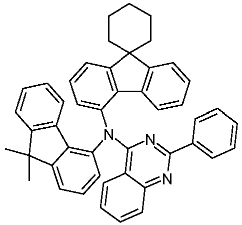 | 62 |
| 8e | 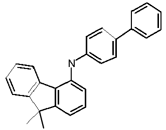 | 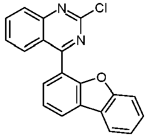 [14003252-55-0] | 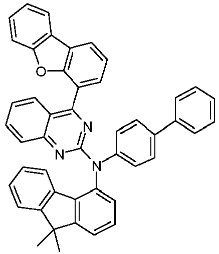 | 71 |
| 9e | 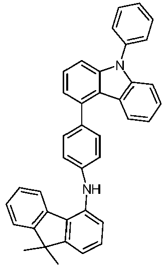 | 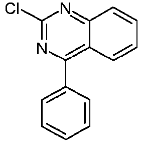 [29874-83-7] | 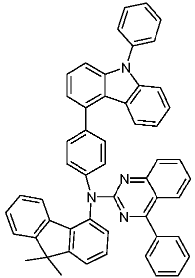 | 64 |
| 10e | 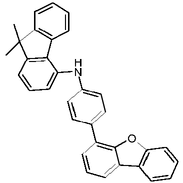 | 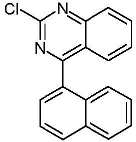 [1413376-86-9] | 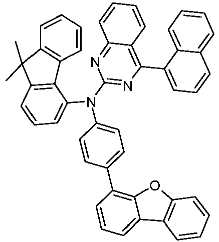 | 65 |
| 11e | 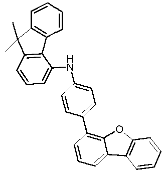 | 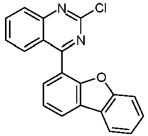 [14003252-55-0] | 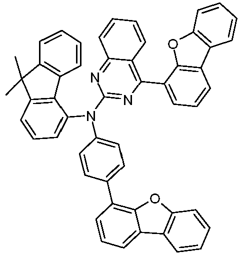 | 54 |
| 12e | 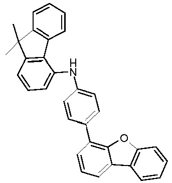 | 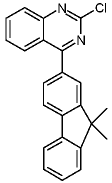 [1373265-66-7] | 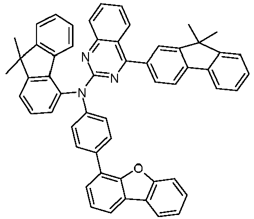 | 63 |
| 13e | 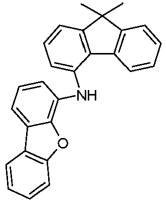 | 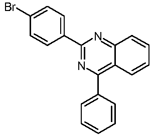 [540466-42-0] | 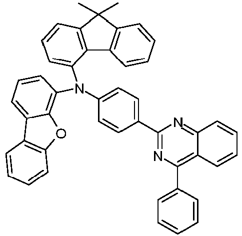 | 62 |
| 14e | 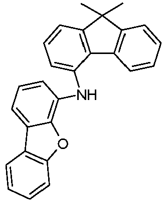 | 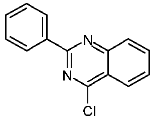 [6484-25-9] | 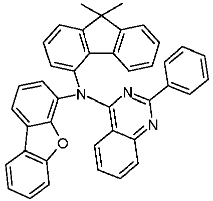 | 57 |
| 15e | 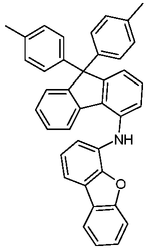 | 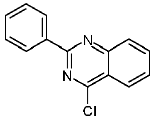 [6484-25-9] | 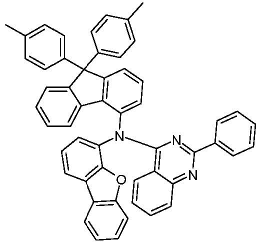 | 61 |
| 16e | 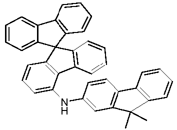 | 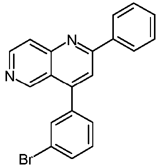 [1632307-99-3] | 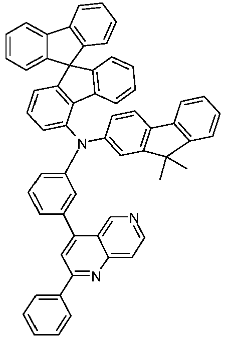 | 62 |
| 17e | 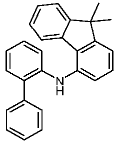 | 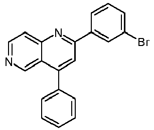 [1632307-97-1] | 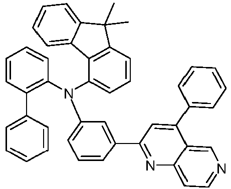 | 57 |
| 18e | 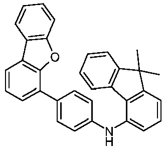 | 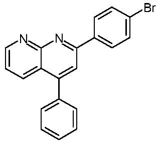 [1632294-77-9] | 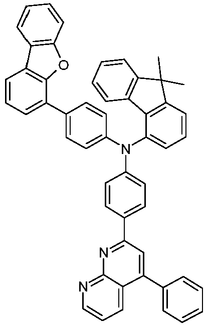 | 66 |
| 19e | 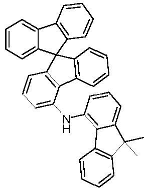 | 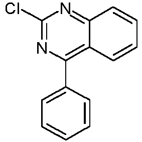 [29874-83-7] | 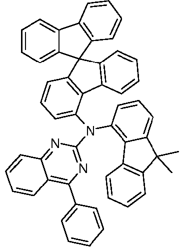 | 58 |
| 20e | 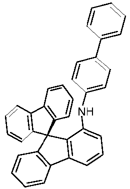 | 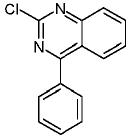 [29874-83-7] | 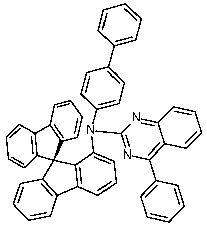 | 67 |
| 21e | 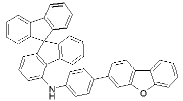 | 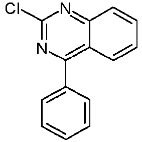 [29874-83-7] | 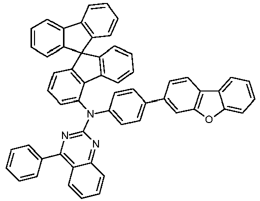 | 76 |
| 22e | 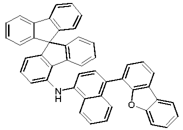 | 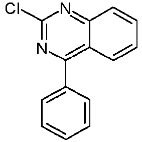 [29874-83-7] | 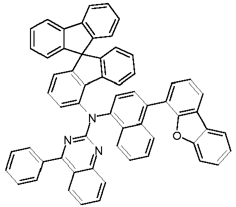 | 70 |
| 23e | 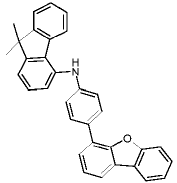 | 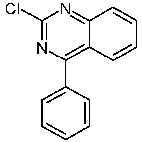 [29874-83-7] | 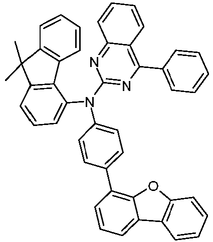 | 68 |
| 24e | 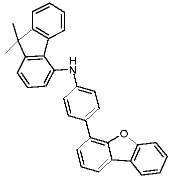 | 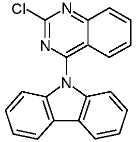 [1262866-84-1] | 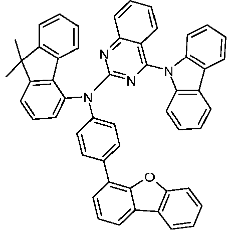 | 71 |
| 25e | 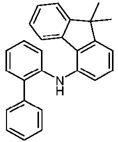 | 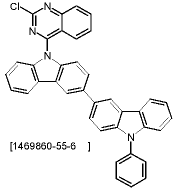 | 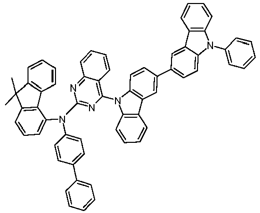 | 64 |
| 26e | 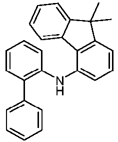 | 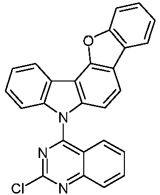 [1469860-57-8] | 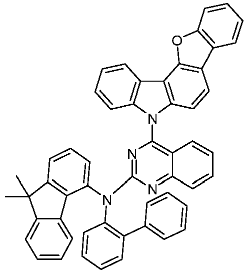 | 58 |
| 27e | 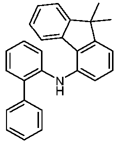 | 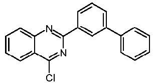 [1561960-15-3] | 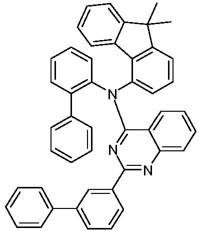 | 63 |
| 28e | 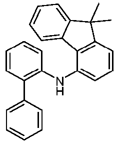 | 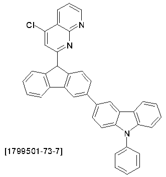 | 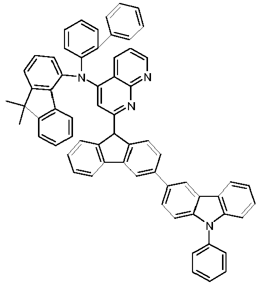 | 67 |

### f) 9-(2-Chlor-chinazolin-4-yl)-3-phenyl-9H-carbazol (nicht erfindungsgemäß)

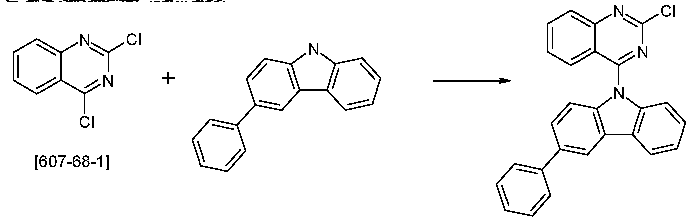

14,4 g (60 mmol) 3-Phenyl-9H-carbazol werden in 300 ml Dimethylformamid unter Schutzgasatmosphäre gelöst und mit 3 g NaH, 60%ig in Mineralöl (75 mmol) versetzt. Nach 1 h bei Raumtemperatur wird eine Lösung von 12,3 g (62 mmol) 2,4-Dichlor-chinazolin in 150 mL Dimethylformamid zugetropft. Das Reaktionsgemisch wird dann 12 h bei Raumtemperatur gerührt, auf Eis gegossen und dreimal mit Dichlormethan extrahiert. Die vereinigten organischen Phasen werden über Na₂SO₄ getrocknet und eingeengt. Der Rückstand wird mit Toluol umkristallisiert. Die Ausbeute beträgt 21 g (51 mmol), entsprechend 75 % der Theorie.

Analog können die folgenden Verbindungen dargestellt werden:

| **Bsp.** | **Edukt 1** | **Edukt 2** | **Produkt** | **Ausbeute [%]** |
|---|---|---|---|---|
| 1f | 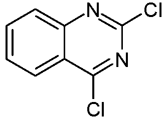 [607-68-1] | 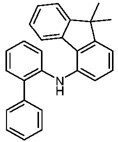 | 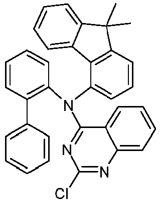 | 55 |
| 2f | 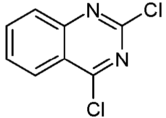 [607-68-1] | 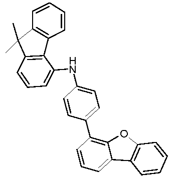 | 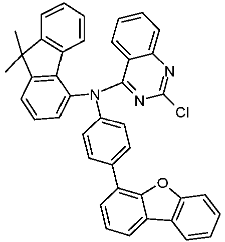 | 57 |
| 3f | 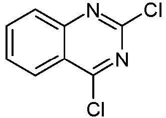 [607-68-1] | 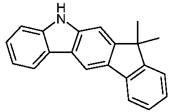 [1257220-47-5] | 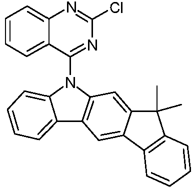 | 57 |
| 4f | 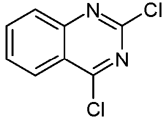 [607-68-1] | 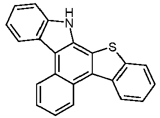 [1313395-18-4] | 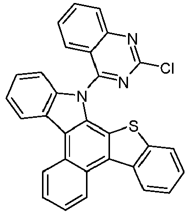 | 55 |

Analog können die folgenden Verbindungen dargestellt werden:

| **Bsp.** | **Edukt 1** | **Edukt 2** | **Produkt** | **Ausbeute [%]** |
|---|---|---|---|---|
| 5f | 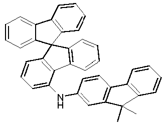 | 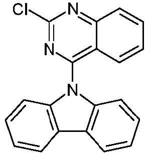 [1262866-84-1] | 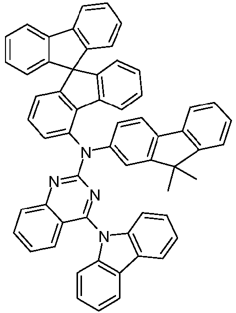 | 66 |
| 6f | 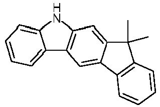 [1257220-47-5] | 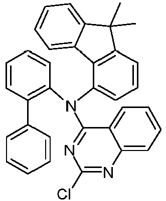 | 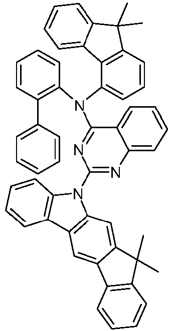 | 65 |
| 7f | 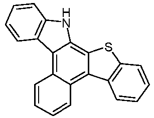 [1313395-18-4] | 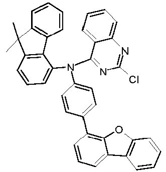 | 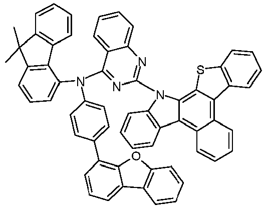 | 57 |
| 8f | 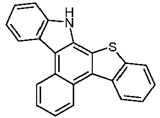 [1313395-18-4] | 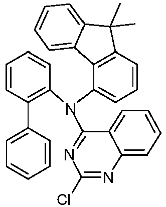 | 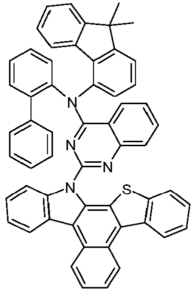 | 53 |
| 9f | 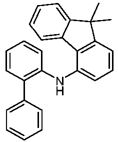 | 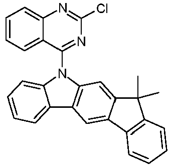 | 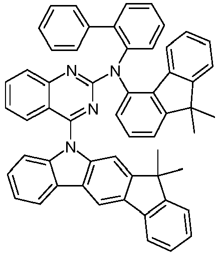 | 54 |
| 10f | 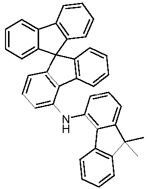 | 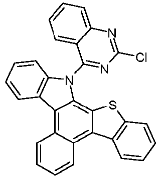 | 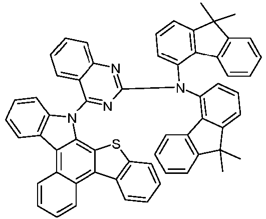 | 50 |
| 11f | 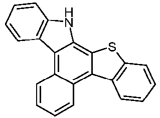 [1313395-18-4] | 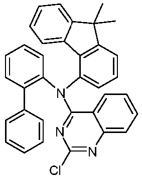 | 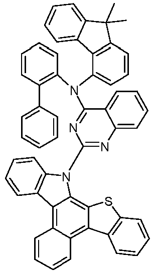 | 51 |
| 12f | 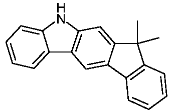 [1257220-47-5] | 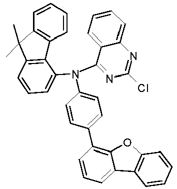 | 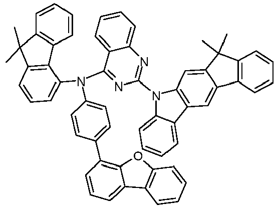 | 55 |
| 13f | 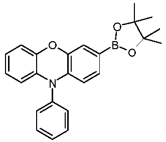 [1333324-90-5] | 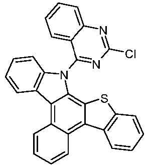 | 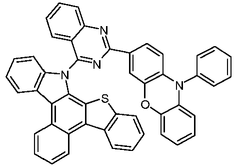 | 51 |
| 14f | 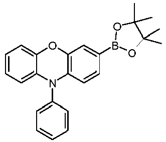 [1333324-90-5] | 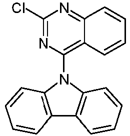 [1262866-84-1] | 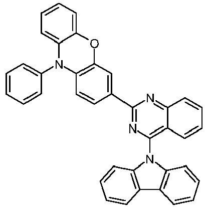 | 56 |

Die Verbindungen 5f bis 14f werden nach Umkristallisation sublimiert (p ca. 10⁻⁶ mbar, T = 350 - 390 °C).

### Herstellung der OLEDs

In den folgenden Beispielen E1 bis E20 (siehe Tabelle 1) wird der Einsatz der erfindungsgemäßen Materialien in OLEDs vorgestellt.

**Vorbehandlung für die Beispiele E1-E20:** Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie EG1:TER (95%:5%) bedeutet hierbei, dass das Material EG1 in einem Volumenanteil von 95% und TER in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik bestimmt. Die Elektrolumineszenzspektren werden bei einer Leuchtdichte von 1000 cd/m² bestimmt und daraus die CIE 1931 x und y Farbkoordinaten berechnet.

### Verwendung von erfindungsgemäßen Mischungen in OLEDs

Die erfindungsgemäße Verbindung EG1 zeigt in dem Aufbau einer OLED gemäß Beispiel E1 bei einer Leuchtdichte von 1000 cd/m² eine Spannung von 3.9 V und eine Effizienz von 24 cd/A bei einer Farbkoordinate von CIEx=0.67 und CIEy= 0.33.

Die erfindungsgemäßen Verbindungen EG2 bis EG17 werden in den Beispielen E2 bis E17 als Matrixmaterial in der Emissionsschicht eingesetzt. Die Farbkoordinaten der Elektrolumineszenzspektren der OLEDs aus diesen Versuchen liegen bei CIEx=0.67 und CIEy= 0.33. Somit eignen sich die Materialien für den Einsatz in der Emissionsschicht von roten OLEDs.

Des Weiteren lassen sich die erfindungsgemäßen Materialien in der Elektronentransportschicht (ETL) bzw. der Lochblockierschicht (HBL) erfolgreich einsetzen. Dies ist in den Versuchen E18 - E20 gezeigt. Auch hier liegen die Farbkoordinaten des Spektrums der OLED bei CIEx=0.67 und CIEy= 0.33.

**Tabelle 1: Aufbau der OLEDs**

| Bsp | HIL Dicke | HTL Dicke | EBL Dicke | EML Dicke | HBL Dicke | ETL Dicke | EIL Dicke |
|---|---|---|---|---|---|---|---|
| E1 | HATCN 5nm | SpMA1 125nm | SpMA2 10nm | EG1:TER (95%:5%) 40nm | --- | ST:LiQ (50%:50%) 35nm | --- |
| E2 | HATCN 5nm | SpMA1 125nm | SpMA2 10nm | EG2:TER (95%:5%) 40nm | --- | ST:LiQ (50%:50%) 35nm | --- |
| E3 | HATCN 5nm | SpMA1 125nm | SpMA2 10nm | EG3:TER (95%:5%) 40nm | --- | ST:LiQ (50%:50%) 35nm | --- |
| E4 | HATCN 5nm | SpMA1 125nm | SpMA2 10nm | EG4:TER (95%:5%) 40nm | --- | ST:LiQ (50%:50%) 35nm | --- |
| E5 | HATCN 5nm | SpMA1 125nm | SpMA2 10nm | EG5:TER (95%:5%) 40nm | --- | ST:LiQ (50%:50%) 35nm | --- |
| E6 | HATCN 5nm | SpMA1 125nm | SpMA2 10nm | EG6:TER (95%:5%) 40nm | --- | ST:LiQ (50%:50%) 35nm | --- |
| E7 | HATCN 5nm | SpMA1 125nm | SpMA2 10nm | EG7:TER (95%:5%) 40nm | --- | ST:LiQ (50%:50%) 35nm | --- |
| E8 | HATCN 5nm | SpMA1 125nm | SpMA2 10nm | EG8:TER (95%:5%) 40nm | --- | ST:LiQ (50%:50%) 35nm | --- |
| E9 | HATCN 5nm | SpMA1 125nm | SpMA2 10nm | EG9:TER (95%:5%) 40nm | --- | ST:LiQ (50%:50%) 35nm | --- |
| E10 | HATCN 5nm | SpMA1 125nm | SpMA2 10nm | EG10:TER (95%:5%) 40nm | --- | ST:LiQ (50%:50%) 35nm | --- |
| E11 | HATCN 5nm | SpMA1 125nm | SpMA2 10nm | EG11:TER (95%:5%) 40nm | --- | ST:LiQ (50%:50%) 35nm | --- |
| E12 | HATCN 5nm | SpMA1 125nm | SpMA2 10nm | EG12:TER (95%:5%) 40nm | --- | ST:LiQ (50%:50%) 35nm | --- |
| E13 | HATCN 5nm | SpMA1 125nm | SpMA2 10nm | EG13:TER (95%:5%) 40nm | --- | ST:LiQ (50%:50%) 35nm | --- |
| E14 | HATCN 5nm | SpMA1 125nm | SpMA2 10nm | EG14:TER (95%:5%) 40nm | --- | ST:LiQ (50%:50%) 35nm | --- |
| E15 | HATCN 5nm | SpMA1 125nm | SpMA2 10nm | EG15:TER (95%:5%) 40nm | --- | ST:LiQ (50%:50%) 35nm | --- |
| E16 | HATCN 5nm | SpMA1 125nm | SpMA2 10nm | EG16:TER (95%:5%) 40nm | --- | ST:LiQ (50%:50%) 35nm | --- |
| E17 | HATCN 5nm | SpMA1 125nm | SpMA2 10nm | EG17:TER (95%:5%) 40nm | --- | ST:LiQ (50%:50%) 35nm | --- |
| E18 | HATCN 5nm | SpMA1 125nm | SpMA2 10nm | EG1:TER (95%:5%) 40nm | --- | EG18:LiQ (50%:50%) 35nm | --- |
| E19 | HATCN 5nm | SpMA1 125nm | SpMA2 10nm | EG1:TER (95%:5%) 40nm | EG19 5nm | ST:LiQ (50%:50%) 35nm | --- |
| E20 | HATCN 5nm | SpMA1 125nm | SpMA2 10nm | EG1:TER (95%:5%) 40nm | EG20 5nm | ST:LiQ (50%:50%) 35nm | --- |

**Tabelle 2: Strukturformeln der Materialien für die OLEDs**

| | |
|---|---|
| 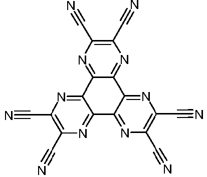 | 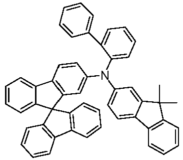 |
| HATCN | SpMA1 |
| 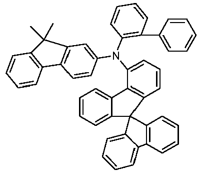 | 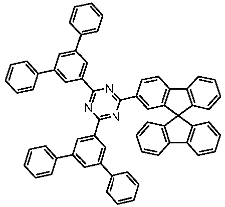 |
| SpMA2 | ST |
| 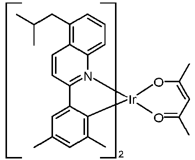 | 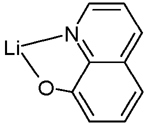 |
| TER | LiQ |
| 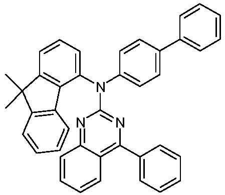 | 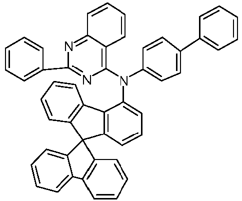 |
| EG1 | EG2 |
| 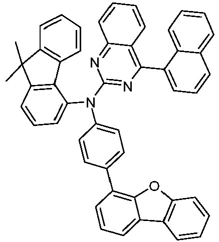 | 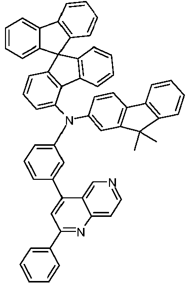 |
| EG3 | EG4 |
| 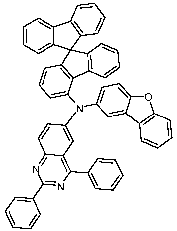 | 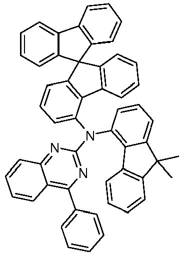 |
| EG5 | EG6 |
| 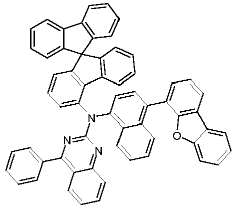 | 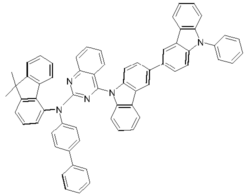 |
| EG7 | EG8 |
| 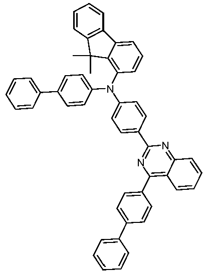 | 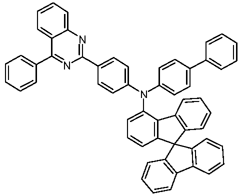 |
| EG9 | EG10 |
| 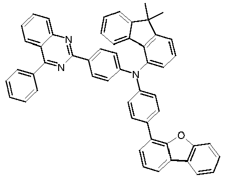 | 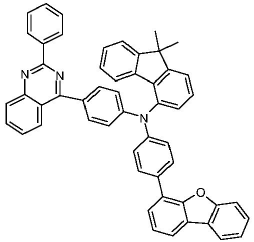 |
| EG11 | EG12 |
| 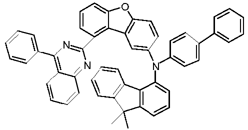 | 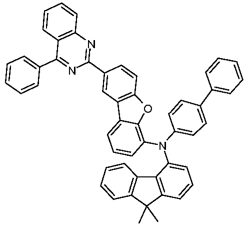 |
| EG13 | EG14 |
| 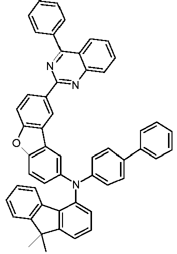 | 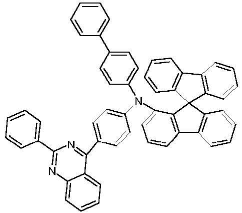 |
| EG15 | EG16 |
| 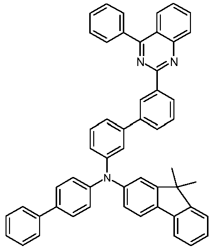 | 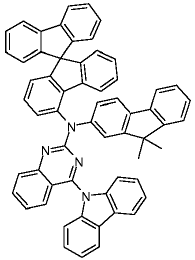 |
| EG17 | EG18 |
| 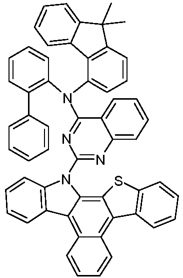 | 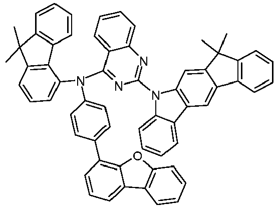 |
| EG19 | EG20 |

## Patentansprüche

1. Verbindung, umfassend mindestens eine Struktur der Formel (IIIa), (IIIb), (XXIa), (XXIb), (XXIVa) oder (XXIVb), 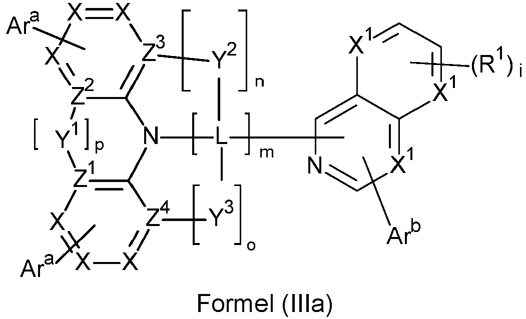 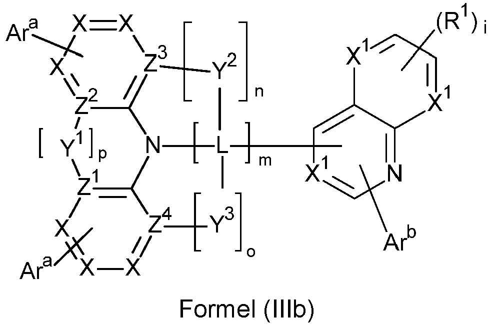 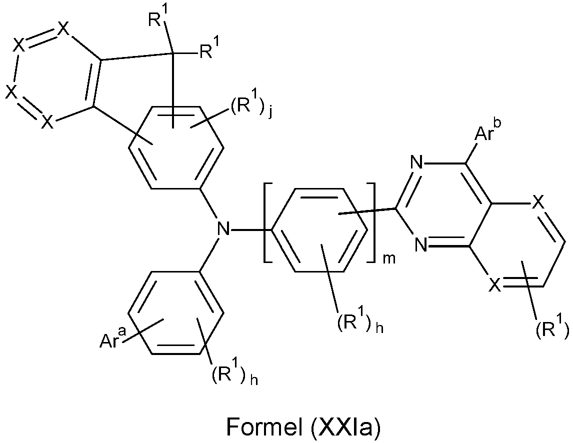 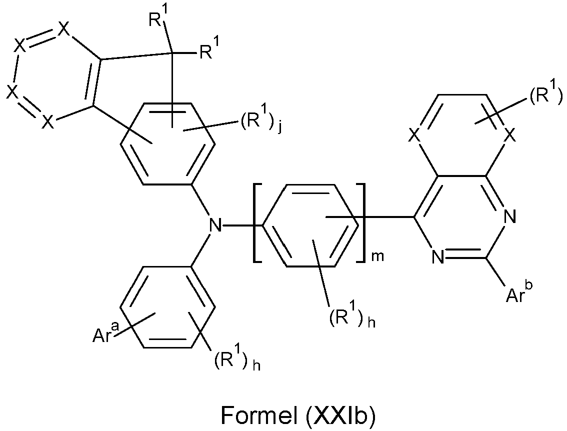 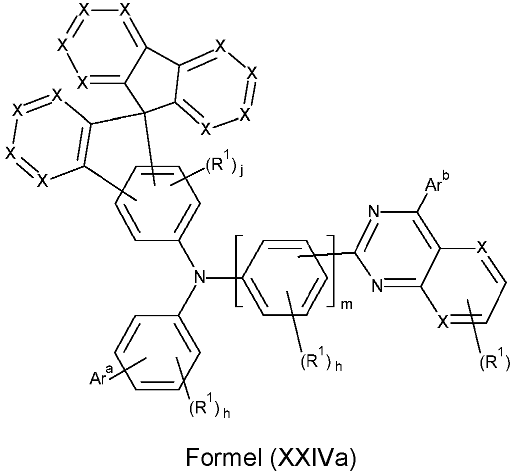 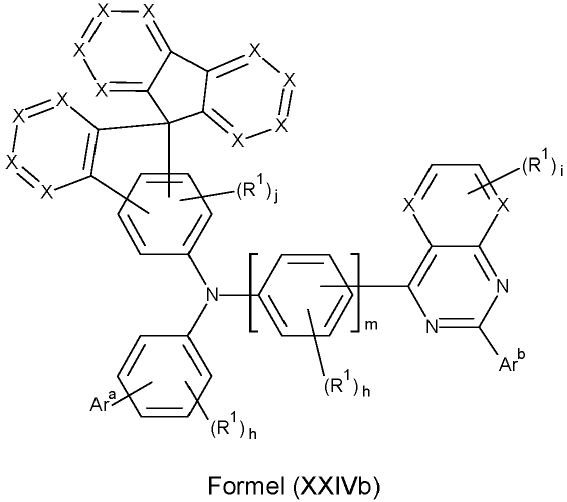
wobei für die verwendeten Symbole gilt:
L ist ein aromatisches oder heteroaromatisches Ringsystem mit 5 bis 30 aromatischen Ringatomen, das mit einem oder mehreren nicht-aromatischen Resten R¹ substituiert sein kann;
Z¹, Z², Z³, Z⁴ sind X;
X ist bei jedem Auftreten gleich oder verschieden N oder CR¹, wobei maximal zwei X pro Ring für N stehen, vorzugsweise CR¹, oder C, falls an X ein Rest Ar^{a} bindet;
X¹ ist bei jedem Auftreten gleich oder verschieden N oder CR¹;
n, o, p ist 0;
m ist 0 oder 1;
i ist 0, 1 oder 2;
j ist 0, 1, 2 oder 3;
h ist 0, 1, 2, 3 oder 4;
Ar^{a} ist bei jedem Auftreten gleich oder verschieden ein aromatisches oder heteroaromatisches Ringsystem mit 5 bis 30 aromatischen Ringatomen, welches durch einen oder mehrere Reste R¹ substituiert sein kann, wobei der Rest Ar^{a} keine Carbazolgruppe umfasst oder mit der Aryl- oder Heteroarylgruppe bildet, an die Ar^{a} bindet, wobei dies Substituenten R¹, R² und R³, die an den Rest Ar^{a} gebunden sein können, einschließt;
Ar^{b} ist ein aromatisches oder heteroaromatisches Ringsystem mit 5 bis 45 aromatischen Ringatomen, welches durch einen oder mehrere Reste R¹ substituiert sein kann;
R¹ ist bei jedem Auftreten gleich oder verschieden H, D, CN, N(Ar¹)₂, eine geradkettige Alkylgruppe mit 1, 2, 3 oder 4 C-Atomen oder eine verzweigte oder cyclische Alkylgruppe mit 3 bis 8 C-Atomen, oder ein aromatisches oder heteroaromatisches Ringsystem mit 5 bis 40 aromatischen Ringatomen, das jeweils durch einen oder mehrere Reste R² substituiert sein kann, oder eine Kombination dieser Systeme; dabei können zwei oder mehrere Substituenten R¹ auch miteinander ein monocyclisches aliphatisches oder ein aromatisches Ringsystem bilden;
Ar¹ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R² substituiert sein kann; dabei können zwei Reste Ar¹, welche an dasselbe Si-Atom, N-Atom, P-Atom oder B-Atom binden, auch durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verbrückt sein;
R² ist bei jedem Auftreten gleich oder verschieden H, D, CN, eine geradkettige Alkylgruppe mit 1, 2, 3 oder 4 C-Atomen oder eine verzweigte Alkylgruppe mit 3 oder 4 C-Atomen, die jeweils mit einem oder mehreren Resten R³ substituiert sein kann, wobei ein oder mehrere H-Atome durch D oder CN ersetzt sein können, oder ein aromatisches oder heteroaromatisches Ringsystem mit 5 bis 40 aromatischen Ringatomen, das jeweils durch einen oder mehrere Reste R³ substituiert sein kann, oder eine Kombination dieser Systeme; dabei können zwei oder mehrere Substituenten R² auch miteinander ein monocyclisches aliphatisches oder ein aromatisches Ringsystem bilden;
R³ ist bei jedem Auftreten gleich oder verschieden ausgewählt aus der Gruppe bestehend aus H, D, CN, einem aliphatischen Kohlenwasserstoffrest mit 1, 2, 3 oder 4 C-Atomen oder einem aromatischen oder heteroaromatischen Ringsystem mit 5 bis 30 aromatischen Ringatomen, in dem ein oder mehrere H-Atome durch D oder CN ersetzt sein können und das durch ein oder mehrere Alkylgruppen mit jeweils 1 bis 4 Kohlenstoffatomen substituiert sein kann; dabei können zwei oder mehr Substituenten R³ miteinander ein monocyclisches aliphatisches Ringsystem bilden;
mit der Maßgabe, dass mindestens eine Gruppe X¹ in Formel (IIIa) bzw. (IIIb) für N steht, und
mit der Maßgabe, dass Verbindungen der Formel (A) 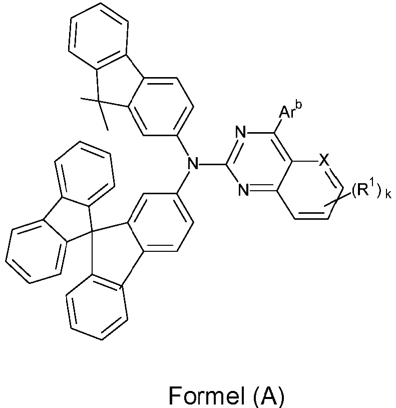
ausgenommen sind, wobei die verwendeten Symbole X, Ar^{b} und R¹ die zuvor dargelegte Bedeutung aufweisen und k 0 oder 1 ist.

2. Verbindung gemäß Anspruch 1, umfassend mindestens eine Struktur der Formel (Va) oder (Vb), 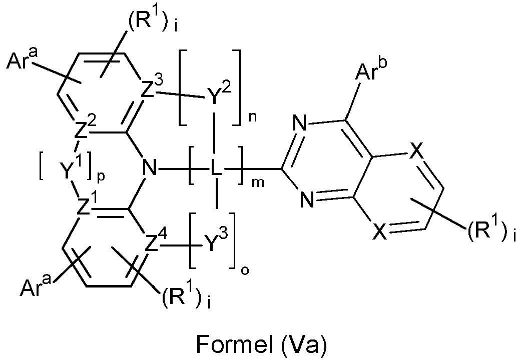 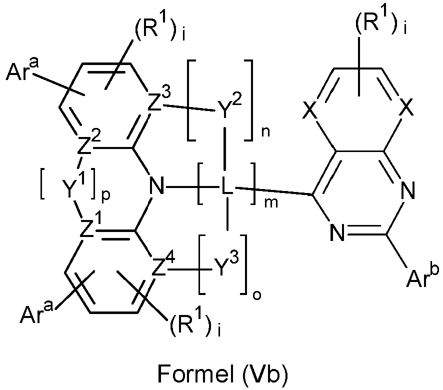 wobei die verwendeten Symbole m, n, o, p, L, R¹, Ar^{a}, Ar^{b}, Z¹, Z², Z³, Z⁴ und X die in Anspruch 1 genannte Bedeutung aufweisen und i für 0, 1 oder 2 steht.

3. Verbindung gemäß Anspruch 2, umfassend mindestens eine Struktur der Formel (XVIIIa) oder (XVIIIb), 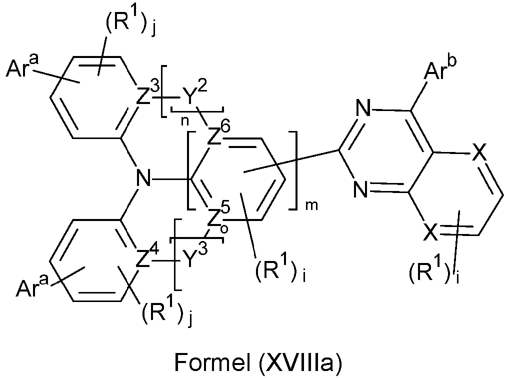 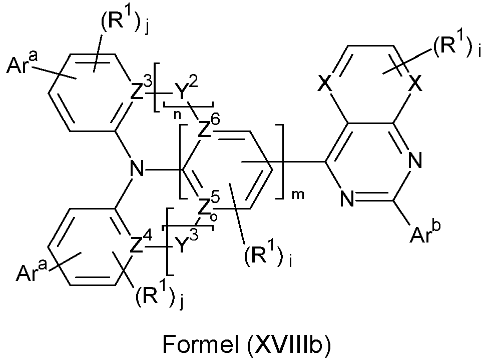 wobei die verwendeten Symbole m, n, o, Ar^{a}, Ar^{b}, Z³, Z⁴ und X die in Anspruch 1 genannte Bedeutung aufweisen, Z⁵ und Z⁶ gleich oder verschieden für X oder C stehen, i für 0, 1 oder 2 und j für 0, 1, 2 oder 3 steht.

4. Verbindung gemäß Anspruch 3, umfassend mindestens eine Struktur der Formel (XIXa) oder (XIXb), 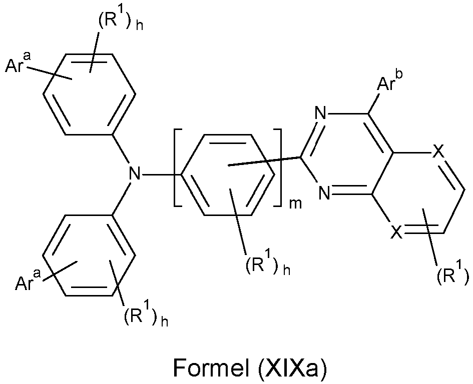 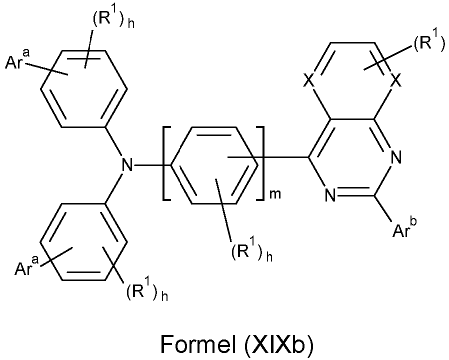 wobei die verwendeten Symbole m, Ar^{a}, Ar^{b}, R¹ und X die in Anspruch 1 genannte Bedeutung aufweisen, i für 0, 1 oder 2 und h für 0, 1, 2, 3 oder 4 steht.

5. Verbindung gemäß Anspruch 1, umfassend mindestens eine Struktur der Formel (XXIIa) oder (XXIIb), 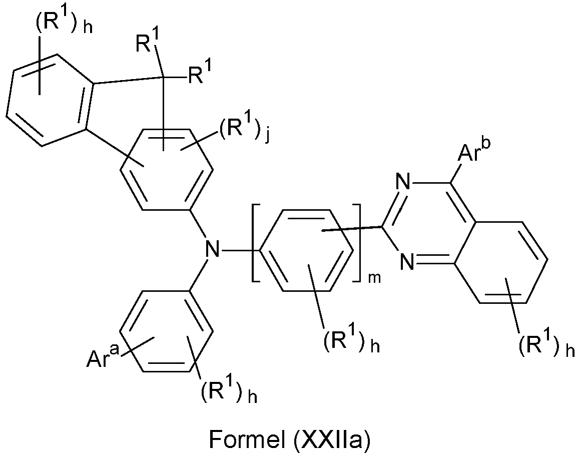 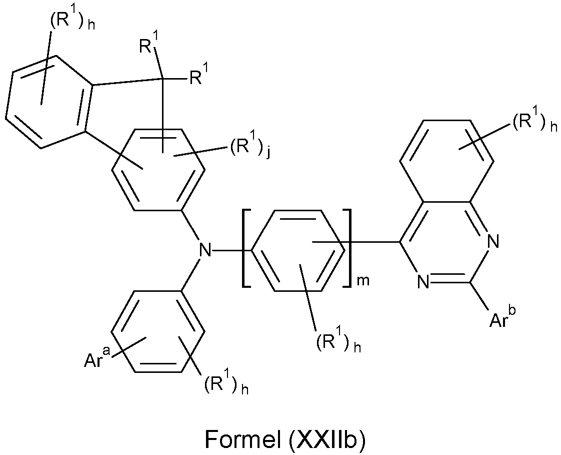 wobei die verwendeten Symbole m, Ar^{a}, Ar^{b} und R¹ die in Anspruch 1 genannte Bedeutung aufweisen, j für 0, 1, 2 oder 3 und h für 0, 1, 2, 3 oder 4 steht.

6. Verbindung gemäß Anspruch 1, umfassend mindestens eine Struktur der Formel (XXIIIa), (XXIIIb), (XXIIIc), (XXIIId), (XXIIIe) oder (XXIIIf), 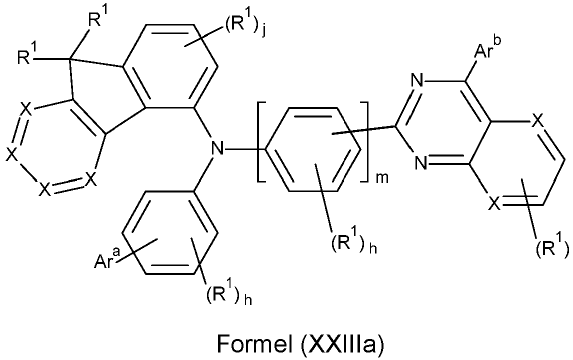 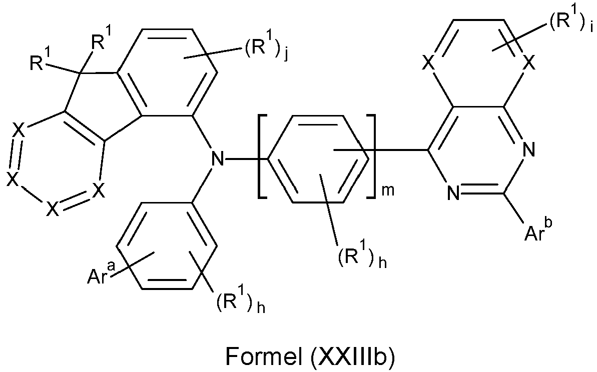 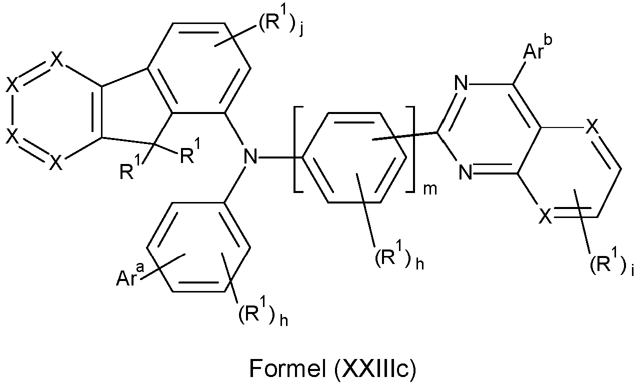 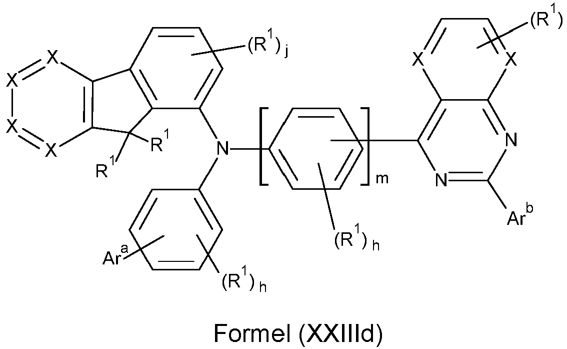 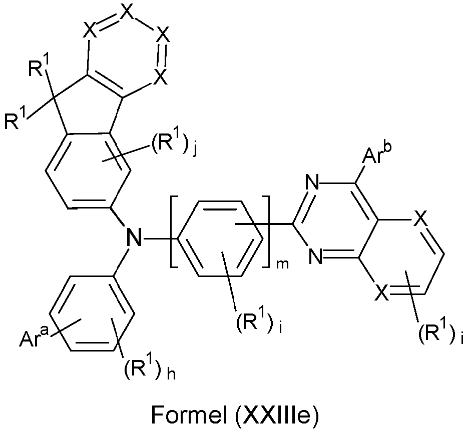 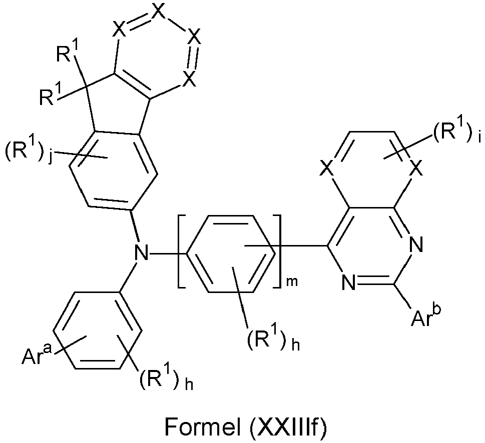 wobei die verwendeten Symbole m, Ar^{a}, Ar^{b}, R¹ und X die in Anspruch 1 genannte Bedeutung aufweisen, i für 0, 1 oder 2, j für 0, 1, 2 oder 3 und h für 0, 1, 2, 3 oder 4 steht.

7. Verbindung gemäß einem der Ansprüche 1 bis 6, umfassend mindestens eine Struktur der Formel (XXVa), (XXVb), (XXVc), (XXVd), (XXVe), (XXVf), (XXVg) oder (XXVh), 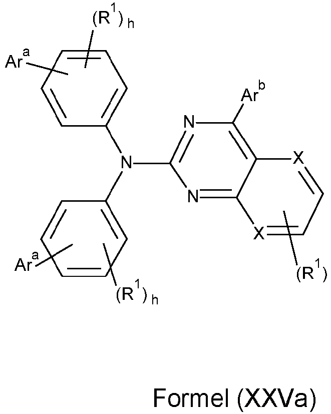 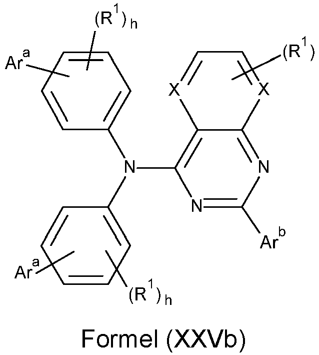 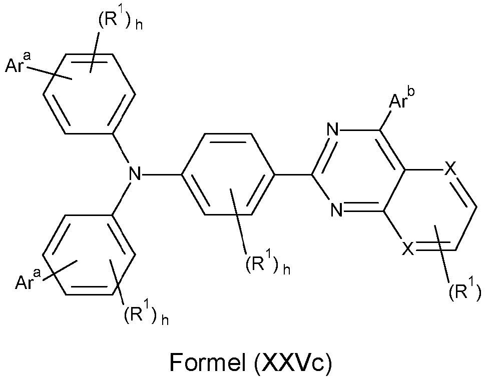 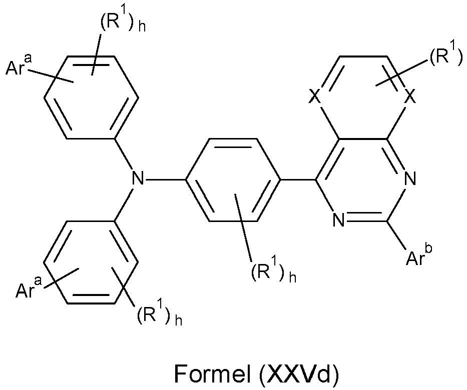 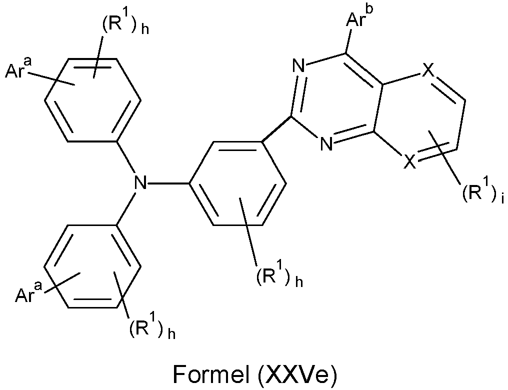 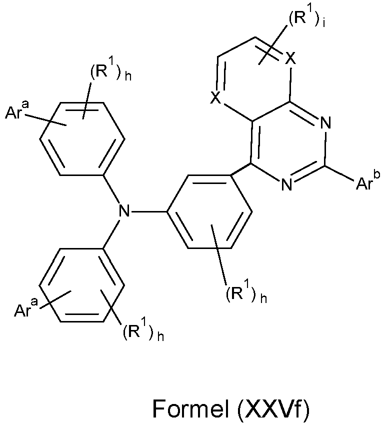 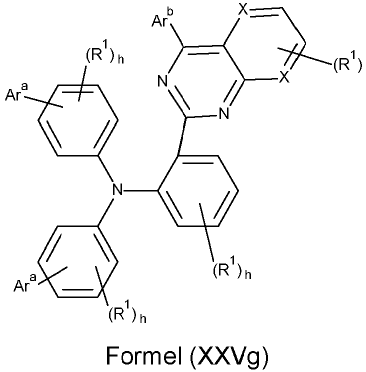 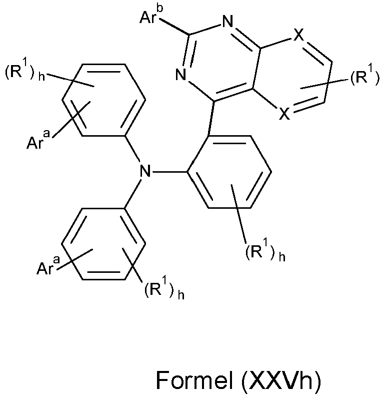 wobei die verwendeten Symbole Ar^{a}, Ar^{b}, R¹ und X die in Anspruch 1 genannte Bedeutung aufweisen, i für 0, 1 oder 2 und h für 0, 1, 2, 3 oder 4 steht.

8. Verbindung gemäß mindestens einem der vorhergehenden Ansprüche, **dadurch gekennzeichnet, dass** die Gruppe Ar^{b} eine Gruppe der Formel (Ar^{b}-1) darstellt, 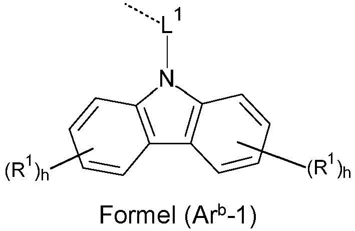 worin L' eine Bindung oder ein aromatisches oder heteroaromatisches Ringsystem mit 5 bis 30 aromatischen Ringatomen ist, welches durch einen oder mehrere Reste R¹ substituiert sein kann, das Symbol R¹ die in Anspruch 1 genannte Bedeutung aufweist, h 0, 1, 2, 3 oder 4 ist und die gestrichelte Linie die Bindung darstellt.

9. Oligomer, Polymer oder Dendrimer enthaltend eine oder mehrere Verbindungen nach einem oder mehreren der Ansprüche 1 bis 8, wobei statt eines Wasserstoffatoms oder eines Substituenten eine oder mehrere Bindungen der Verbindungen zum Polymer, Oligomer oder Dendrimer vorhanden sind.

10. Zusammensetzung enthaltend wenigstens eine Verbindung nach einem oder mehreren der Ansprüche 1 bis 8 oder ein Oligomer, Polymer oder Dendrimer nach Anspruch 9 und wenigstens eine weitere Verbindung ausgewählt aus der Gruppe bestehend aus fluoreszierenden Emittern, phosphoreszierenden Emittern, Emittern, die TADF zeigen, Hostmaterialien, Elektronentransportmaterialien, Elektroneninjektionsmaterialien, Lochleitermaterialien, Lochinjektionsmaterialien, Elektronenblockiermaterialien und Lochblockiermaterialien.

11. Formulierung, enthaltend mindestens eine Verbindung nach einem oder mehreren der Ansprüche 1 bis 8 und/oder ein Oligomer, Polymer oder Dendrimer nach Anspruch 9 und/oder eine Zusammensetzung nach Anspruch 10 und mindestens ein Lösemittel.

12. Verwendung einer Verbindung nach einem oder mehreren der Ansprüche 1 bis 8, eines Oligomers, Polymers oder Dendrimers nach Anspruch 9 oder einer Zusammensetzung nach Anspruch 10 in einer elektronischen Vorrichtung, insbesondere als Hostmaterial oder Elektronentransportmaterial.

13. Verfahren zur Herstellung einer Verbindung nach einem oder mehreren der Ansprüche 1 bis 8 oder eines Oligomers, Polymers und/oder Dendrimers nach Anspruch 9, **dadurch gekennzeichnet, dass** in einer Kupplungsreaktion eine Diarylamin-Verbindung mit einer Verbindung, umfassend mindestens eine Diazanaphthyl-Gruppe, verbunden wird.

14. Elektronische Vorrichtung, enthaltend mindestens eine Verbindung nach einem oder mehreren der Ansprüche 1 bis 8, ein Oligomer, Polymer oder Dendrimer nach Anspruch 9 oder eine Zusammensetzung gemäß Anspruch 10, wobei die elektronische Vorrichtung bevorzugt ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laserdioden.

## Claims

1. Compound comprising at least one structure of the formula (IIIa), (IIIb), (XXIa), XXIb), (XXIVa) or (XXIVb), 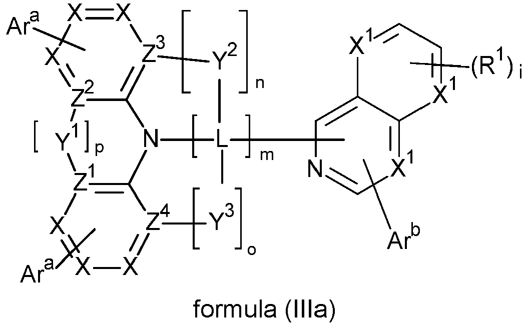 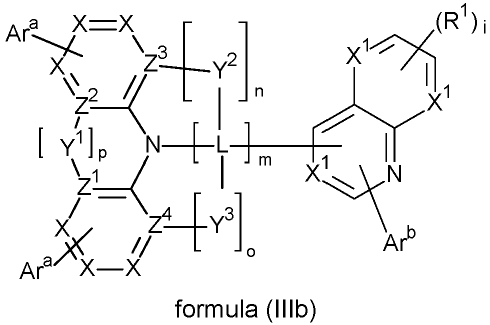 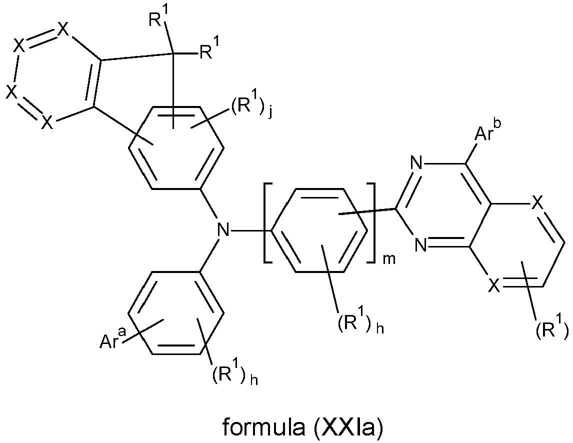 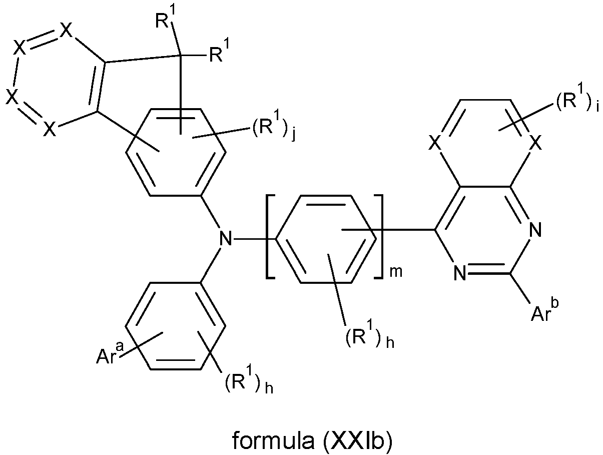 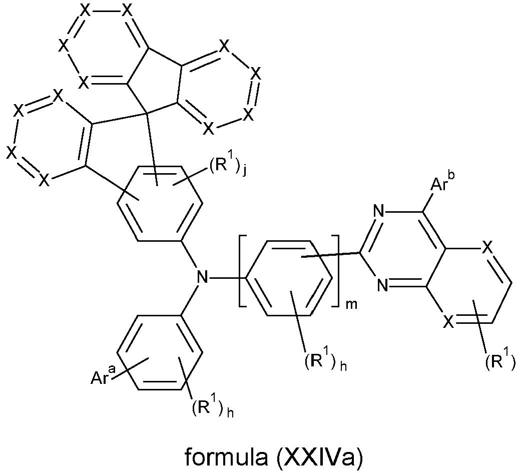 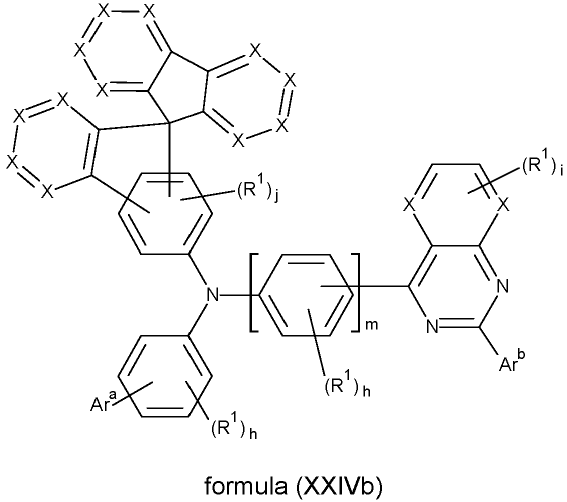
where the following applies to the symbols used:
L is an aromatic or heteroaromatic ring system having 5 to 30 aromatic ring atoms, which may be substituted by one or more non-aromatic radicals R¹;
Z¹, Z², Z³, Z⁴ are X;
X is on each occurrence, identically or differently, N or CR¹, where a maximum of two X per ring stand for N, preferably CR¹, or C if a radical Ar^{a} is bonded to X;
X¹ is on each occurrence, identically or differently, N or CR¹;
n, o, p are 0;
m is 0 or 1;
i is 0, 1 or 2;
j is 0, 1, 2 or 3;
h is 0, 1, 2, 3 or 4;
Ar^{a} is on each occurrence, identically or differently, an aromatic or heteroaromatic ring system having 5 to 30 aromatic ring atoms, which may be substituted by one or more radicals R¹, where the radical Ar^{a} does not comprise a carbazole group or form a carbazole group with the aryl or heteroaryl group to which Ar^{a} is bonded, where this includes substituents R¹, R² and R³ which may be bonded to the radical Ar^{a};
Ar^{b} is an aromatic or heteroaromatic ring system having 5 to 45 aromatic ring atoms, which may be substituted by one or more radicals R¹;
R¹ is on each occurrence, identically or differently, H, D, CN, N(Ar')₂, a straight-chain alkyl group having 1, 2, 3 or 4 C atoms or a branched or cyclic alkyl group having 3 to 8 C atoms, or an aromatic or heteroaromatic ring system having 5 to 40 aromatic ring atoms, which may in each case be substituted by one or more radicals R², or a combination of these systems; two or more substituents R¹ may also form a monocyclic aliphatic ring system or an aromatic ring system with one another;
Ar¹ is on each occurrence, identically or differently, an aromatic or heteroaromatic ring system having 5 to 30 aromatic ring atoms, which may be substituted by one or more non-aromatic radicals R²; two radicals Ar¹ that are bonded to the same Si atom, N atom, P atom or B atom may also be bridged to one another by a single bond or a bridge selected from B(R²), C(R²)₂, Si(R²)₂, C=O, C=NR², C=C(R²)₂, O, S, S=O, SO₂, N(R²), P(R²) and P(=O)R²;
R² is on each occurrence, identically or differently, H, D, CN, a straight-chain alkyl group having 1, 2, 3 or 4 C atoms or a branched alkyl group having 3 or 4 C atoms, which may in each case be substituted by one or more radicals R³, where one or more H atoms may be replaced by D or CN, or an aromatic or heteroaromatic ring system having 5 to 40 aromatic ring atoms, which may in each case be substituted by one or more radicals R³, or a combination of these systems; two or more substituents R² may also form a monocyclic aliphatic ring system or an aromatic ring system with one another;
R³ is selected on each occurrence, identically or differently, from the group consisting of H, D, CN, an aliphatic hydrocarbon radical having 1, 2, 3 or 4 C atoms or an aromatic or heteroaromatic ring system having 5 to 30 aromatic ring atoms, in which one or more H atoms may be replaced by D or CN and which may be substituted by one or more alkyl groups, each having 1 to 4 carbon atoms; two or more substituents R³ may form a monocyclic aliphatic ring system with one another;
with the proviso that at least one group X¹ in formula (IIIa) or (IIIb) stands for N, and
with the proviso that compounds of the formula (A) 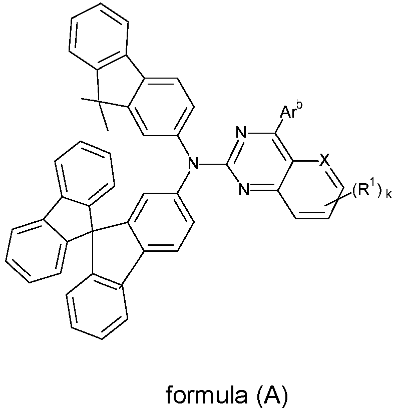
are excluded, where the symbols X, Ar^{b} and R¹ used have the meaning described above and k is 0 or 1.

2. Compound according to Claim 1, comprising at least one structure of the formula (Va) or (Vb), 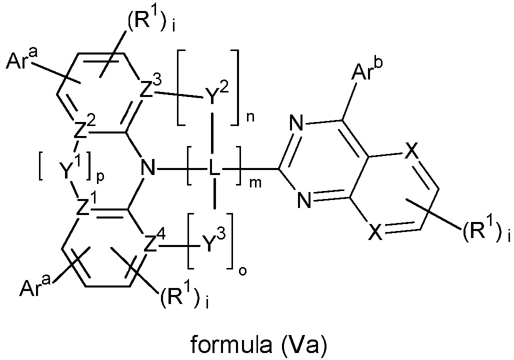 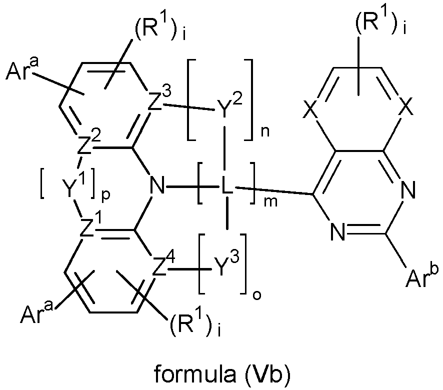 where the symbols m, n, o, p, L, R¹, Ar^{a}, Ar^{b}, Z¹, Z², Z³, Z⁴ and X used have the meaning given in Claim 1, and i stands for 0, 1 or 2.

3. Compound according to Claim 2, comprising at least one structure of the formula (XVIIIa) or (XVIIIb), 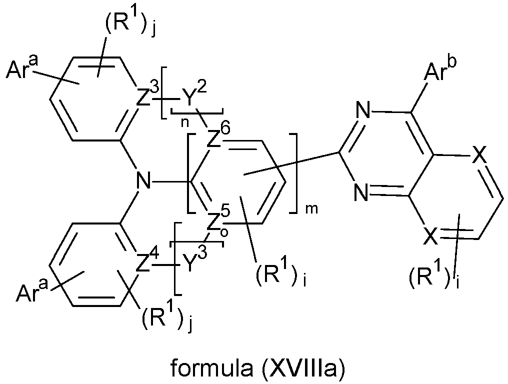 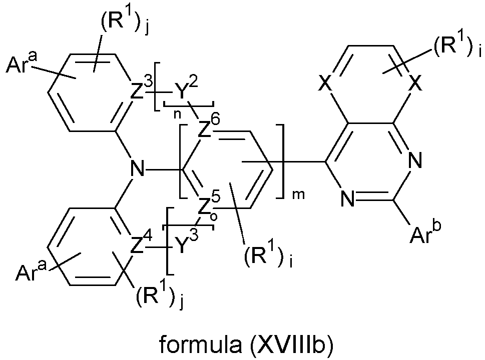 where the symbols m, n, o, Ar^{a}, Ar^{b}, Z³, Z⁴ and X used have the meaning given in Claim 1, Z⁵ and Z⁶ stand, identically or differently, for X or C, i stands for 0, 1 or 2 and j stands for 0, 1, 2 or 3.

4. Compound according to Claim 3, comprising at least one structure of the formula (XIXa) or (XIXb), 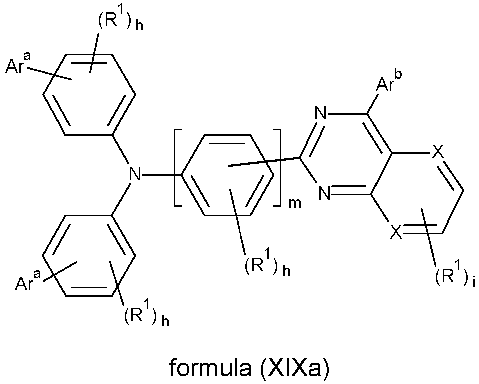 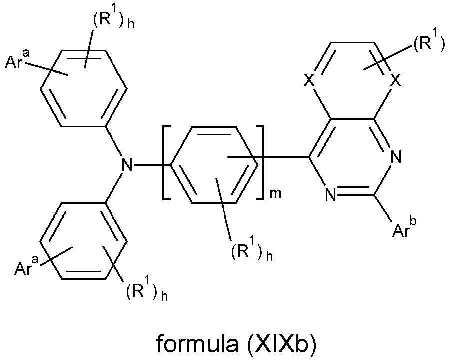 where the symbols m, Ar^{a}, Ar^{b}, R¹ and X used have the meaning given in Claim 1, i stands for 0, 1 or 2 and h stands for 0, 1, 2, 3 or 4.

5. Compound according to Claim 1, comprising at least one structure of the formula (XXIIa) or (XXIIb), 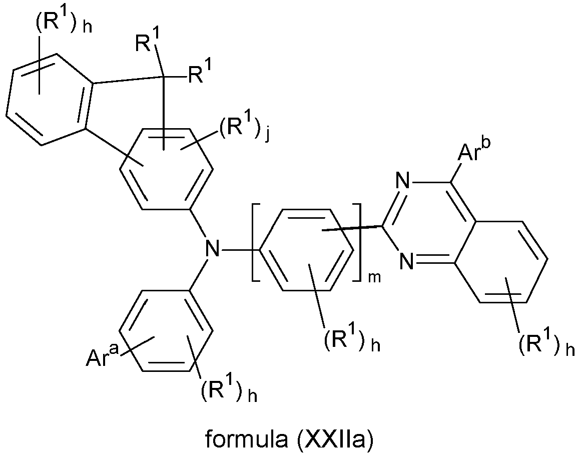 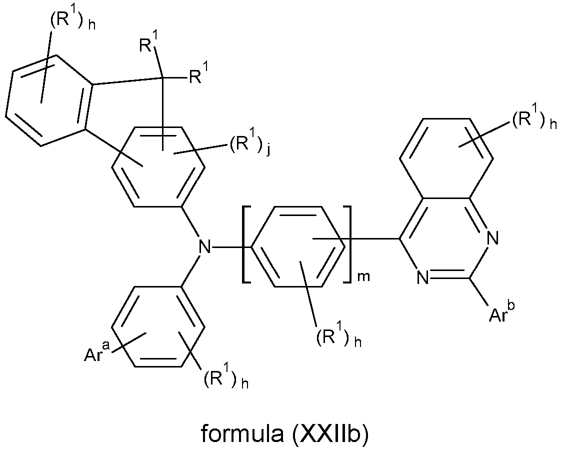 where the symbols m, Ar^{a}, Ar^{b} and R¹ used have the meaning given in Claim 1, j stands for 0, 1, 2 or 3 and h stands for 0, 1, 2, 3 or 4.

6. Compound according to Claim 1, comprising at least one structure of the formula (XXIIIa), (XXIIIb), (XXIIIc), (XXIIId), (XXIIIe) or (XXIIIf), 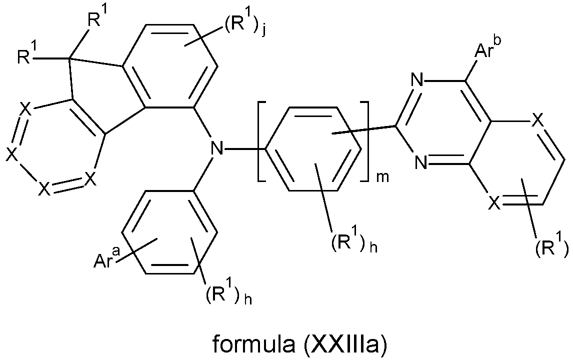 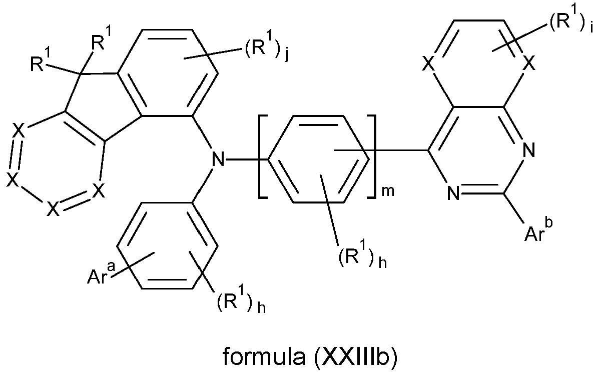 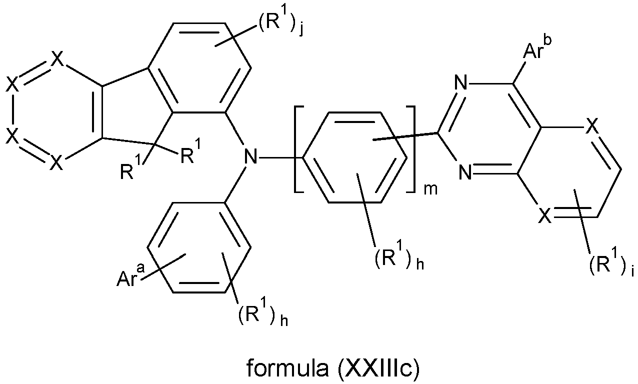 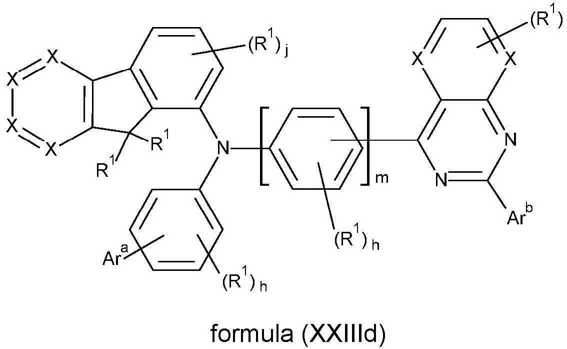 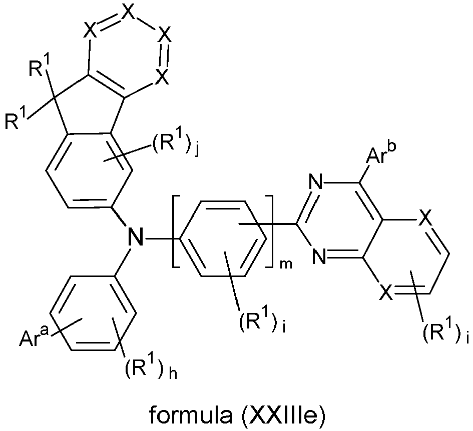 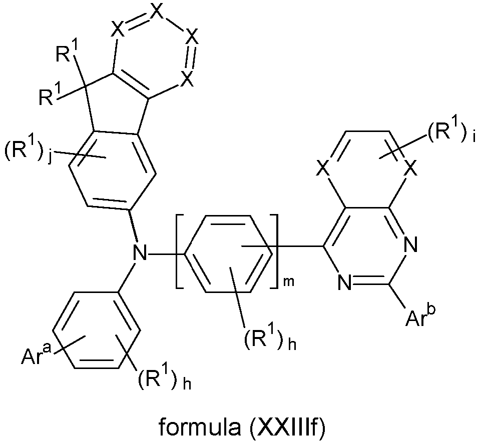 where the symbols m, Ar^{a}, Ar^{b}, R¹ and X used have the meaning given in Claim 1, i stands for 0, 1 or 2, j stands for 0, 1, 2 or 3 and h stands for 0, 1, 2, 3 or 4.

7. Compound according to one of Claims 1 to 6, comprising at least one structure of the formula (XXVa), (XXVb), (XXVc), (XXVd), (XXVe), (XXVf), (XXVg) or (XXVh), 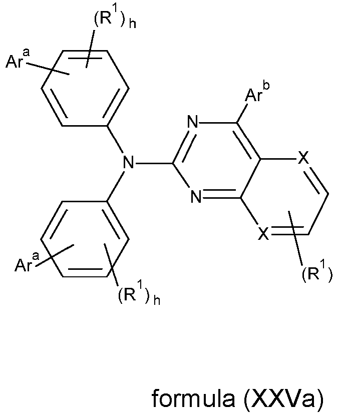 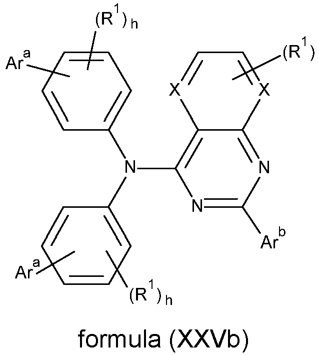 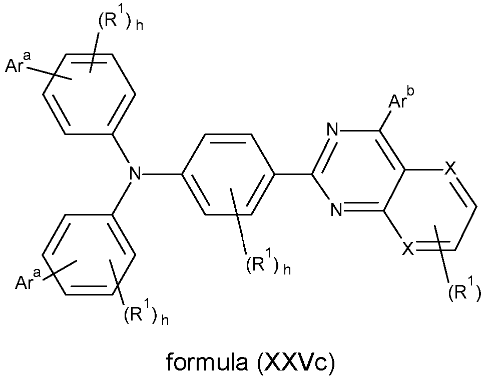 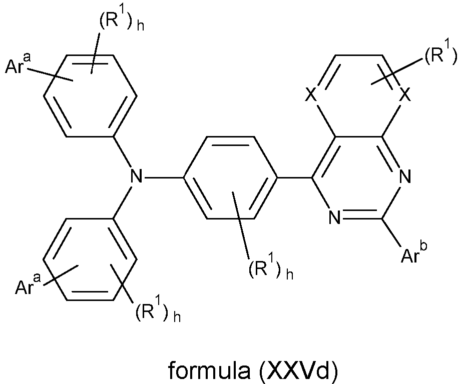 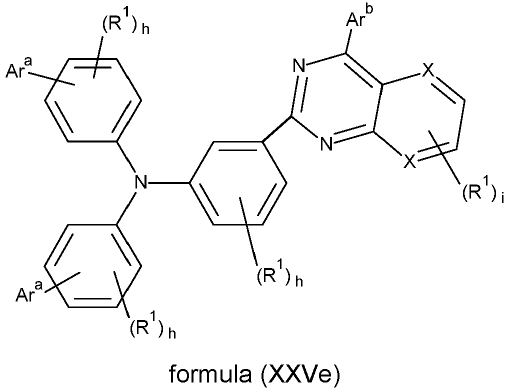 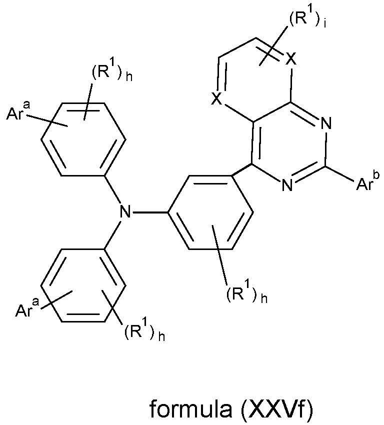 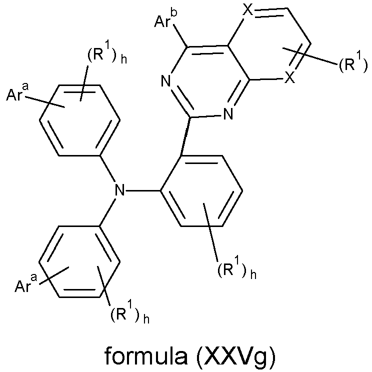 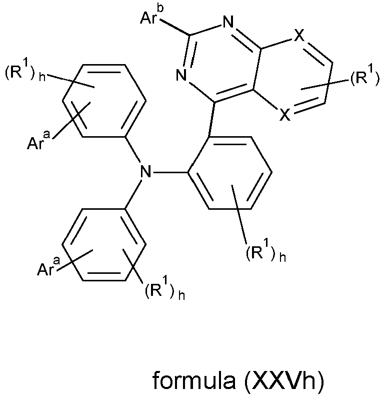 where the symbols Ar^{a}, Ar^{b}, R¹ and X used have the meaning given in Claim 1, i stands for 0, 1 or 2 and h stands for 0, 1, 2, 3 or 4.

8. Compound according to at least one of the preceding claims, **characterised in that** the group Ar^{b} represents a group of the formula (Ar^{b}-1), 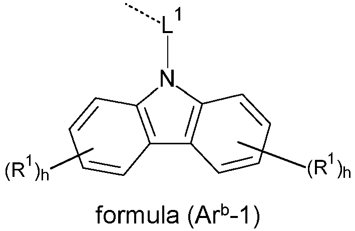 in which L¹ is a bond or an aromatic or heteroaromatic ring system having 5 to 30 aromatic ring atoms, which may be substituted by one or more radicals R¹, the symbol R¹ has the meaning given in Claim 1, h is 0, 1, 2, 3 or 4 and the dashed line represents the bond.

9. Oligomer, polymer or dendrimer comprising one or more compounds according to one or more of Claims 1 to 8, where, instead of a hydrogen atom or a substituent, one or more bonds are present from the compounds to the polymer, oligomer or dendrimer.

10. Composition comprising at least one compound according to one or more of Claims 1 to 8 or an oligomer, polymer or dendrimer according to Claim 9 and at least one further compound selected from the group consisting of fluorescent emitters, phosphorescent emitters, emitters which exhibit TADF, host materials, electron-transport materials, electron-injection materials, hole-conductor materials, hole-injection materials, electron-blocking materials and hole-blocking materials.

11. Formulation comprising at least one compound according to one or more of Claims 1 to 8 and/or an oligomer, polymer or dendrimer according to Claim 9 and/or a composition according to Claim 10 and at least one solvent.

12. Use of a compound according to one or more of Claims 1 to 8, an oligomer, polymer or dendrimer according to Claim 9 or a composition according to Claim 10 in an electronic device, in particular as host material or electron-transport material.

13. Process for the preparation of a compound according to one or more of Claims 1 to 8 or an oligomer, polymer and/or dendrimer according to Claim 9, **characterised in that** a diarylamine compound is connected to a compound comprising at least one diazanaphthyl group in a coupling reaction.

14. Electronic device containing at least one compound according to one or more of Claims 1 to 8, an oligomer, polymer or dendrimer according to Claim 9 or a composition according to Claim 10, where the electronic device is preferably selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells or organic laser diodes.

## Revendications

1. Composé comprenant au moins une structure de formule (IIIa), (IIIb), (XXIa), (XXIb), (XXIVa) ou (XXIVb), 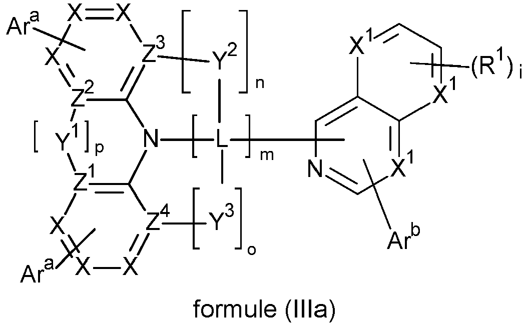 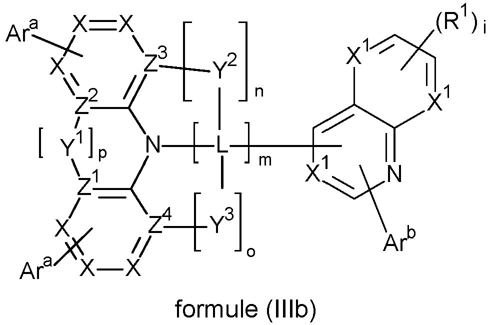 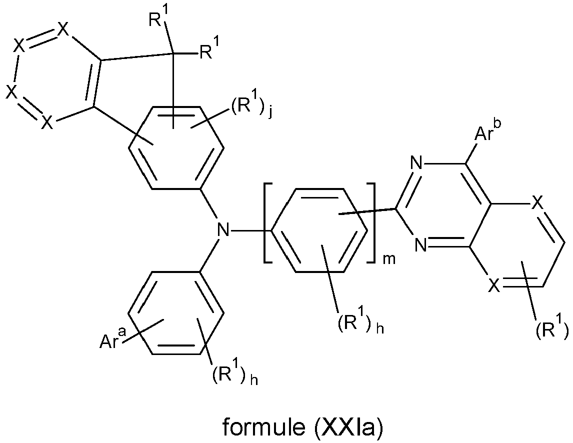 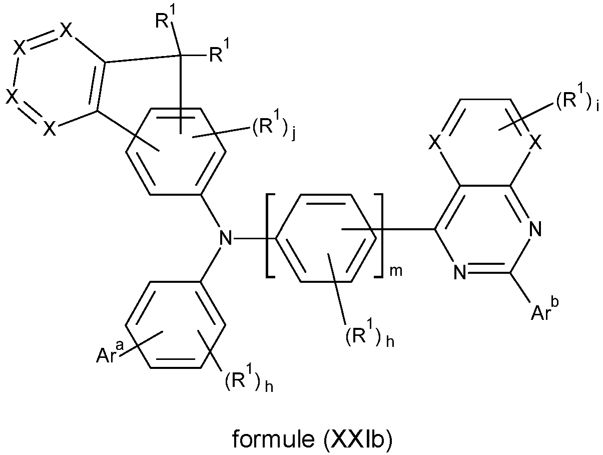 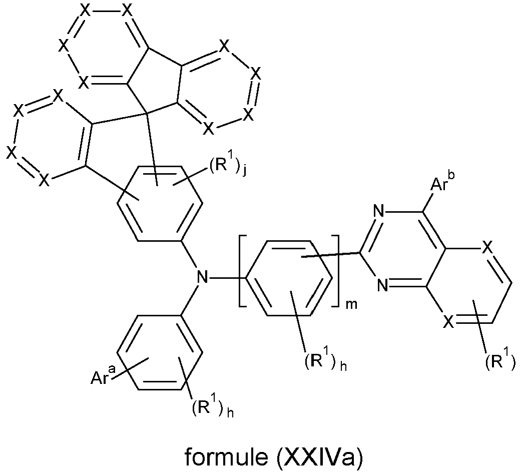 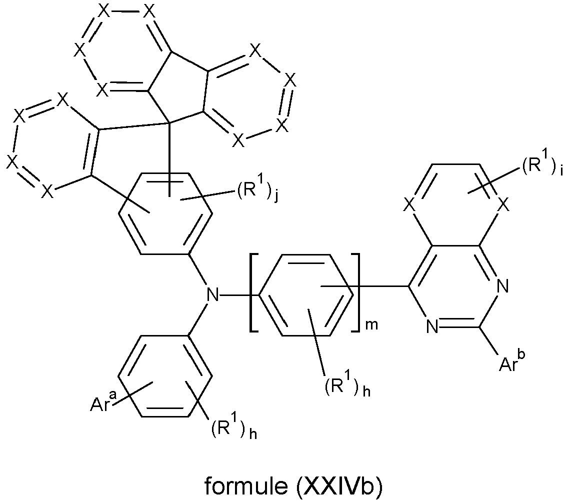
dans laquelle ce qui suit s'applique aux symboles utilisés :
L est un noyau aromatique ou hétéroaromatique ayant de 5 à 30 atomes de cycle aromatique, qui peut être substitué par un ou plusieurs radicaux non aromatiques R¹ ;
Z¹, Z², Z³, Z⁴ sont X ;
X est à chaque occurrence, de manière identique ou différente, N ou CR¹, où un maximum de deux X par cycle représentent N, préférablement CR¹, ou C si un radical Ar^{a} est lié à X ;
X¹ est à chaque occurrence, de manière identique ou différente, N ou CR¹ ;
n, o, p valent 0 ;
m vaut 0 ou 1 ;
i vaut 0, 1 ou 2 ;
j vaut 0, 1, 2 ou 3 ;
h vaut 0, 1, 2, 3 ou 4 ;
Ar^{a} est à chaque occurrence, de manière identique ou différente, un noyau aromatique ou hétéroaromatique ayant de 5 à 30 atomes de cycle aromatique, qui peut être substitué par un ou plusieurs radicaux R¹, où le radical Ar^{a} ne comprend pas de groupement carbazole ou forme un groupement carbazole avec le groupement aryle ou hétéroaryle auquel Ar^{a} est lié, où ceci inclut les substituants R¹, R² et R³ qui peuvent être liés au radical Ar^{a} ;
Ar^{b} est un noyau aromatique ou hétéroaromatique ayant de 5 à 45 atomes de cycle aromatique, qui peut être substitué par un ou plusieurs radicaux R¹ ;
R¹ est à chaque occurrence, de manière identique ou différente, H, D, CN, N(Ar¹)₂, un groupement alkyle à chaîne linéaire ayant 1, 2, 3 ou 4 atomes de C ou un groupement alkyle ramifié ou cyclique ayant de 3 à 8 atomes de C, ou un noyau aromatique ou hétéroaromatique ayant de 5 à 40 atomes de cycle aromatique, qui peut dans chaque cas être substitué par un ou plusieurs radicaux R², ou une combinaison de ces systèmes ; deux, ou plus, substituants R¹ peuvent également former un noyau aliphatique monocyclique ou un noyau aromatique les uns avec les autres ;
Ar¹ est à chaque occurrence, de manière identique ou différente, un noyau aromatique ou hétéroaromatique ayant de 5 à 30 atomes de cycle aromatique, qui peut être substitué par un ou plusieurs radicaux non aromatiques R² ; deux radicaux Ar¹ qui sont liés au même atome de Si, atome de N, atome de P ou atome de B peuvent également être pontés l'un avec l'autre par une liaison simple ou un pont choisi parmi B(R²), C(R²)₂, Si(R²)₂, C=O, C=NR², C=C(R²)₂, O, S, S=O, SO₂, N(R²), P(R²) et P(=O)R² ;
R² est à chaque occurrence, de manière identique ou différente, H, D, CN, un groupement alkyle à chaîne linéaire ayant 1, 2, 3 ou 4 atomes de C ou un groupement alkyle ramifié ayant 3 ou 4 atomes de C, qui peut dans chaque cas être substitué par un ou plusieurs radicaux R³, où un ou plusieurs atomes de H peuvent être remplacés par D ou CN, ou un noyau aromatique ou hétéroaromatique ayant de 5 à 40 atomes de cycle aromatique, qui peut dans chaque cas être substitué par un ou plusieurs radicaux R³, ou une combinaison de ces systèmes ; deux, ou plus, substituants R² peuvent également former un noyau aliphatique monocyclique ou un noyau aromatique les uns avec les autres ;
R³ est choisi à chaque occurrence, de manière identique ou différente, dans le groupe constitué par H, D, CN, un radical hydrocarboné aliphatique ayant 1, 2, 3 ou 4 atomes de C ou un noyau aromatique ou hétéroaromatique ayant de 5 à 30 atomes de cycle aromatique, où un ou plusieurs atomes de H peuvent être remplacés par D ou CN et qui peut être substitué par un ou plusieurs groupements alkyle, chacun ayant de 1 à 4 atomes de carbone ; deux, ou plus, substituants R³ peuvent former un noyau aliphatique monocyclique les uns avec les autres ;
à condition qu'au moins un groupement X¹ dans la formule (IIIa) ou (IIIb) représente N, et
à condition que les composés de formule (A) 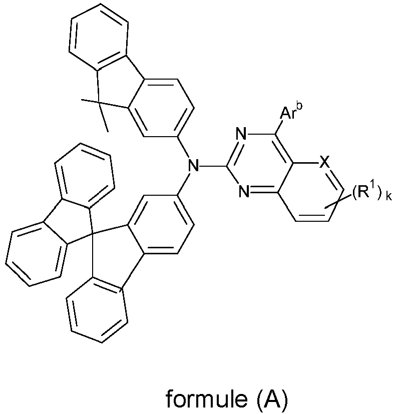
soient exclus, dans laquelle les symboles X, Ar^{b} et R¹ utilisés revêtent la signification décrite ci-dessus et k vaut 0 ou 1.

2. Composé selon la revendication 1, comprenant au moins une structure de formule (Va) ou (Vb), 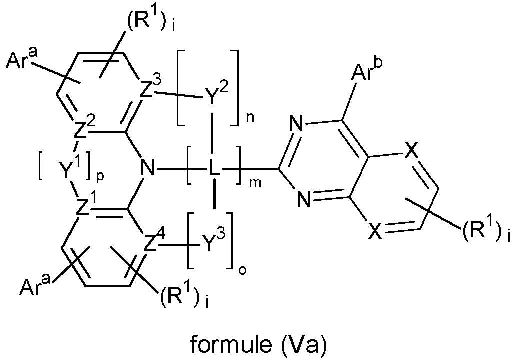 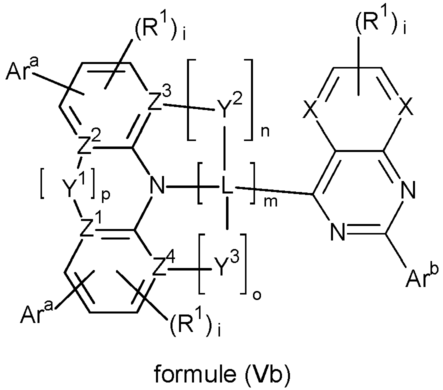 dans laquelle les symboles m, n, o, p, L, R¹, Ar^{a}, Ar^{b}, Z¹, Z², Z³, Z⁴ et X utilisés revêtent la signification donnée selon la revendication 1 et i représente 0, 1 ou 2.

3. Composé selon la revendication 2, comprenant au moins une structure de formule (XVIIIa) ou (XVIIIb), 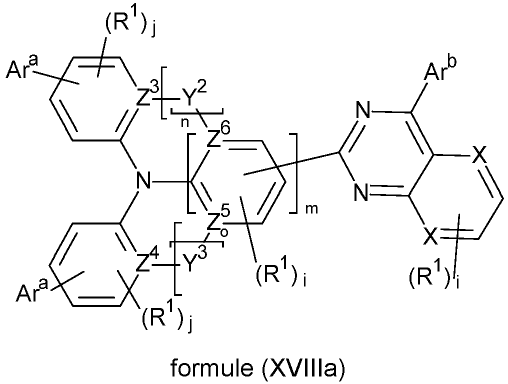 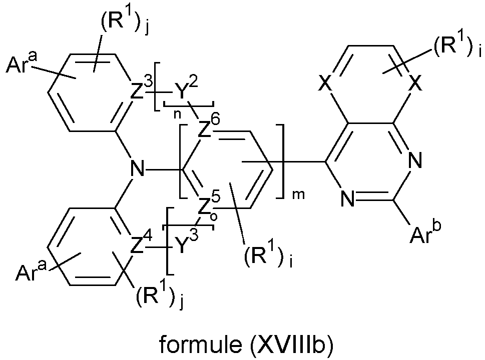 dans laquelle les symboles m, n, o, Ar^{a}, Ar^{b}, Z³, Z⁴ et X utilisés revêtent la signification donnée selon la revendication 1, Z⁵ et Z⁶ sont identiques ou différents et représentent X ou C, i représente 0, 1 ou 2 et j représente 0, 1, 2 ou 3.

4. Composé selon la revendication 3, comprenant au moins une structure de formule (XIXa) ou (XIXb), 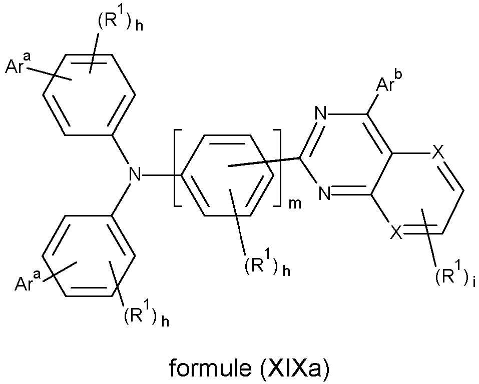 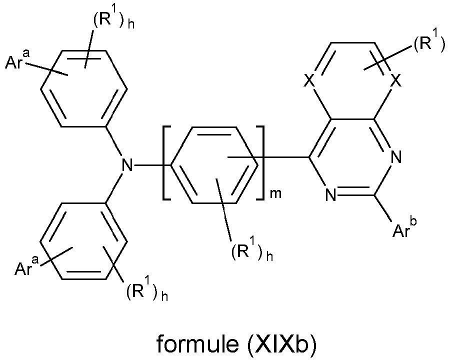 dans laquelle les symboles m, Ar^{a}, Ar^{b}, R¹ et X utilisés revêtent la signification donnée selon la revendication 1, i représente 0, 1 ou 2 et h représente 0, 1, 2, 3 ou 4.

5. Composé selon la revendication 1, comprenant au moins une structure de formule (XXIIa) ou (XXIIb), 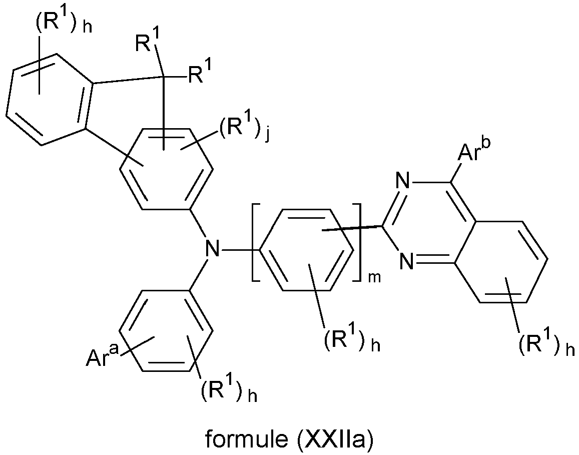 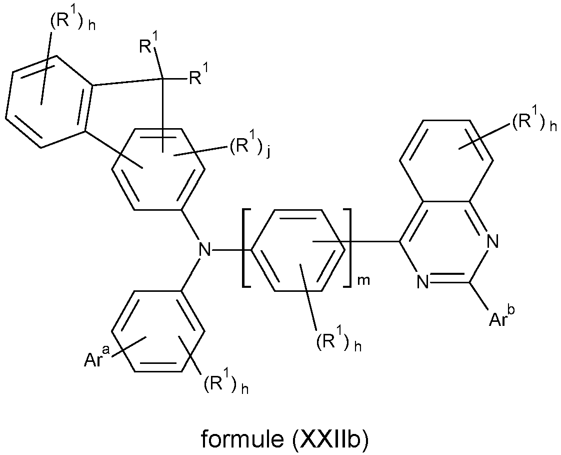 dans laquelle les symboles m, Ar^{a}, Ar^{b} et R¹ utilisés revêtent la signification donnée selon la revendication 1, j représente 0, 1, 2 ou 3 et h représente 0, 1, 2, 3 ou 4.

6. Composé selon la revendication 1, comprenant au moins une structure de formule (XXIIIa), (XXIIIb), (XXIIIc), (XXIIId), (XXIIIe) ou (XXIIIf), 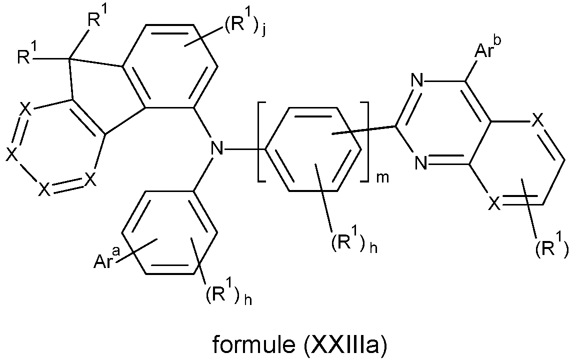 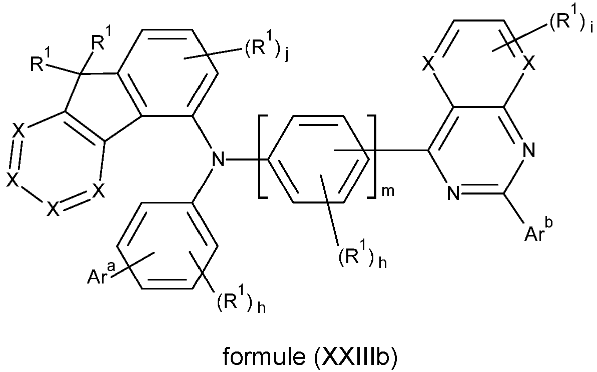 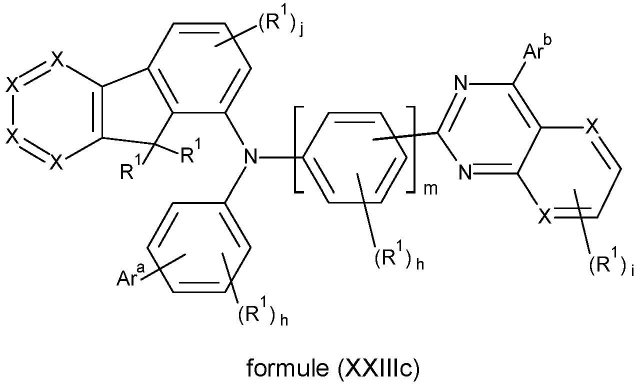 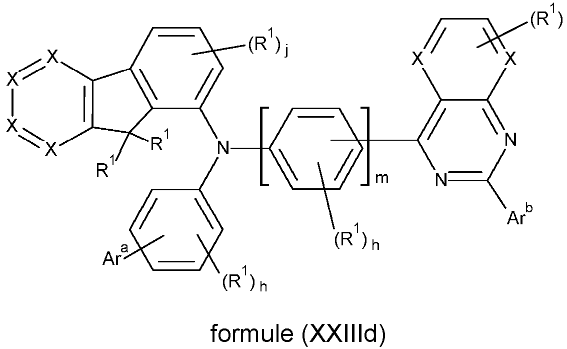 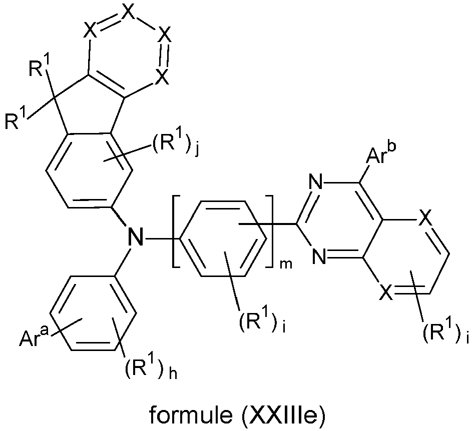 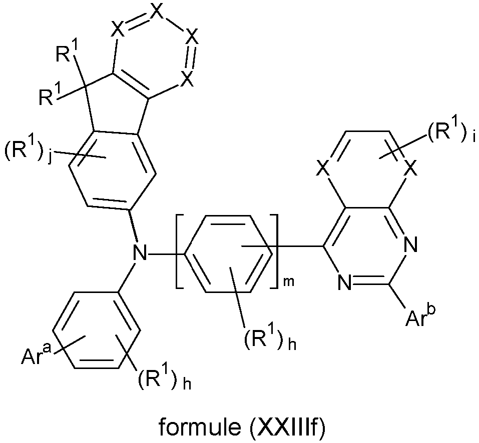 dans laquelle les symboles m, Ar^{a}, Ar^{b}, R¹ et X utilisés revêtent la signification donnée selon la revendication 1, i représente 0, 1 ou 2, j représente 0, 1, 2 ou 3 et h représente 0, 1, 2, 3 ou 4.

7. Composé selon l'une parmi les revendications 1 à 6, comprenant au moins une structure de formule (XXVa), (XXVb), (XXVc), (XXVd), (XXVe), (XXVf), (XXVg) ou (XXVh), 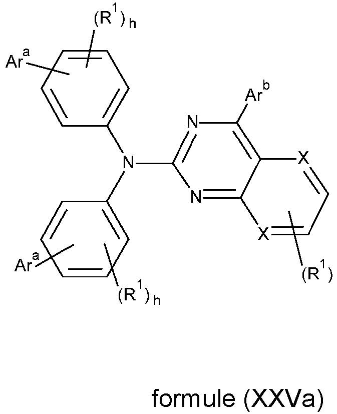 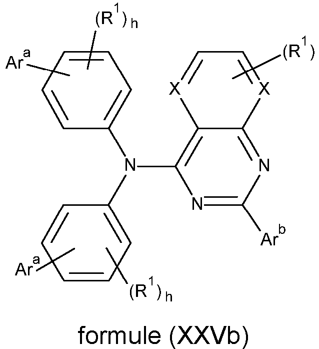 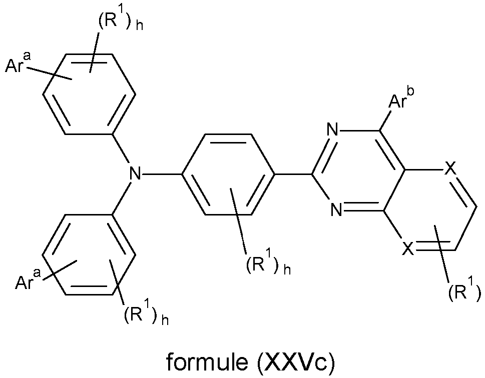 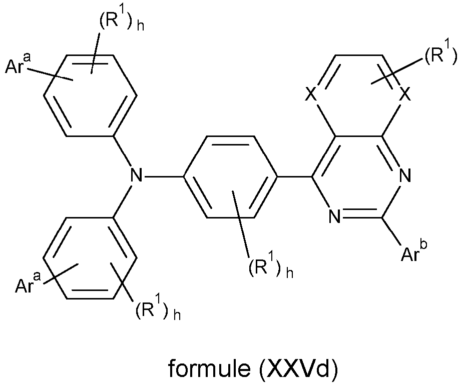 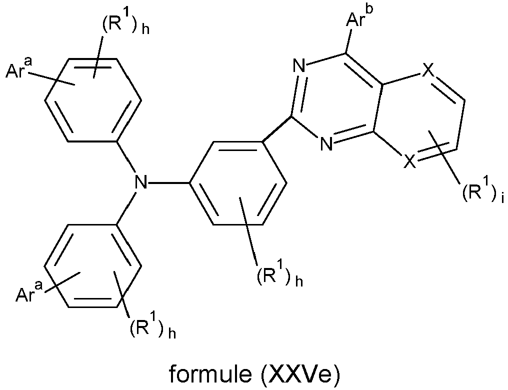 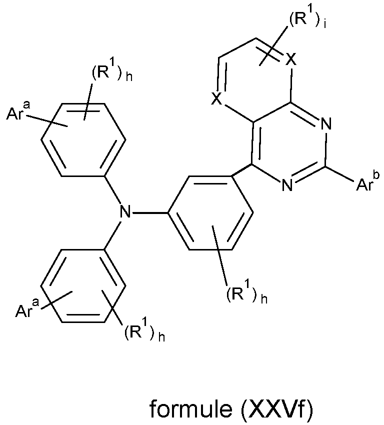 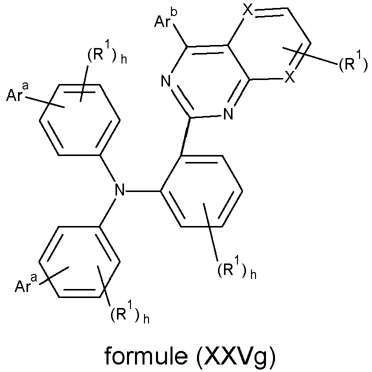 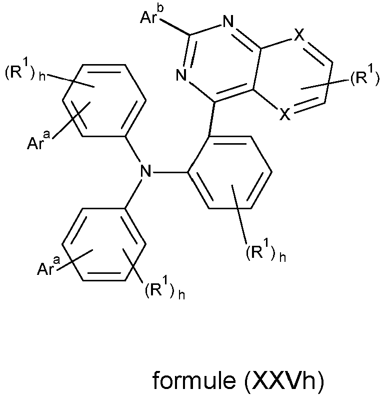 dans laquelle les symboles Ar^{a}, Ar^{b}, R¹ et X utilisés revêtent la signification donnée selon la revendication 1, i représente 0, 1 ou 2 et h représente 0, 1, 2, 3 ou 4.

8. Composé selon au moins l'une parmi les revendications précédentes, **caractérisé en ce que** le groupement Ar^{b} représente un groupement de formule (Ar^{b}-1), 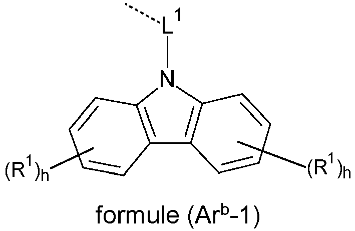 dans laquelle L' est une liaison ou un noyau aromatique ou hétéroaromatique ayant de 5 à 30 atomes de cycle aromatique, qui peut être substitué par un ou plusieurs radicaux R¹, le symbole R¹ revêt la signification donnée selon la revendication 1, h vaut 0, 1, 2, 3 ou 4 et la ligne en pointillés représente la liaison.

9. Oligomère, polymère ou dendrimère comprenant un ou plusieurs composés selon l'une ou plusieurs parmi les revendications 1 à 8, où, au lieu d'un atome d'hydrogène ou d'un substituant, une ou plusieurs liaisons sont présentes entre les composés et le polymère, l'oligomère ou le dendrimère.

10. Composition comprenant au moins un composé selon l'une ou plusieurs parmi les revendications 1 à 8 ou un oligomère, polymère ou dendrimère selon la revendication 9 et au moins un autre composé choisi dans le groupe constitué par les émetteurs fluorescents, les émetteurs phosphorescents, les émetteurs présentant une TADF, les matériaux hôtes, les matériaux de transport d'électrons, les matériaux d'injection d'électrons, les matériaux conducteurs de trous, les matériaux d'injection de trous, les matériaux de blocage d'électrons et les matériaux de blocage de trous.

11. Formulation comprenant au moins un composé selon l'une ou plusieurs parmi les revendications 1 à 8 et/ou un oligomère, polymère ou dendrimère selon la revendication 9 et/ou une composition selon la revendication 10 et au moins un solvant.

12. Utilisation d'un composé selon l'une ou plusieurs parmi les revendications 1 à 8, d'un oligomère, polymère ou dendrimère selon la revendication 9, ou d'une composition selon la revendication 10, dans un dispositif électronique, en particulier comme matériau hôte ou matériau de transport d'électrons.

13. Procédé de préparation d'un composé selon l'une ou plusieurs parmi les revendications 1 à 8 ou d'un oligomère, polymère ou dendrimère selon la revendication 9, **caractérisé en ce qu'**un composé de diaryl-amine est relié à un composé comprenant au moins un groupement diazanaphtyle dans une réaction de couplage.

14. Dispositif électronique contenant au moins un composé selon l'une ou plusieurs parmi les revendications 1 à 8, un oligomère, polymère ou dendrimère selon la revendication 9, ou une composition selon la revendication 10, le dispositif électronique étant préférablement choisi dans le groupe constitué par les dispositifs électroluminescents organiques, les circuits intégrés organiques, les transistors organiques à effet de champ, les transistors organiques en couche mince, les transistors organiques émetteurs de lumière, les cellules solaires organiques, les détecteurs optiques organiques, les photorécepteurs organiques, les dispositifs organiques à extinction de champ, les cellules électrochimiques émettrices de lumière ou les diodes laser organiques.
